# EUROPEAN PATENT APPLICATION

(11) **EP 2 974 739 A1**
(43) Date of publication of application: **20.01.2016**
(21) Application number: 14177182.4
(22) Date of filing: 15.07.2014
(51) Int. Cl.: A61K 39/155, C07K 19/00, C07K 14/005

(54) **RSVF trimerization domains**

(71) Applicant: Novartis AG, 4056 Basel (CH)
(72) Inventor: Carfi, Andrea, East Hanover, NJ New Jersey 07936 (US); Mason, Peter W., East Hanover, NJ New Jersey 07936-1080 (US); Settembre, Ethan C., East Hanover, NJ New Jersey 07936 (US); Swanson, Kurt, East Hanover, NJ New Jersey 07936-1080 (US); Chandramouli, Sumana, East Hanover, NJ New Jersey 07936 (US)
(74) Representative: Courgeon, Antoine

(57) **Abstract**

Complexes that contain RSVF ectodomain polypeptides and methods for making the complexes are disclosed. The RSVF ectodomain polypeptides can be in the prefusion form.

## Description

### BACKGROUND OF THE INVENTION

Respiratory syncytial virus (RSV) is an enveloped non-segmented negative-strand RNA virus in the family *Paramyxoviridae,* genus *Pneumovirus.* It is the most common cause of bronchiolitis and pneumonia among children in their first year of life. RSV also causes repeated infections including severe lower respiratory tract disease, which may occur at any age, especially among the elderly or those with compromised cardiac, pulmonary, or immune systems.

To infect a host cell, paramyxoviruses such as RSV, like other enveloped viruses such as influenza virus and HIV, require fusion of the viral membrane with a host cell's membrane. For RSV the conserved fusion protein (RSVF) fuses the viral and cellular membranes by coupling irreversible protein refolding with juxtaposition of the membranes. In current models based on paramyxovirus studies, the RSVF protein initially folds into a metastable "pre-fusion" conformation. During cell entry, the pre-fusion conformation undergoes refolding and conformational changes to its stable "post-fusion" conformation.

The RSVF protein is translated from mRNA into an approximately 574 amino acid protein designated F₀. Post-translational processing of F₀ includes removal of an N-terminal signal peptide by a signal peptidase in the endoplasmic reticulum. F₀ is also cleaved at two sites (approximately 109/110 and approximately 136/137) by cellular proteases (in particular furin) in the trans-Golgi. This cleavage results in the removal of a short intervening sequence and generates two subunits designated F₁ (∼50 kDa; C-terminal; approximately residues 137-574) and F₂ (∼20 kDa; N- terminal; approximately residues 1-109) that remain associated with each other. F₁ contains a hydrophobic fusion peptide at its N-terminus and also two amphipathic heptad-repeat regions (HRA and HRB). HRA is near the fusion peptide and HRB is near the transmembrane domain. Three F₁-F₂ heterodimers are assembled as homotrimers of F₁-F₂ in the virion.

A vaccine against RSV infection is not currently available but is desired. One potential approach to producing a vaccine is a subunit vaccine based on purified RSVF protein. However, for this approach it is desirable that the purified RSVF protein is in a single form and conformation that is stable over time, consistent between vaccine lots, and conveniently purified.

The RSVF protein can be truncated, for example by deletion of the transmembrane domain and cytoplasmic tail, to permit its expression as an ectodomain, which may be soluble. In addition, although RSVF protein is initially translated as a monomer, the monomers are cleaved and assemble into trimers. When RSVF protein is in the form of cleaved trimers, the hydrophobic fusion peptide is exposed. The exposed hydrophobic fusion peptides on different trimers, e.g., soluble ectodomain trimers, can associate with each other, resulting in the formation of rosettes. The hydrophobic fusion peptides can also associate with lipids and lipoproteins, for example from cells that are used to express recombinant soluble RSVF protein. Due to the complexity of RSVF protein processing, structure and refolding, purified, homogeneous, immunogenic preparations are difficult to obtain.

The structure of the pre-fusion form of RSVF was recently solved (McLellan, JS et al., Science, 342(6158):592-8(2013), incorporated herein by reference.) The pre-fusion form of RSVF contains epitopes that are not present on the post-fusion form. See, *e.g.,* Magro, M. et al., Proc. Natl. Acad. Sci. USA, 109(8):3089-94 (2012)). Thus, for vaccines, the stabilized pre-fusion form is generally considered more desirable antigenically. Several RSVF constructs have been generated using the general theme of GCN-stabilization. However, in each case, whether the HRB was stabilized with a GCN, engineered disulfide bonds or point mutations designed to strengthen the trimer HRB hydrophobic core interactions, the result was a protein that was not expressed and exported from the cell efficiently. Bacteriophage T4 foldon sequences have also been used to promote trimer formation and stabilization. Attempts to make a post-fusion RSVF that has mutations to its furin cleavage sites to prevent fusion peptide release resulted in failure of the RSVF to form trimers similar to those observed in the well studied parainfluenza virus F's.

Thus, there is a need for improved RSVF protein compositions and methods for making RSVF protein compositions.

### SUMMARY OF THE INVENTION

The invention provides compositions comprising a respiratory syncytial virus F (RSVF) complex or populations thereof, methods and compositions for making the complexes, and uses of the complexes.

The invention provides a respiratory syncytial virus F (RSVF) complex, comprising a six helix bundle, comprising:
three RSVF ectodomain polypeptides each comprising an endogenous HRA region, an endogenous HRB region, further comprising one of an inserted RSVF HRA region or an inserted RSVF HRB region and
at least one oligomerization polypeptide, wherein the three ectodomain polypeptides and the at least one oligomerization polypeptide form a six-helix bundle, with the proviso that the six-helix bundle does not include the endogenous HRA regions and endogenous HRB regions of the RSVF polypeptides, wherein the inserted HRA region or inserted HRB region is optionally operably linked C-terminal to the ectodomain endogenous RSVF HRB region in the RSVF ectodomain polypeptide.

In certain embodiments, the six helix bundle comprises the inserted HRA region of each RSVF ectodomain polypeptide and the oligomerization polypeptide comprises an HRB region; or the the six helix bundle comprises the inserted HRB region of each RSVF ectodomain polypeptide and the oligomerization polypeptide comprises an HRA region. In certain embodiments, the oligomerization polypeptide is not operably linked to an RSVF ectodomain polypeptide. In certain embodiments, the oligomerization polypeptide is operably linked to at least one RSVF ectodomain polypeptide.

In certain embodiments, the at least one operable link between the ectodomain and the inserted six-helix bundle forming moiety and the operable link, when present, between the ectodomain and the oligomerization domain, comprises a polypeptide linker and/or a structurally constrained linker. In all embodiments, when two linkers are present, the composition of the linkers is selected independently, e.g., for length and rigidity, although the linkers may be the same.

In certain embodiments, the inserted six-helix bundle forming moiety and the oligomerization polypeptide comprise complementary heptad repeat regions of a fusion protein of an enveloped virus, optionally selected from the group consisting of an RSVF HRA and an RSVF HRB; an HIV gp41 HR1 region and an HIV gp41 HR2 region; Newcastle disease virus (NDV) HR1 and NVD HR2, human metapneumovirus HR1 and human metapneumovirus HR; and other class 1 fusion viral proteins that contain 6-helix bundles in their postfusion state. In preferred embodiments, the inserted domain and the oligomerization domain comprise of an RSVF HRA and an RSVF HRB.

In certain embodiments, the at least one oligomerization polypeptide comprises three oligomerization polypeptides.

In certain embodiments, the complex includes a purification tag, for example on the oligomerization polypeptide which preferably can be cleaved from the complex without cleaving the ectodomain polypeptides.

In certain embodiments, one or more of the RSVF ectodomain polypeptides comprises an uncleaved RSVF ectodomain polypeptide or a cleaved RSVF ectodomain polypeptide. Cleavage sites can include, for example furin cleavage sites, including optimized furin cleavage sites.

In certain embodiments, the ectodomains in the RSVF complexes comprises substitutions selected from the group consisting of S155C and S290C substitutions; S190F, and V207L substitutions; and S155C, S290C, S190F, and V207L substitutions. Preferably, all four substitutions are present.

The invention further provides methods for producing a respiratory syncytial virus F (RSVF) complex comprising a six-helix bundle, comprising:
a) providing RSVF protein ectodomain polypeptides comprising an endogenous HRA region and an endogenous HRB region, further comprising a six helix bundle forming moiety; and at least one oligomerization polypeptide, and
b) combining the RSVF ectodomain polypeptides and the at least one oligomerization polypeptide under conditions suitable for the formation of an RSVF complex, whereby an RSVF complex is produced in which three of the RSVF ectodomain polypeptides and at least one of the oligomerization polypeptides form a six-helix bundle, with the proviso that the endogenous HRA regions and the endogenous HRB regions of the RSVF ectodomain polypeptides are not part of the six-helix bundle.

In certain embodiments, the inserted six-helix bundle forming moiety and the oligomerization polypeptide comprise complementary heptad repeat regions of a fusion protein of an enveloped virus, selected from the group consisting of an RSVF HRA and an RSVF HRB; an HIV gp41 HR1 region and an HIV gp41 HR2 region; Newcastle disease virus (NDV) HR1 and NVD HR2, human metapneumovirus HR1 and human metapneumovirus HR; and other class 1 fusion viral proteins that contain 6-helix bundles in their postfusion state.

In certain embodiments, the RSVF protein ectodomain polypeptides and the oligomerization polypeptide are provided in a cell. In certain embodiments, the RSVF protein ectodomain polypeptides and the oligomerization polypeptide are provided in conditioned cell culture media. In certain embodiments, the RSVF protein ectodomain polypeptides and the oligomerization polypeptide are provided as expression constructs, including expression constructs transfected into a cell. In certain embodiments, the RSVF protein ectodomain polypeptides and the oligomerization polypeptide are provided and as purified components mixed together in the absence of cells or substantially contaminanting material from cells of synthetic purification methods. The invention provides compositions for making the RSVF complexes of the invention including, for example, expression constructs and cells containing expression constructs.

The invention provides populations of the RSVF complexes of the invention. In certain embodiments, the population of the RSVF complexes, at least 80% of the ectodomain polypeptides are cleaved. In certain embodiments, the population of the RSVF complexes, at least 80% of the ectodomain polypeptides are present in a trimer. In certain embodiments, the population of the RSVF complexes, at least 80% of the ectodomain polypeptides are cleaved and at least 80% of the ectodomain polypeptides are present in a trimer.

The invention provides RSVF complexes made by the methods of the invention.

The invention provides RSVF complexes in pharmaceutical compositons. The invention provides RSVF complexes in immunogenic compositons. The invention provides RSVF complexes in vaccine compositons. The invention further provides for methods of administering such compositions to a subject, e.g., for the purpose of immunization and/or raising an immune response in the subject, preferably a neutralizing immune response in the subject.

Other embodiments are disclosed *infra.*

### BRIEF DESCRIPTION OF THE DRAWINGS

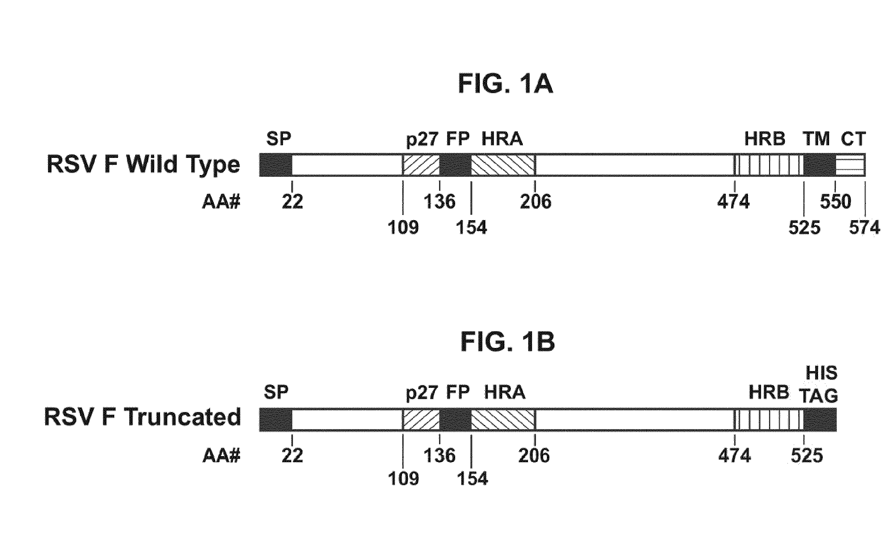

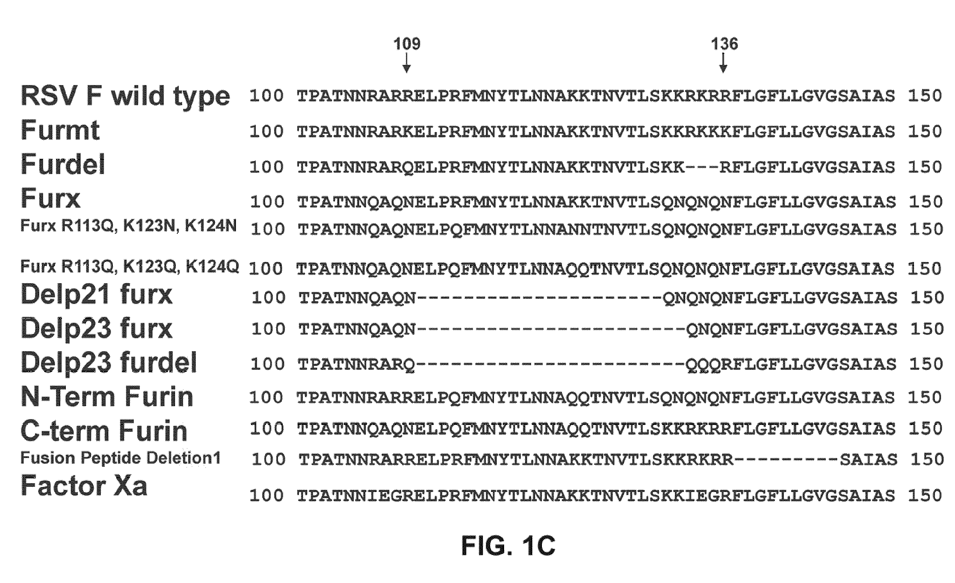

FIG. 1 shows the schematic of the wild-type RSVF (FIG 1A) and of an ectodomain construct in which the transmembrane domain and cytoplasmic tail have been removed and an optional HIS6-tag (SEQ ID NO: 39) has been added to the C-terminus (FIG 1B). For clarity, residue numbering is related to the wild-type A2 strain RSVF beginning at the N-terminal signal peptide and is not altered in constructs containing amino acid deletions. Labeled in the schematics is the signal sequence or signal peptide (sp). FIG. 1A is a schematic of RSVF protein showing the signal sequence or signal peptide (SP), p27 linker region, fusion peptide (FP), HRA domain (HRA), HRB domain (HRB), transmembrane region (TM), and cytoplasmic tail (CT). The C-terminal bounds of the ectodomain can vary. FIG 1B is a general schematic of the RSVF ectodomain construct depicting the shared features with the schematics in FIG. 1A and including an optional HIS₆-tag (HIS TAG) (SEQ ID NO: 39). Furin cleavage sites are present at amino acid positions 109/110 and 136/137. FIG. 1C also shows the amino acid sequence of amino acids 100 - 150 of RSVF (wild-type) (SEQ ID NO:25) and several proteins (Furmt-SEQ ID NO:3; Furdel-SEQ ID NO:4; Furx-SEQ ID NO:5; Furx R113Q, K123N, K124N-SEQ ID NO:6; Furx R113Q, K123Q, K124Q-SEQ ID NO:7; Delp21 furx-SEQ ID NO:8; Delp23 furx-SEQ ID NO:9; Delp23 furdel-SEQ ID NO:11; N-Term Furin-SEQ ID NO:12; C-term Furin-SEQ ID NO:13; Fusion Peptide Deletion 1-SEQ ID NO:26; and Factor Xa-SEQ ID NO:14) in which one or both furin cleavage sites and/or fusion peptide region were mutated or deleted. In FIG. 1C, the symbol "-" indicates that the amino acid at that position is deleted.

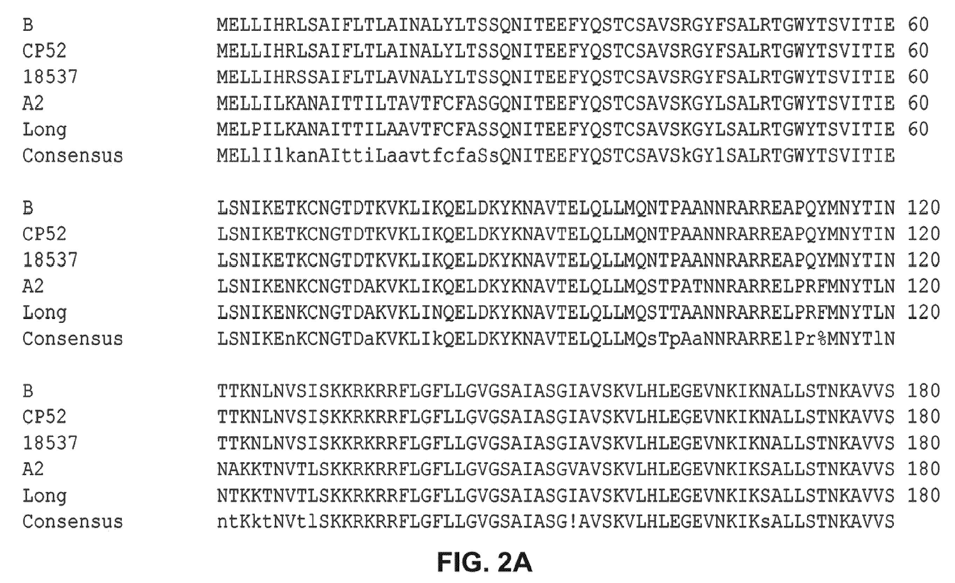

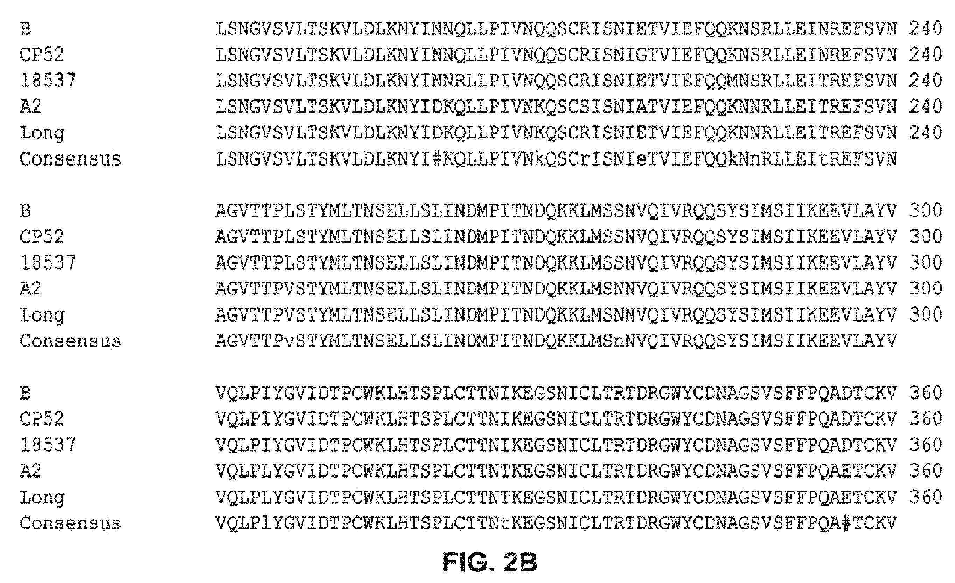

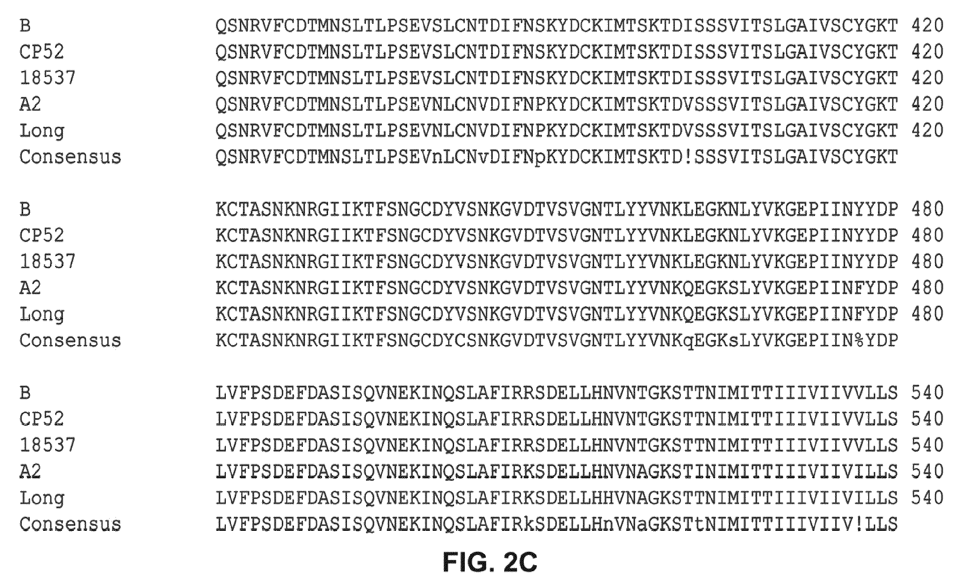

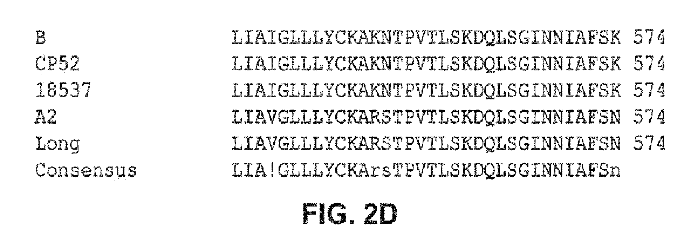

FIG. 2 is an alignment of the amino acid sequences of F proteins from several strains of RSV. The alignment was prepared using the algorithm disclosed by Corpet, Nucleic Acids Research, 1998, 16(22):10881-10890, using default parameters (Blossum 62 symbol comparison table, gap open penalty: 12, gap extension penalty: A2, F protein of the strain A2 (accession number AF035006) (SEQ ID NO: 27); CP52, F protein of the CP52 strain (accession number AF013255) (SEQ ID NO: 28); B, F protein of the B strain (accession number AF013254) (SEQ ID NO: 29); long, F protein of the long strain (accession number AY911262) strain (SEQ ID NO: 30), and 18537strain, F protein of the 18537 strain (accession number Swiss Prot P13843) (SEQ ID NO: 31). A consensus of F protein sequences is also shown (SEQ ID NO: 24). Each Accession No. is incorporated herein by reference in the version available on the date of filing the instant application.

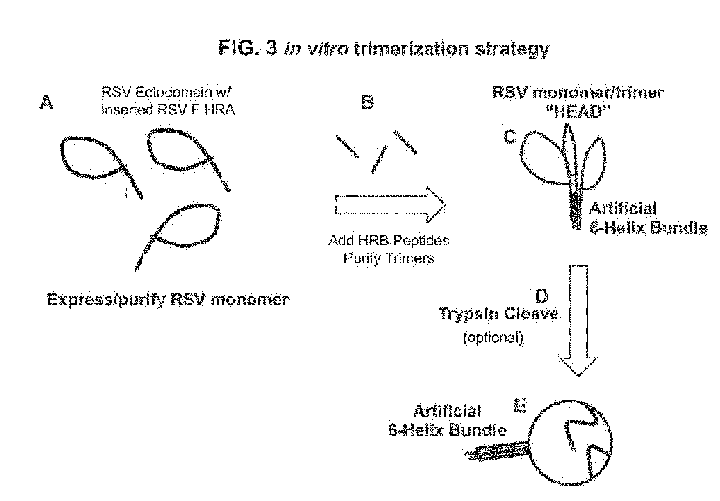

FIG. 3 is an exemplary schematic showing an *in vitro* trimerization process, whereby RSVF monomer solution containing endogenous HRA and endogenous HRB further comprising an inserted HRA domain (the ectodomain peptides) are expressed and purified, then mixed with HRB peptides (the oligomerization peptides), inducing the formation of a six molecule complex that contains an inserted HRA operably linked to the ectodomain F protein and an added HRB oligomerization peptide in the form of an RSV monomer/trimer "head" and an artificial 6-helix bundle (A, B and C). Trimers are purified, and optionally a protease can be used to cleave a cleavable monomer, which may allow the globular head of prefusion F to form (D and E).

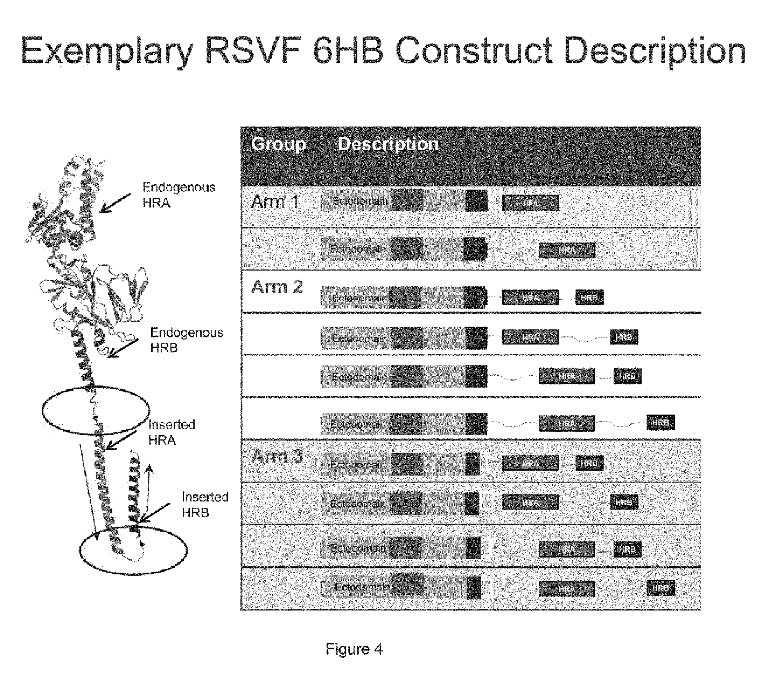

FIG. 4 shows exemplary constructs for use in the invention. The Arm 1 constructs include an ectodomain with endogenous HRA and HRB domains. Short or long linkers are used to operably link the inserted HRA domain to the ectodomain. The Arm 2 constructs include an ectodomain with endogenous HRA and HRB domains. Short and long linkers are used to to operably link the inserted HRA domain and the HRB oligomerization domain (through the inserted HRA domain) to the ectodomain. As shown schematically, the inserted HRA domain and the HRB oligomerization domain can be linked to each other with a short or long linker. The Arm 3 constructs are similar to the Arm 2 constructs except that they include a truncated endogenous HRB domain in which the truncated HRB has the sequence FYDPLVFPSDEFDASISQVNEKINQS (SEQ ID NO: 41).

### DETAILED DESCRIPTION OF THE INVENTION

The inventors have developed an *in vitro* approach that uses oligomerizing peptides or inserted oligomerizing moieties to produce RSVF complexes in which all or a portion of the oligomerizing polypeptide or the inserted oligomerizing moieties forms a six-helix bundle with an inserted portion of the RSVF polypeptide (e.g., inserted RSVF HRA or inserted RSVF HRB sequence). Accordingly, in some aspects, the invention relates to soluble RSVF polypeptide complexes that contain three RSVF ectodomain polypeptides comprising an endogenous RSVF HRA domain and an endogenous RSVF HRB domain; and one of an inserted 6-helix bundle forming domain e.g., from a class 1 fusion viral proteins that contain 6-helix bundles, preferably an HRA, HRB, HR1, or HR2 domain, preferably an RSVF HRA domain or an inserted RSVF HRB domain; and three oligomerization, domains typically provided in three oligomerization domain polypeptides, comprising a 6-helix bundle forming domain e.g., from a class 1 fusion viral proteins that contain 6-helix bundles, preferably an HRA, HRB, HR1, or HR2 domain, typically an RSVF HRB domain containing peptide when the ecotodomain comprises an inserted RSVF HRA domain; or typically an RSVF HRA domain containing peptide when the ecotodomain comprises an inserted RSVF HRB domain.

As described herein, the complexes are stable and can conveniently be produced on a commercial scale. Stable complexes are able to produce immunogenic compositions in which the protein has a decreased tendency to aggregate or degrade, which provides a more predictable immune response when the composition is administered to a subject. In some embodiments, the structure of the RSVF ectodomain in the complex is in the pre-fusion conformation. The epitopes of the pre-fusion conformation may be better able to elicit antibodies that can recognize and neutralize natural virions. The invention also relates to methods for producing such complexes, immunogenic compositions comprising the complexes and methods of using the complexes and compositions.

### Definitions

The "post-fusion conformation" of RSVF protein when present in a trimer is characterized by the presence of a six-helix bundle, comprising 3 endogenous HRB and 3 endogenous HRA regions. Post-fusion conformations are further characterized by a cone-shape when viewed in negatively stained electron micrographs and/or by a lack of prefusion epitopes. See, *e.g.,* Magro, M. et al., Proc. Natl. Acad. Sci. USA, 109(8):3089-94 (2012)). RSVF proteins with a post-fusion conformation are not bound by the D25 antibody (Kwakkenbos et al. Nature Medicine 16:123-128(2010)), whereas both pre-fusion and post-fusion RSVF are bound by the Motavizumab antibody (Beeler and van Wyke Coelingh, J. Virol. 63:2941-2950 (1989), incorporated herein by reference). It is understood that the whole RSVF protein need not be present for portions of the protein to be present in a post-fusion conformation.

The "pre-fusion conformation" of RSVF protein when present in a trimer is characterized at least in part by having endogenous HRA regions that do not interact with the endogenous HRB regions to form a six-helix bundle. A six-helix bundle may be present in the pre-fusion conformation, provided that the endogenous HRA regions and the endogenous HRB regions are not a part of the six-helix bundle. Pre-fusion conformations are further characterized by a rounded shape when viewed in negatively stained electron micrographs, similar to that seen in the PIV5 pre-fusion F structure (See, e.g., McLellan, JS et al., Science, 342(6158):592-8(2013); Yin HS, et al. (2006) Nature 439(7072):38-44) and/or by prefusion epitopes that are not present on post-fusion conformations. See, *e.g.,* Magro, M. et al., Proc. Natl. Acad. Sci. USA, 109(8):3089-94 (2012)) RSVF proteins with a pre-fusion conformation are bound by the D25 antibody, whereas both prefusion and post-fusion RSVF are bound by the Motavizumab antibody. It is understood that the whole RSVF protein need not be present for portions of the protein to be present in a pre-fusion conformation.

As used herein, the term "endogenous HRA region" or domain refers to an HRA region that is present in a F polypeptide at substantially the same position as the HRA region in the amino acid sequence of the F0 form of the naturally occurring F protein from which the cleaved F1 and F2 peptides are derived. In the case of RSVF proteins, such as an RSVF ectodomain polypeptide or recombinant RSVF ectodomain polypeptide, the endogenous HRA region is from about amino acid 154 to about amino acid 206 of the polypeptide. Amino acid numbering of the RSVF protein throughout the application, unless otherwise noted, is based on the sequence of wild-type A2 strain of RSVF (SEQ ID NO:1) including the signal peptide, and amino acid positions are assigned to residues that are deleted. For example, if the fusion peptide of RSVF is deleted in whole or in part, the deleted amino acids would be numbered so that the amino acids of HRA region have the same position numbers as in the wild-type sequence. In preferrered embodiments, the endogenous HRA region is a full length HRA region. However, deletions and truncations of the HRA region are included within the scope of the invention as long as such deletions and truncations do not substantially alter the ability of RSVF to fold into an acceptable conformation (either pre-fusion or post-fusion) for its intended purpose. An endogenous HRA region can include one or more mutations such as those discussed herein.

As used herein, the term "inserted HRA region" or domain refers to an HRA region that is present in an F polypeptide at a different position than the HRA region in the amino acid sequence of the F0 form of the naturally occurring F protein. For example, an RSVF polypeptide can contain an inserted HRA region, for example that is located carboxy terminally (i.e., C-terminal) to the endogenous HRA region and the HRB region, and proximal to the endogenous HRB region. In certain embodiments, the inserted HRA region is a full length HRA region. However, mutations, deletions, and truncations of the HRA region are included within the scope of the invention as long as such deletions and truncations do not substantially alter the ability of the HRA domain to form an alpha-helix which can subsequently assemble into a six helix bundle. The sequence of the inserted HRA region is selected independently from the sequence of the endogenous HRA region.

Truncated HRA domains can be truncated at either the C-terminus, the N-terminus, or both the C-terminus and N-terminus of the HRA domain. In certain embodiments, truncation comprises deletion of about 1-20, 1-15, 1-12, 1-11, 1-10, 1-9, 1-8, 1-7, 1-6, 1-5, 1-4, 1-3, 1-2, or 1 amino acids at one or both ends of the HRA domain. A truncated HRA preferably has at least 70%, 75%, 80%, 85%, 90%, or 95% identity over the full length of the domain and forms an alpha-helical structure to permit formation of a six helix bundle.

As used herein, the term "endogenous HRB region" or domain refers to an HRB region that is present in an F polypeptide at substantially the same position as the HRB region in the amino acid sequence of the F0 form of the naturally occurring F protein. In the case of RSVF proteins, such as an RSVF ectodomain polypeptide or recombinant RSVF ectodomain polypeptide, the endogenous HRB region is from about amino acid 474 to about amino acid 525. Amino acid numbering is based on the sequence of wild-type A2 strain of RSVF (SEQ ID NO: 1) including the signal peptide, and amino acid positions are assigned to residues that are deleted. For example, if the fusion peptide of RSVF is deleted in whole or in part, the deleted amino acids would be numbered so that the amino acids of HRB region have the same position numbers as in the wild-type sequence. In certain embodiments, the endogenous HRB region is a full length HRB region. However, deletions and truncations of the HRB region are included within the scope of the invention as long as such deletions and truncations do not substantially alter the ability of RSVF to fold into an acceptable conformation (either pre-fusion or post-fusion) for its intended purpose. An endogenous HRB region can include one or more mutations such as those discussed herein.

As used herein, the term "inserted HRB region" or domain refers to an HRB region that is present in a F polypeptide at a different position than the HRB region in the amino acid sequence of the F0 form of the naturally occurring F protein. For example, an RSVF polypeptide can contain an inserted HRB region, for example that is located carboxy terminally to the endogenous HRA region and the endogenous HRB region, and proximal to the endogenous HRB region. In certain embodiments, the inserted HRB region is a full length HRB region. However, mutations, deletions, and truncations of the HRB region are included within the scope of the invention as long as such deletions and truncations do not substantially alter the ability of the HRB domain to form an alpha-helix which can subsequently assemble into a six helix bundle. The sequence of the inserted HRB region is selected independently from the sequence of the endogenous HRB region.

Truncated HRB domains can be truncated at either the C-terminus, the N-terminus, or both the C-terminus and N-terminus of the HRB domain. In certain embodiments, truncation comprises deletion of about 1-20, 1-15, 1-12, 1-11, 1-10, 1-9, 1-8, 1-7, 1-6, 1-5, 1-4, 1-3, 1-2, or 1 amino acids at one or both ends of the HRB domain. In a preferred embodiment, the HRB domain is truncated on the C-terminal end. A truncated HRB preferably has at least 70%, 75%, 80%, 85%, 90%, or 95% identity over the full length of the domain and forms an alpha-helical structure to permit formation of a six helix bundle.

As used herein, "RSVF ectodomain polypeptide" refers to an RSVF polypeptide that contains substantially the extracellular portion of mature RSVF protein, with or without the signal peptide (e.g., about amino acid 1 to about amino acid 524, or about amino acid 22 to about amino acid 524) but lacks the transmembrane domain and cytoplasmic tail of naturally occurring RSVF protein. The RSVF ectodomain polypeptide comprises an endogenous HRA domain and an endogenous HRB domain. In certain embodiments, an RSVF ectodomain polypeptide includes a truncated endogenous HRB domain. RSVF ectodomain polypeptides of the invention further include an inserted 6-helix bundle forming domain, e.g., a 6-helix bundle forming domain from a class 1 fusion viral protein, including, but not limited to HIV gp41 HR1 region, an HIV gp41 HR2 region, Newcastle disease virus (NDV) HR1/HR2, human metapneumovirus HR1/HR2. Most typically a 6-helix bundle forming domain from a class 1 fusion viral protein is an inserted RSVF HRA domain or an inserted RSVF HRB domain.

As used herein, "cleaved RSVF ecto-domain polypeptide" refers to a RSVF ectodomain polypeptide that has been cleaved at one or more positions from about 101/102 to about 160/161 to produce two subunits, in which one of the subunits comprises F₁ and the other subunit comprises F₂. In cetain embodiments, all or a portion of the sequence between amino acids 101 to 161, e.g., all or a portion of the sequence between amino acids 101 and 132, for example, all or a portion of the sequence between amino acids 109 and 133 is removed from the RSVF ectodomain polypeptide such that a single cleavage results in a cleaved ectodomain polypeptide that does not include most or all of the p27 sequence. As used herein, cleavage of a complex to remove a purification tag is not understood as a cleaved RSVF ecto-domain polypeptide. The amount of cleaved polypeptide in a sample can be determined, for example, by western blot or SEC and can be expressed as a percent by weight.

As used herein, "uncleaved RSVF ectodomain polypeptide" refers to an RSVF ectodomain polypeptide that is not cleaved at one or more positions from about 101/102 to about 160/161. An uncleaved RSVF ectodomain polypeptide can be, for example, a monomer or a trimer.

As used herein, a "purified" protein, polypeptide, or complex is a protein, polypeptide, or complex which is recombinantly or synthetically produced, or produced by its natural host, and has been isolated from other components of the recombinant or synthetic production system or natural host such that the amount of the protein relative to other macromolecular components present in a composition is substantially higher than that present in a crude preparation or the amount of the desired complex present relative to the amount of the components not present in the desired complex, i.e., monomers, improperly assembled complexes. In general, a purified protein will comprise at least about 50% of the protein in the preparation and more preferably at least about 75%, at least about 80%, at least about 85%, at least about 90%, at least about 95% of the protein in the preparation by weight.

As used herein, "substantially free of lipids and lipoproteins" refers to compositions, proteins and polypeptides that are at least about 95% (e.g., at least 96%, at least 97%, at least 98%, at least 99%) free of lipids and lipoproteins on a mass basis when protein and/or polypeptide (e.g., RSVF polypeptide) purity is observed on an SDS PAGE gel and total protein content is measured using either UV280 absorption or BCA analysis, and lipid and lipoprotein content is determined using the Phospholipase C assay (Wako, code no. 433-36201).

As used herein, "altered furin cleavage site" refers to the amino acid sequence at about positions 106-109 or at about positions 133-136 in naturally occurring RSVF protein that are recognized and cleaved by furin or furin-like proteases, but in an uncleaved RSVF protein ecto-domain polypeptide contains one or more amino acid replacements, one or more amino acid deletions, or a combination of one or more amino acid replacement and one or more amino acid deletion, so that an RSVF ecto-domain polypeptide that contains an altered furin cleavage site is secreted from a cell that produces it uncleaved at the altered furin cleavage site at one or both of the furin cleavage sites present in RSVF. As used herein, an altered furin cleavage site is an inactivated furin cleavage site.

As use herein, an "optimized furin cleavage site" is a peptide cleavage site that includes selection of the optimal furin consensus sequence (e.g., RARR (SEQ ID NO: 109) and RKRR (SEQ ID NO: 110)) for the specific furin cleavage site within the context of the protein in which the site is present. Optionally an optimized furin cleavage site further includes one or more mutations (i.e., substitution, insertion, or deletion) to the native RSV sequence adjacent to the furin cleavage site to promote furin cleavage, e.g., insertion of a serine and optionally a glycine immediately C-terminal to the final R in the furin cleavage site, or substitution of the native amino acids phenylalaine with a serine, and optionally substitution of the native leucine with a glycine immediately C-terminal to the furin cleavage site. In a preferred embodiment, an optimized furin cleavage site is cleaved more efficiently, e.g., more completely than a native furin cleavage site at a comparable position in RSVF. In the instant invention, an optimized cleavage site can include, for example, the sequence RKRR immediately followed by a serine wherein the S is preferably an F137S mutation in the RSVF protein.

As used herein, "oligomerization polypeptide" refers to a polypeptide or polypeptide conjugate that may be a separate molecule from the RSVF polypeptides described herein, and that preferably contains one oligomerization domain, but may contain two or three oligomerization domains, and optionally a functional region. The oligomerization region contains an amino acid sequence that can form a six-helix bundle with the inserted domain of the RSVF ectodomain polypeptide. For example, the oligomerization polypeptide can comprise a 6-helix bundle forming domain from a class 1 fusion viral protein, e.g., an HRA domain, an HRB domain, an HR1 domain, or an HR2 domain. In certain embodiments, the oligomerization polypeptide comprises an RSVF HRB amino acid sequence, it can form a six-helix bundle with the inserted HRA region of a RSVF polypeptide. Alternatively, when the oligomerization polypeptide comprises an RSVF HRA amino acid sequence, it can form a six-helix bundle with the inserted HRB region of a RSVF polypeptide. In certain embodiments, the oligomerization polypeptide is operably linked to the inserted HRA or inserted HRB domain. In certain embodiments, the oligomerization polypeptide is not operably linked to the inserted HRA domain or the inserted HRB domain. When the oligomerization polypeptide contains an oligomerization region and a functional region, the two regions are operably linked so that the oligomerization region can form a six helix bundle with the RSVF ectodomain polypeptide and the functional region retains the desired functional activity.

As used herein, "C-terminal 6-helix bundle forming moiety" or "C-terminal 6-helix bundle forming domain" refers to a portion of a recombinant RSVF ectodomain polypeptide that can form a six-helix bundle and is 1) located C-terminal to the endogenous HRB region of naturally occurring RSVF protein, and 2) is not found in that location in naturally occurring RSVF protein. In one example, the C-terminal 6-helix bundle forming moiety is an HRA region of RSVF that is inserted C-terminally of the endogenous HRB region of RSVF, with or without the use of a linker sequence. A C-terminal 6-helix bundle forming moiety can form a six-helix bundle with one or more oligomerization polypeptides.

As used herein, "linker" is understood as a chemical moiety that joins two molecules, e.g., peptide molecules. In certain embodiments, the linker is a non-covalent linker, e.g., avidin and biotin; antibody and antigen. In certain embodiments, the linker is a covalent linker, e.g., peptide bond, a chemical cross-linking agent. In certain embodiment, peptide bond linkers can include the insertion of additional amino acids between the peptides to be joined. In certain embodiments, the linker can be structurally constrained. In certain embodiments structurally constrained linkers include a Pro-Gly sequence to induce a bend, e.g., GPGA (SEQ ID NO: 111) sequences. In certain embodiments, the structurally contstrained linkers can comprise alpha-helical peptides. In certain embodiments, linker peptides comprise flexible (i.e., non-structurally constrained) peptides. In certain embodiments, the linkers are based on RSV sequences. In certain embodiments, the linkers are not based on RSV sequences. Linkers can be of any length. Preferred linkers are about 3-30, 3-21, 3-15, 3-10, 4-8, or 5-7 amino acids in length. When multiple linkers are present, the nature and length of each linker is selected independently.

Linkers are used to "operably link" or simply "link" molecules together. It is understood that the presence of a linker does not substantially inhibit, and optionally promotes the desired activity of the molecules that it joins. For example, a peptide linker between an inserted HRA region and an oligomerization domain comprising an HRB region preferably promotes pairing of the inserted HRA region and the HRB region in the oligomerization domain HRB region to permit formation of a six-helix bundle and/or inhibits the pairing of the inserted HRA region or HRA region in an oligomerization domain with the endogenous HRB region; and/or inhibits the pairing of the inserted HRB region or the HRB region in an oligomerization domain with the endogenous HRA region.

Features of RSVF protein ectodomains suitable for use in this invention are described herein with reference to particular amino acids that are identified by the position of the amino acid in the sequence of RSVF protein from the A2 strain (SEQ ID NO:1) unless otherwise clearly indicated. RSVF protein ectodomains can have the amino acid sequence of the F protein from the A2 strain or any other desired strain. When the F protein ectodomain from a strain other than the A2 strain is used, the amino acids of the F protein are to be numbered with reference to the numbering of the F protein from the A2 strain, with the insertion of gaps as needed. This can be achieved by aligning the sequence of any desired RSVF protein with the F protein of the strain A2, as shown herein for F proteins from the A2 strain, CP52 strain, B strain, long strain, and the 18537 strain. *See,* FIG. 2. Sequence alignments are preferably produced using the algorithm disclosed by Corpet, Nucleic Acids Research, 1998, 16(22):10881-10890 (incorporated herein by reference), using default parameters (Blossum 62 symbol comparison table, gap open penalty: 12, gap extension penalty: 2).

### RSVF glycoprotein

The F glycoprotein of RSV directs viral penetration by fusion between the virion envelope and the host cell plasma membrane. It is a type I single-pass integral membrane protein having four general domains: N-terminal ER-translocating signal sequence (SS), ectodomain (ED), transmembrane domain (TM), and a cytoplasmic tail (CT). CT contains a single palmitoylated cysteine residue. The sequence of F protein is highly conserved among RSV isolates, but is constantly evolving (Kim et al. (2007) J Med Virol 79: 820-828). Unlike most paramyxoviruses, the F protein in RSV can mediate entry and syncytium formation independent of the other viral proteins (HN is usually necessary in addition to F in other paramyxoviruses).

The hRSVF mRNA is translated into a 574 amino acid precursor protein designated F₀, which contains a signal peptide sequence at the N-terminus that is removed by a signal peptidase in the endoplasmic reticulum. F₀ is cleaved at two sites (a.a. 109/110 and 136/137) by cellular proteases (in particular furin) in the trans-Golgi, removing a short glycosylated intervening sequence and generating two subunits designated F₁ (∼50 kDa; C-terminus; residues 137-574) and F₂ (∼20 kDa; N- terminus; residues 1-109) (See, *e.g.,* FIG. 1). F₁ contains a hydrophobic fusion peptide at its N-terminus and also two hydrophobic heptad-repeat regions (HRA and HRB). HRA is near the fusion peptide and HRB is near to the transmembrane domain (See, *e.g.,* FIG. 1). The F₁-F₂ heterodimers are assembled as homotrimers in the virion.

RSV exists as a single serotype but has two antigenic subgroups: A and B. The F glycoproteins of the two groups are about 90% identical in amino acid sequence. The A subgroup, the B subgroup, or a combination or hybrid of both can be used in the invention. An example sequence for the A subgroup is SEQ ID NO: 1 (A2 strain; GenBank GI: 138251; Swiss Prot P03420, incorporated by reference in the verison available on the date of filing the instant application), and for the B subgroup is SEQ ID NO: 2 (18537 strain; GI: 138250; Swiss Prot P13843, incorporated by reference in the verison available on the date of filing the instant application). SEQ ID NO:1 and SEQ ID NO:2 are both 574 amino acid sequences. The signal peptide in A2 strain is a.a. 1-21, but in 18537 strain it is 1-22. In both sequences the TM domain is from about a.a. 530-550, but has alternatively been reported as 525-548.

The invention may use any desired RSVF amino acid sequence, such as the amino acid sequence of SEQ ID NO: 1 or 2, or a sequence having identity to SEQ ID NO: 1 or 2. Typically it will have at least 75% identity to SEQ ID NO: 1 or 2 *e.g.,* at least 80%, at least 85%, at least 90%, at least 95%, at least 97%, at least 98%, at least 99%,identity to SEQ ID NO:1 or 2. The sequence may be found naturally in RSV.

Preferably an ectodomain of F protein, in whole or in part, is used, which may comprise:
(i) a polypeptide comprising about amino acid 22-525 of SEQ ID NO: 1;
(ii) a polypeptide comprising about amino acids 23-525 of SEQ ID NO: 2;
(iii) a polypeptide comprising an amino acid sequence having at least 75% identity (e.g., at least 80%, at least 85%, at least 90%, at least 95%, at least 97%, at least 98%, at least 99% identity) to (i) or (ii), preferably over the entire length of the polypeptide; or
(iv) a polypeptide comprising a fragment of (i), (ii) or (iii), wherein the fragment comprises at least one F protein epitope. The fragment will usually be at least about 100 amino acids long, *e.g.,* at least about 150, at least about 200, at least about 250, at least about 300, at least about 350, at least about 400, at least about 450 amino acids long.

The ectodomain can be an F₀ form with or without the signal peptide, or can comprises two separate peptide chains *(e.g.,* an F₁ subunit and a F₂ subunit) that are associated with each other, for example, the subunits may be linked by a disulfide bridge. Accordingly, all or a portion of about amino acid 101 to about 161, such as amino acids 110-136 (i.e., the p27 domain), may be absent from the ectodomain. Thus the ectodomain, in whole or in part, can comprise:
(v) a first peptide chain and a second peptide chain that is associated with the first polypeptide chain, where the first peptide chain comprises an amino acid sequence having at least 75% identity (e.g., at least 80%, at least 85%, at least 90%, at least 95%, at least 97%, at least 98%, at least 99%, or even 100% identity) to about amino acid 22 to about amino acid 101 of SEQ ID NO: 1 or to about amino acid 23 to about amino acid 101 of SEQ ID NO: 2, and the second peptide chain comprises an amino acid sequence having at least 75% identity (*e.g*., at least 80%, at least 85%, at least 90%, at least 95%, at least 97%, at least 98%, at least 99%, or even 100% identity) to about amino acid 162 to about 525 of SEQ ID NO: 1, or to about amino acid 137 to about 525 of SEQ ID NO: 1; or to about amino acid 162 to 525 of SEQ ID NO: 2, or to about amino acid 137 to about 525 of SEQ ID NO: 2;
(vi) a first peptide chain and a second peptide chain that is associated with the first polypeptide chain, where the first peptide chain comprises an amino acid sequence comprising a fragment of about amino acid 22 to about amino acid 101 of SEQ ID NO: 1 or of about amino acid 23 to about amino acid 109 of SEQ ID NO: 2, and the second peptide chain comprises a fragment of about amino acid 162 to about amino acid 525, or about amino acid 137 to about amino acid 525 of SEQ ID NO: 1 or of about amino acid 161 to about amino acid 525, or about amino acid 137 to about amino acid 525 of SEQ ID NO: 2. One or both of the fragments will comprise at least one F protein epitope. The fragment in the first peptide chain will usually be at least 20 amino acids long, *e.g.,* at least 30, at least 40, at least 50, at least 60, at least 70, at least 80 amino acids long. The fragment in the second peptide chain will usually be at least 100 amino acids long, *e.g.,* at least 150, at least 200, at least 250, at least 300, at least 350, at least 400, at least 450 amino acids long; or
(vii) a molecule obtainable by furin digestion of (i), (ii), (iii) or (iv).

Thus an amino acid sequence used with the invention may be found naturally within RSVF protein (e.g., a soluble RSVF protein lacking TM and CT, about amino acids 522-574 of SEQ ID NOS: 1 or 2), and/or it may have one or more *(e.g.,* 1, 2, 3, 4, 5, 6, 7, 8, 9, 10, 11, 12, 13, 14, 15, 16, 17, 18, 19, 20, 21, 22, 23, 24, 25, 26, 27, 28, 29, 30) single amino acid mutations (insertions, deletions or substitutions) relative to a natural RSV sequence. For instance, it is known to mutate F proteins to eliminate their furin cleavage sequences, thereby preventing intracellular processing. In certain embodiments, the RSVF protein lacks TM and CT (about amino acids 522-574 of SEQ ID NOS: 1 or 2) and contains one or more *(e.g.,* 1, 2, 3, 4, 5, 6, 7, 8, 9, 10, 11, 12, 13, 14, 15, 16, 17, 18, 19, 20, 21, 22, 23, 24, 25, 26, 27, 28, 29, 30) single amino acid mutations (insertions, deletions or substitutions) relative to a natural RSV sequence.

RSVF polypeptides or proteins may contain one or more mutations that prevent cleavage at one or both of the furin cleavage sites (*i.e*., amino acids 109 and 136 of SEQ ID NOS: 1 and 2). RSVF ectodomain polypeptides that contain such mutations are not cleaved in vivo by cells that produce the polypeptides and are produced as monomers. Examples of suitable furin cleavage mutations to disrupt furn cleavage include replacement of amino acid residues 106 - 109 of SEQ ID NO: 1 or 2 with RARK (SEQ ID NO: 32), RARQ (SEQ ID NO: 33), QAQN (SEQ ID NO: 34), or IEGR (SEQ ID NO: 35). Alternatively, or in addition, amino acid residues 133 - 136 of SEQ ID NO: 1 or 2 can be replaced with RKKK (SEQ ID NO: 36), R, QNQN (SEQ ID NO: 37), QQQR (SEQ ID NO: 38) or IEGR (SEQ ID NO: 35). (indicates that the amino acid residue has been deleted.) These mutations can be combined, if desired, with other mutations described herein or known in the art, such as mutations in the p27 region (amino acids 110-136 of SEQ ID NOS: 1 or 2), including deletion of the p27 region in whole or in part.

In certain embodiments, the amino acid sequence of an uncleaved RSVF protein ecto-domain is altered to prevent cleavage at the furin cleavage sites at about position 109/110 and about position 136/137, but contains a naturally occurring or inserted protease cleavage site, that when cleaved produce a F₁ subunit and a F₂ subunit (e.g., the p27 sequence is deleted so only a single cleavage is required to produce the F1 and F2 polypeptides). For example, the uncleaved RSVF protein ectodomain polypeptide can have an amino acid sequence that is altered to prevent cleavage at the furin cleavage sites at about position 109/110 and about position 136/137 (optionally with the intervening sequence deleted), but contain one or more naturally occurring or inserted protease cleavage sites from about position 101 to about position 161. Protease cleavage sites can include, but are not limited to TEV protease (tobacco etch virus) cleavage sites, Rhinovirus 3c protease cleavage sites, thrombin cleavage sites, trypsin cleavage sites, and furin cleavage sites. In certain embodiments, the FP domain is deleted at least in part and a furin cleavage domain is inserted. In certain embodiments, the RSVF protein ecto-domain does not contain an inserted protease cleavage site. In certain embodiments, the RSVF protein ecto-domain does not contain a furin cleavage site.

In certain embodiments, the RSVF protein ecto-domain contains a furin cleavage site. In certain embodiments, the furin cleavage site is an optimized furin cleavage site. In certain embodiments, the furin cleavage site is present in an RSVF ectodomain polypeptide of the invention in which the p27 sequence is deleted. In certain embodiments, the RSVF protein ecto-domain contains one furin cleavage site. In certain embodiments, the RSVF protein ecto-domain contains two furin cleavage sites.

A variety of particular amino acid sequences that will allow uncleaved RSVF protein ecto-domain polypeptides to be produced and expressed by host cells, including amino acid sequences that are not cleaved at the furin cleavage sites at about position 109/110 and about position 136/137 can be readily designed and envisioned by one of skill in the art. In general, one or more amino acids that are part of or are located nearby the furin cleavage sites at about position 109/110 and about position 136/137 are independently replaced or deleted. Some amino acid substitutions and deletions that are suitable to prevent cleavage of RSVF protein ecto-domain polypeptides are known. For example, the substitutions R108N, R109N, R108N/R109N, which inhibit cleavage at 109/110, and the substitution K131Q or the deletion of the amino acids at positions 131-134, which inhibit cleavage at 136/137, have been described Gonzalez-Reyes et al., Proc. Natl. Acad. Sci. USA, 98:9859-9864 (2001). An uncleaved RSVF ectodomain polypeptide that contains the amino acid substitutions R108N/R109N/K131Q/R133Q/R135Q/R136Q has been described. Ruiz-Arguello et al., J. Gen. Virol. 85:3677687 (2004). As described herein, additional RSVF protein amino acid sequences that result in the RSVF ecto-domain polypeptide being secreted from a host cell uncleaved contain altered furin cleavage sites, e.g., altered amino acid sequences at about positions 106-109 and at about positions 133-136. The altered furin cleavage sites contain at least one amino acid substitution or deletion at about positions 106-109, and at least one amino acid substitution or deletion at about positions 133-136 to prevent cleavage at the native furin cleavage site.

Similarly, a variety of particular amino acid sequences of uncleaved RSVF protein ectodomain polypeptides that contain a protease cleavage site (e.g., naturally occurring or inserted) that when cleaved produce a first subunit that comprises an F₁ and a second subunit that comprises F₂ can be readily designed and envisioned. For example, the amino acid sequence of RSVF protein from about position 101 to about position 161 contains trypsin cleavage sites, and one or more of the trypsin cleavage sites can be cleaved, e.g., *in vitro,* by trypsin to generate F₁ and F₂ subunits. If desired, one or more suitable protease recognition sites can be inserted into the uncleaved RSVF protein ecto-domain polypeptide, for example, between about positions 101 to about position 161. The inserted protease recognition sites can be cleaved using the appropriate protease to generate F₁ and F₂ subunits.

In particular embodiments, the sequence of amino acid residue 100 - 150 of the RSVF polypeptide or protein, such as SEQ ID NO:1, SEQ ID NO:2, or the soluble ecto domains thereof, is Furmt, (SEQ ID NO:3); Furdel (SEQ ID NO:4); Furx (SEQ ID NO:5); Furx R113Q, K123N, K124N (SEQ ID NO:6); Furx R113Q, K123Q, K124Q (SEQ ID NO:7); Delp21 Furx (SEQ ID NO:8); Delp23 Furx (SEQ ID NO:9); Delp21 furdel (SEQ ID NO: 10); Delp23 furdel (SEQ ID NO:11); Nterm Furin (SEQ ID NO:12); Cterm Furin (SEQ ID NO:13); Factor Xa (SEQ ID NO:14) as shown in Figure 1C; or SEQ ID NO:15 (from WO 2010/077717, incorporated herein by reference.).

In certain embodiments, RSVF polypeptides or proteins may contain one or more furin cleavage site optionally including mutations that promote cleavage at one or both of the furin cleavage sites (*i.e*., amino acids 109/110 and 136/137 of SEQ ID NOS: 1 and 2) or at positions corresponding to amino acids 109/110 and 136/137 of SEQ ID NOS: 1 and 2. In certain embodiments, the p27 region is deleted or substantially deleted (e.g., 10 or fewer consecutive amino acids of the p27 domain retained) while a single furin cleavage site is retained. In certain embodiments, the p27 region is retained wherein a single furin cleavage site is retained within or adjacent to the p27 region. In certain embodiments, the p27 region is retained wherein a single furin cleavage site is retained adjacent to the p27 region. In certain embodiments, a single furin cleavage site is retained adjacent to the p27 region. In certain embodiments, a furin cleavage site is optimized in the RSVF polypeptide. In certain embodiments, amino acids about 106 to about 132 are deleted, retaining the second furin cleavage site at amino acids 133-136. Further, the sequence is modified to insert a serine immediately adjacent to amino acid 136 either by insertion of a serine or by the substitution F137S to optimize the cleavage site.

### RSVF Complexes

The complexes contain an RSVF ectodomain trimer and are characterized by a six-helix bundle, with the proviso that the endogenous HRA and the endogenous HRB are not part of the six-helix bundle.

In one aspect, the complexes may contain an RSVF ectodomain trimer in the form of a complex that contains three RSVF ectodomain polypeptides and at least one oligomerization polypeptide. The oligomerization polypeptide contains an oligomerization region or moiety that can bind with inserted portions of RSVF ectodomain polypeptides to form a six-helix bundle. Thus, the complex contains a six-helix bundle that is formed by an inserted portion of the RSVF ectodomain polypeptides and all or a portion of the oligomerization polypeptides. In certain embodiments, the oligomerization polypeptide is operationally linked to an RSVF ectodomain polypeptide typically C-terminal to the inserted six-helix bundle forming domain which is attached C-terminal to the endogenous RSVF HRB domain. In certain embodiments, an oligomerization polypeptide is operationally linked to each RSVF ectodomain polypeptide. In certain embodiments, an oligomerization polypeptide includes multiple oligomerization domains. In certain embodiments, the oligomerization polypeptide further includes a functional domain (e.g., a purification tag, an antigen, especially a clinically relevant antigen).

The RSVF ectodomain further contains portions that are capable of forming a six-helix bundle. For example, when present the an inserted HRB region of an RSVF ectodomain polypeptide can form a six-helix bundle with an oligomerization polypeptide, either expressed in the same protein as the inserted six-helix bundle forming domain, e.g., an HRB region or expressed as a separate peptide, that contains the amino acid sequence of a complementary six-helix bundle forming moiety, e.g., the HRA region of RSVF.

If desired, one or more of the RSVF ectodomains present in the complexes described herein can be a recombinant RSVF ectodomain polypeptide that includes an inserted C-terminal 6-helix bundle forming moiety (e.g., both an inserted RSVF HRA region, optionally operably linked oligomerization domain comprising an RSVF HRB region). Such recombinant RSVF ectodomain polypeptides can be prepared using methods that are conventional in the art. The C-terminal 6-helix bundle forming moiety can be from RSVF, but is present at a C-terminal location that is different from (and in addition to) the location in which the moiety appears in naturally occurring RSVF. In one example, the C-terminal 6-helix bundle forming moiety is the HRA region of RSVF. Such a recombinant RSVF ectodomain polypeptide can form a six-helix bundle with an added oligomerization polypeptide that contains the amino acid sequence of the HRB region of RSVFRSVF. Alternatively, the C-terminal 6-helix bundle forming moiety can be an exogenous moiety that is obtained from a protein other than RSVF, such as the HR1 region of HIV gp41. Many six-helix bundle forming polypeptides are well known in the art, such as the heptad repeat regions (e.g., HRA and HRB) of Type I fusion proteins of enveloped viruses, such as RSVF, PIV and the like. See, e.g., Weissenhorm et al., FFBS Letters 581: 2150-2155 (2007), Table 1, which is hereby incorporated by reference.

The oligomerization polypeptide comprises an oligomerization region that can bind with an inserted amino acid 6-helix bundle forming domain sequence attached to the ectodomain of an RSVF polypeptide, e.g., a class 1 fusion viral protein 6-helix bundle forming domain, HIV gp41 HR1A region, an HIV gp41 HR2B region, Newcastle disease virus (NDV) HR1/HR2, HRA, HRB, or other inserted C-terminal 6-helix bundle forming moiety, and thereby promote six-helix bundle complex formation. Many suitable polypeptide sequences that are suitable for use as oligomerization regions are well known in the art, such as the heptad repeat regions (e.g., HRA and HRB) of the fusion proteins of enveloped viruses such as RSVF, PIV, and the like.

For example, when the RSVF ectodomain polypeptide further comprises an inserted HRB region, the oligomerization region can contain the amino acid sequence of anRSVFHRA region. Similarly, when the recombinant RSVF ectodomain polypeptide further comprises a C-terminal 6-helix bundle forming moiety that is the HRA region of RSVF or HR1 region of HIV gp41, for example, the oligomerization region can be the HRB region of RSVF or the HR2 region of HIV gp41, respectively.

If desired, the oligomerization polypeptide can further comprise a functional region that is operably linked to the oligomerization region. Suitable methods for producing operable linkages between a polypeptide (i.e., the oligomerization region) and a desired functional region, such as another polypeptide, a lipid, a synthetic polymer, are well known in the art. For example, the oligomerization polypeptide can be a polypeptide in which an amino acid sequence comprising the oligomerization region and an amino acid sequence comprising the functional region are components of a contiguous polypeptide chain, with or without an intervening linker sequence. In one embodiment, the oligomerization polypeptide can be expressed and purified as a fusion of the oligomerization peptide and the additional functional region. For example, the oligomerization polypeptide may comprise the RSVF HRB region and be fused to the RSV G central domain, with or without an intervening linker sequence. Additionally, two polypeptides or a polypeptide and another molecule (e.g., a lipid, a synthetic polymer) can be chemically conjugated directly or through a linker using a variety of known approaches. See, e.g., Hermanson, G. T., Bioconjugate Techniques, 2nd Edition, Academic Press, Inc. 2008, incorporated herein by reference.

Suitable functional regions include all or a portion of an immunogenic carrier protein, an antigen (e.g., a clinically relevant antigen, e.g., an immunogen to stimulate a respose to a pathogen), a particle forming polypeptide (e.g., viral particle or a non-infectious virus-like particle), a lipid, and polypeptides that can associate the oligomerization polypeptide with a liposome or particle (e.g., hydrophobic peptides, such as a transmembrane region, or a polypeptide that forms a coiled coil). When the functional region contains a portion of an immunogenic carrier protein, an antigen, a particle forming peptide, a lipid, or a polypeptide that can associate the oligomerization polypeptide with a liposome or particle, the portion that is contained is sufficient for the desired function. For example, when the oligomerization polypeptide contains a portion of an immunogenic carrier protein, the portion is sufficient to improve the immunogenicity of the RSVF complex. Similarly, when the oligomerization polypeptide contains a portion of an antigen, the portion is sufficient to induce an immune response.

Suitable immunogenic carrier proteins are well known in the art and include, for example, albumin, keyhole limpet hemocyanin, tetanus toxoid, diphtheria toxoid, CRM197, rEPA (nontoxic Pseudomonas aeruginosa ExoProteinA), non-typeable Haemophilus influenzae protein D (NTHiD), N19 polyepitope, and the like.

Suitable antigens are well known in the art and include any antigen from a pathogen (e.g., a viral, bacterial, or fungal pathogen). Exemplary antigens include, for example, RSV proteins such as RSVF and RSV G, HIV proteins such as HIV gp41, influenza proteins such as hemagglutinin, and paramyxovirus proteins such as the fusion protein of hPIV5, hPIV3, or Newcastle Disease virus.

Suitable particle forming peptides are well known in the art and include, for example, viral polypeptides that form viral particles, such as capsid proteins from nodavirus, norovirus, human papillomavirus (L1 and L2), parvovirus B 19 (VP 1 and VP2), hepatitis B virus (core protein), as well as monomers of self-assembling peptide nanoparticles, e.g., as described in Untied States Patent Application Publication No. 2011/0020378. In one embodiment, the oligomerizing polypeptide comprises an oligomerization region that is operably linked to a monomer of a self-assembling peptide nanoparticle as described in United States Patent Application Publication No. 2011/0020378 (incorporated herein by reference).

Suitable lipids are well known in the art and include, for example, fatty acids, sterols, mono-, di- and triglycerides, and phospholipids. Such lipids can anchor RSVF complexes that contain them to liposomes, membranes, oil in water emulsion droplets, and other structures. Exemplary lipids that can be used as a functional region of an oligomerization polypeptide include myristoyl, palmitoyl, glycophosphatidylinositol, pegylated lipids, neutral lipid, and nanodisks. Advantageously, myristoyl, palmitoyl, and glycophosphatidylinositol can be incorporated into the oligomerization polypeptide in vivo by expression of a construct that enclodes the oligomerization polypeptide in a suitable host cell.

A variety of suitable polypeptides that can associate the oligomerization polypeptide with a liposome or particle can be included in the oligomerization polypeptide and are well known in the art (see, e.g., WO2010/009277 and WO2010/009065, both incorporated herein by reference). For example, hydrophobic polypeptides e.g., a transmembrane region or a fusion peptide, that associate with or insert into liposomes or lipid nanoparticles can be used. Polypeptides that form a coiled coil can be used to link the oligomerization polypeptide to other structures that contain a coiled coil-forming peptide, e.g., a synthetic nanoparticle or liposome; viral polypeptides, or viral particles. In one embodiment, the oligomerizing polypeptide comprises an oligomerization region that is operably linked to coiled coil forming peptide that can bind the complex to a self-assembling peptide nanoparticle, as described in United States Patent Application Publication No. 2011/0020378.

In certain embodiments, the oligomerization peptide can further include an affinity tag to facilitate purification (e.g., 6X His (SEQ ID NO: 39), HAI, GST, Strep (SEQ ID NO: 108), Myc, etc). In certain embodiments, more than one copy of the tag is present (e.g., 2 copies, 3 copies, 4 copies). In certain embodiments, the tags are present in tandem. In certain embodiments, the tags are present in tandem with little or no intervening sequences. In certain embodiments, the affinity purification tag(s) can be removed by protease cleavage. In a preferred embodiment, removal of the tag by protease cleavage does not result in substantial cleavage of the RSVF protein, the inserted HRA or HRB domain, or the six helix bundle forming portion of the oligomerization polypeptide.

In some embodiments, the invention is a RSVF complex that contains three RSVF ectodomain polypeptides and three oligomerization polypeptides. The complex is characterized by a six-helix bundle formed, for example by an inserted HRB region of each of the three RSVF ectodomain polypeptides and all or a portion (i.e., the oligomerization region) of each of the three HRA containing oligomerization polypeptides.

In particular embodiments, the RSVF ectodomain polypeptides are recombinant and each comprises a C-terminal 6-helix bundle forming moiety. The complex in these embodiments is characterized by a six-helix bundle formed by the C-terminal 6-helix bundle forming moiety of each of the three RSVF ectodomain polypeptides and all or a portion (i.e., the oligomerization region) of each of the three oligomerization polypeptides.

In other aspects, the complex does not include an oligomerization polypeptide not linked to at least one of the ectodomain polypeptides. The complexes of this aspect contain three RSVF ectodomain polypeptides further comprising two paired domains of a C-terminal 6-helix bundle forming moiety, e.g., both an inserted HRA domain and an HRB domain as the oligomerization domain. The complex is characterized by a six-helix bundle that is formed by the C-terminal 6-helix bundle forming moiety linked to the endogenous portions of the RSVF ectodomain polypeptides. For example, such a complex can contain one, two or three recombinant RSVF ectodomain polypeptides that contain a first C-terminal 6-helix bundle forming moiety, such as an inserted RSVF HRA amino acid sequence, and the oligomerization domain which provides a second C-terminal 6-helix bundle forming moiety, such as an RSVF HRB amino acid sequence. The first C-terminal 6-helix bundle forming moiety (e.g., inserted HRA sequence) can form a six-helix bundle with the oligomerization domain which provides a second 6-helix bundle forming moiety (e.g., HRB sequence) region. Without wishing to be bound by any particular theory, it is believed that the first C-terminal 6-helix bundle forming moiety can fold back on the second six-helix bundle forming moiety and form the six-helix bundle in combination with the paired six helix bundles from two other RSV ectodomain polypeptides. Accordingly, in this aspect linker sequences can be included to limit interaction between the C-terminal 6-helix bundle with endogenous portions of the polypeptide and to promote interaction between the inserted C-terminal 6-helix bundle forming domain in the RSV ectodomain polypeptide and the 6-helix bundle forming domain in the oligomerization polypeptide to form the six-helix bundle that does not include the endogenous HRA or the endogenous HRB sequences. Further, it is understood that the 6-helix bundle forming moieties are appropriately arranged in the RSVF ectodomain polypeptides to permit pairing of the first and second 6-helix bundle forming moieties, and further to permit trimerization of the RSVF ectodomains through the paired 6-helix bundle forming moieties. Methods to detect trimer formation are well known in the art and provided herein (e.g., western blot using non-boiled, non reduced samples; size exclusion chromatography including size exclusion chromatography in conjunction with antibody binding). Although such methods do not permit direct observation of the six helix bundle, the formation of a trimer without significant formation of dimers or higher order structures as determined by western blot, can be understood to be an indication of the formation of a six helix bundle. In size exclusion chromatography, the formation of a complex of the appropriate mobility for a trimer peak at the appropriate position without a significant shoulder, peaks indicative of non-trimer forms, or significant flow through indicative of higher order structures. Appropriate controls to determine the mobility of trimers (e.g., DS-CAV1 with a foldon trimerization domain as provided in McClellan et al., 2013) and monomers (e.g., delta p23 furdel) can be readily identified by those of skill in the art. In a preferred embodiment, at least 60% of the RSVF ectodomain polypeptides (by weight) in a sample are present in a trimer. In a preferred embodiment, at least 70% of the RSVF ectodomain polypeptides (by weight) in a sample are present in a trimer. In a preferred embodiment, at least 75% of the RSVF ectodomain polypeptides (by weight) in a sample are present in a trimer. In a preferred embodiment, at least 80% of the RSVF ectodomain polypeptides (by weight) in a sample are present in a trimer. In a preferred embodiment, at least 85% of the RSVF ectodomain polypeptides (by weight) in a sample are present in a trimer. In a preferred embodiment, at least 90% of the RSVF ectodomain polypeptides (by weight) in a sample are present in a trimer. In a preferred embodiment, at least 95% of the RSVF ectodomain polypeptides (by weight) in a sample are present in a trimer. Methods such as western blot can be used to determine the amount of protein present in monomer and trimer forms. It is understood that western blots can have variations of about 3%, typically up to about 5%, or more. Those of skill in the art understand the use of proper control and standard samples to provide quantitative results, or results from western blots that would permit determination of the relative portion of the protein present in a monomer, trimer, or other form. Similarly, chromatography methods can be performed with appropriate controls to determine at least the relative amounts of protein in monomers, trimer, and other forms. Similarly, such methods can be used to determine the amount of cleaved protein in a mixture or to determine other characteristics of a population of RSVF proteins of the invention. It is understood that after formation of the trimers, purification steps such as those provided herein and known in the art can be used to achieve the required level of assembled trimers.

One or more of the RSVF ectodomain polypeptides in the complex can be an uncleaved RSVF ectodomain polypeptide, and the remaining can be a cleaved RSVF ectodomain polypeptide. In certain embodiments, each of the RSVF ectodomain polypeptides in the complex contains one or more altered furin cleavage sites. In certain embodiments, the RSVF ectodomain polypeptide comprises at least one furin cleavage site. In certain embodiments, the furin cleavage site is an optimized furin cleavage site.

In particular embodiments, the amino acid sequence of the RSVF ectodomain polypeptides comprises a sequence selected from the group consisting of: SEQ ID NO:8 (Del21 Furx), SEQ ID NO: 3 (Furmt), SEQ ID NO: 4 (Furdel), SEQ ID NO: 5 (Furx), SEQ ID NO: 6 (Furx R113Q, K123N, K124N), SEQ ID NO: 7 (Furx R113Q, K123Q, K124Q), SEQ ID NO:9 (Delp23Furx), SEQ ID NO: 10 (Delp21 furdel), SEQ ID NO: 11 (Delp23 furdel), SEQ ID NO: 12 (N-term Furin), SEQ ID NO: 13 (C-term Furin), SEQ ID NO: 14 (Factor Xa), SEQ ID NO: 15, SEQ ID NO: 26 (Fusion Peptide Deletion 1), and any of the foregoing in which the signal peptide and/or HIS tag, is omitted.

In more particular embodiments, the amino acid sequence of the RSVF ectodomain polypeptides is selected from the group consisting of: SEQ ID NO:8 (Del21 Furx), SEQ ID NO: 3 (Furmt), SEQ ID NO: 4 (Furdel), SEQ ID NO: 5 (Furx), SEQ ID NO: 6 (Furx R113Q, K123N, K124N), SEQ ID NO: 7 (Furx R113Q, K123Q, K124Q), SEQ ID NO:9 (Delp23Furx), SEQ ID NO: 10 (Delp21 furdel), SEQ ID NO: 11 (Delp23 furdel), and any of the foregoing in which the signal peptide and/or HIS tag, is omitted.

In further particular embodiments, the amino acid sequence of the RSVF ectodomain polypeptides corresponding to residues 100 - 150 of the wild-type RSVF polypeptide, such as SEQ ID NO:1 or SEQ ID NO:2, is selected from the group consisting of: SEQ ID NO:8 (Del21 Furx), SEQ ID NO: 3 (Furmt), SEQ ID NO: 4 (Furdel), SEQ ID NO: 5 (Furx), SEQ ID NO: 6 (Furx R113Q, K123N, K124N), SEQ ID NO: 7 (Furx R113Q, K123Q, K124Q), SEQ ID NO:9 (Delp23Furx), SEQ ID NO: 10 (Delp21 furdel), SEQ ID NO: 11 (Delp23 furdel), and any of the foregoing in which the signal peptide and/or HIS tag, is omitted.

In certain embodiments, the RSVF ectodomain polypeptide includes one, or at least one, furin cleavage site, which is optionally an optimized furin cleavage site. In a preferred embodiment, the furin cleavage site results in cleavage of the RSVF protein at a position such that the resulting peptides are similar in sequence to wild-type F1 and F2 domains. In a preferred embodiment, cleavage results in the production of peptides that are at least 80% identical to the sequence of the native RSVF peptide over the full length of the F1 and F2 domains. In certain embodiments, the identity of each of the F1 and F2 domains is independently selected from at least 85% identical, at least 90% identical, at least 95% identical, at least 98% identical, at least 99% identical, or is identical to the sequences of the wild-type RSVF sequences. In certain embodiments, alterations in the RSVF 1 and RSVF2 sequences are different from the wild-type sequence within the furin cleavage site. In certain embodiments, alterations in the RSVF1 and RSVF2 sequences are different from the wild-type sequence within and/or adjacent to the furin cleavage site (e.g., within 3 aa, within 2 aa, or directly adjacent to the furin cleavage site). In certain embodiments, the RSVF ectodomains of the invention contain mutations to promote or stabilize the ectodomain in the prefusion conformation. For example, the ectodomains can include mutaitons to promote the formation of disulfide bonds, for example, S155C and S290C mutations. Alternatively, or preferably in conjunction, the ectodomains contain one, or preferably two mutations at amino acids 190 and 207, preferably S190F and V207L to further promote stabilization of the ectodomain polypeptide in the prefusion conformation.

In certain embodiments, a single furin cleavage site, either having the native sequence or having an optimized sequence, is present at a position of a furin cleavage site in the RSVF ectocomain. In certain embodiments, the sequence of the p27 domain is absent from the RSVF ectodomain such that cleavage results in the formation of an F1 and an F2 domain similar in length to the wild-type sequences. In certain embodiments, the sequence of the p27 domain is retained in the RSVF domain such that cleavage at a single furin cleavage site at about amino acid 109 results in the formation of an F1 domain comparable to the wild-type F1 domain and an F2 domain with the p27 sequence attached to the N-terminus. Alternatively, in certain embodiments, the cleavage at a single furin cleavage site at about amino acid 136 results in the formation of an F1 domain with the p27 sequence attached to the C-terminus of the F1 domain and an F2 domain comparable to the wild-type F2 domain. The use of protease cleavage sites for proteases not expressed in cells used for expression of the RSVF ectodomains would be used in methods in which cleavage of the trimers was performed outside of the cells (on trimers assembled inside or outside of cells). The use of furin cleavage sites permits the cleavage of the RSVF ectodomain polypeptides in the cells during expression and assembly of the RSVF trimers of the invention.

In certain embodiments, the furin cleavage site or the optimized furin cleavage site in the embodiments above is replaced with a cleavage site for an alternative protease. For example, a furin cleavage site can be replaced with a protease cleavage site such as, but not limited to, trypsin, TEV protease, Rhinovirus 3c protease, and thrombin. In a preferred embodiment, the protease used in place of furin does not result in cleavage of the RSVF ectodomain polypeptide at sites other than positions corresponding to the native furin cleavage site(s) and does not result in cleavage of the oligomerization peptide.

In particular embodiments, the amino acid sequence of the oligomerization polypeptide is selected from the group consisting of: SEQ ID NO: 16 (RSV HRA, an oligmerization peptide of HRA), SEQ ID NO:17 (HRA_short, an oligomerization peptide that is slightly shorter than RSV HRA, SEQ ID NO:16), or any of the forgoing in which the GST sequence, cleavage sequence and/or linker sequence is omitted. In SEQ ID NO:16, amino acids 1-237 are glutathione S-transferasse (GST), amino acids 238-245 are a cleavage sequence, amino acids 246-252 are a linker, and amino acids 253-300 are HRA. In SEQ ID NO:17, amino acids 1-237 are glutathione S-transferasse (GST), amino acids 238-245 are a cleavage sequence, amino acids 246-252 are a linker, and amino acids 253-290 are HRA. It is understood that HRB sequences can be exchanged for the HRA sequences for production of HRB oligomerization domains. Further it s understood that alternative purification tags (e.g., strep, 6 X HIS) can be used in place of the exemplified GST tag.

In particular embodiments, the RSVF complex contains an RSVF ectodomain polypeptide and an oligomerization polypeptide that includes a functional region, such as an antigen. For example, the oligomeriztion polypeptide can comprise the amino acid sequence SEQ ID NO:18 (RSV Gb CC HRA short, in which an HRA oligomerization sequence is fused to the central domain of RSV G from strain b), SEQ ID NO:19 (RSV Ga CC HRA short, in which an HRA oligomerization sequence is fused to the central domain of RSV G from strain a), SEQ ID NO:20 (RSV Gb CC HRB, in which an HRB oligomerization sequence is fused to the central domain of RSV G from strain b), SEQ ID NO:21 (RSV Ga CC HRB, in which an HRB oligomerization sequence is fused to the central domain of RSV G from strain a), or any of the foregoing in which the glutathione S-transferase (GST) sequence, cleavage sequence and/or amino terminal linker sequence is omitted, and/or the GST sequence is replaced with an alternate purification tag (e.g., strep, 6 X HIS). In SEQ ID NOS 18 and 19, amino acids 1-237 are a GST sequence, amino acids 238-245 are a protease cleavage site, amino acids 246-252 are a linker sequence, amino acids 253-292 are a Gb or a Ga sequence, respectively, amino acids 293-299 are a second linker sequence, and amino acids 300-337 are an HRA sequence. In SEQ ID NOS: 20 and 21, amino acids 1-233 are a GST sequence, amino acids 234-241 are a protease cleavage site, amino acids 242-248 are a linker sequence, amino acids 249-288 are a Ga or a Gb sequence, respectively, amino acids 289-295 are a second linker sequence, and amino acids 296-329 are an HRB sequence.

In other particular embodiments, the RSVF complex comprises an RSVF ectodomain construct selected from the group consisting of SEQ ID NO:22 (RSVF delP23 furdel Truncated HRA HIS), or SEQ ID NO:23 (RSVF delP23 furdel C509C510 C481C489 HRA HIS); or any one of the foregoing in which the HIS tag and/or linker are omitted. In SEQ ID NOS:22-23 amino acids 1-495 are an RSVF ectodomain sequence, amino acids 496-533 are an inserted C-terminal HRA sequence, amino acids 534-540 are a linker sequence, and amino acids 541-546 are a the HIS tag sequence. SEQ ID NO:23 also includes introduced cysteines at positions 481, 489, 509 and 510.

In preferred embodiments, the RSVF ectodomain polypeptides in the complex are in the pre-fusion conformation. Without wishing to be bound by any particular theory, it is believed that the prefusion form of the RSVF trimer is stabilized in the complexes described herein because the inserted 6-helix bundle forming domains and the oligomerization domains induce complex formation and prevent the endogenous HRA and endogenous HRB regions of the RSVF protein from interacting with each other. The interaction of the endogenous HRA and endogenous HRB region of the RSVF protein leads to refolding into the postfusion form.

In other preferred embodiments, the complex is characterized by a rounded (pre-fusion) shape when viewed in negatively stained electron micrographs.

In other embodiments, the complex is characaterized by the ability to bind the D25 antibody which has been characterized as binding to the prefusion structure of RSVF.

In other preferred embodiments, the complex comprises prefusion epitopes that are not present on postfusion forms of RSVF.

Optionally, additional cysteine residues may be inserted into the HRB region to form disulfide bonds and further stabilize the RSVF ectodomains described herein.

In certain embodiments, the RSVF ectodomain polypeptide includes an S155C mutation and a S290C mutation.

In certain embodiments, the RSVF ectodomain polypeptide includes a mutation at amino acid 190 or amino acid 207. In certain embodiments, the RSVF ectodomains include a S190F mutation and/or a V207F mutation.

In certain embodiments, the RSVF ectodomain polypeptide includes an S155C mutation, a S290C mutation, an S190F mutation, and a V207F mutation.

In certain embodiments, the RSVF ectodomain polypeptide further includes an internal deletion of at least the p27 sequence. In certain embodiments, the RSVF ectodomain includes an internal deletion of about amino acid 103 to about amino acid 136, or about amino acid 103 to 161. In certain embodiments, the RSVF ectodomain polypeptide comprises the RSVF sequences of the DS-CAV1 (McClellan et al., 2013).

In certain embodiments, the RSVF complex may be further stabilized in the prefusion form using interchain disulfides including those disclosed in WO 2012/158613, incorporated herein by reference in its entirety, using peptides conjugated to oligomerizing agents including but not limited to virus-like particles (VLP's), albumin or RSV G, or using other mutations which further stabilize the monomer so that it retains its prefusion conformation upon formulation and immunization.

In certain embodiments, the RSVF complex may be further stabilized in the prefusion form using disulfide bonds or cavity filling mutations such as disclosed in RSVFMcLellan, JS et al., Science, 342(6158):592-8(2013) (incorporated herein by reference).

### Methods for Preparing Complexes

The invention also relates to methods for producing the RSVF complexes described herein. In one aspect, the invention relates to methods for producing a RSVF complex that comprises three RSVF ectodomain polypeptides each further comprising one of an inserted HRA domain or inserted HRB domain, three oligomerization domains present in one or more polypeptides, and is characterized by a six-helix bundle. The method includes a) providing RSVF ectodomain polypeptides each further comprising an inserted HRA domain or inserted HRB domain and at least one oligomerization polypeptide, and b) combining the RSVF ectodomain polypeptides further comprising an inserted HRA domain or inserted HRB domain and the oligomerization polypeptide(s) under conditions suitable for the formation of an RSVF complex, whereby a RSVF complex is produced that comprises three RSVF ectodomain polypeptides each comprising one of an inserted HRA domain or inserted HRB domain, three oligomerization domains present in one or more polypeptides, and is characterized by a six-helix bundle. As described herein, the six-helix bundle is formed by the inserted HRA domain or inserted HRB domain portion of each of the RSVF ectodomain polypeptides and all or a portion of the oligomerization polypeptides wherein the six-helix bundle does not include the endogenous RSVF HRA domain or the endogenous RSVF HRB domain.

If desired, one or more of the RSVF ectodomain polypeptides can be a recombinant RSVF ectodomain polypeptide that includes an inserted C-terminal 6-helix bundle forming moiety e.g., a heptad repeat region from a class 1 fusion viral protein that contain 6-helix bundles, such as the HRA region of RSVF or the HR1 region of HIV gp41, for example. In this practice of the method, the oligomerization polypeptide comprises an oligomerization region that can bind with an inserted portion of the RSVF ectodomain polypeptide further comprising a 6-helix bundle forming moiety, e.g., HRB region of RSVF or the HR1 region of HIV gp41, or a complementary 6-helix bundle forming moiety from a class 1 fusion viral proteins that contain 6-helix bundles, and thereby promote complex formation.

Optionally, the method can further comprise the step c) cleaving the RSVF protein ectodomain polypeptides (within a portion of the protein corresponding to the native RSVF ectodomain sequence) in the produced complex with a suitable protease, whereby a RSVF complex is produced that comprises three cleaved RSVF ectodomain polypeptides each further comprising an inserted C-terminal 6-helix bundle forming moiety, and the oligomerization polypeptide(s), which may or may not be linked to the RSVF ectodomain polypeptides, and is characterized by a six-helix bundle.

It is understood that when the complexes are expressed and assembled in the cells, the order of the steps is not directly controlled and occurs by normal celluar processes. Therefore, the order of the steps is not binding.

In certain embodiments, when the RSVF ectodomain polypeptide and/or the oligomerization peptide include a purification tag. In certain embodiments, typically after assembly and at least partial purification of the trimer, the purification tag can be removed from the RSVF complex by protease cleavage after assembly of the complex.

### Complex assembly from purified components

Complexes of the invention can be formed from purified components. The complex formed by combining three RSVF ectodomain polypeptides each further comprising an inserted C-terminal 6-helix bundle forming moiety; and at least one, preferably three, oligomerization polypeptides, which may or may not be linked to the RSVF ectodomain polypeptides. When linked to the ectodomain polypeptides, the oligomerization domains are present at a 1:1 ratio with the ectodomain polypeptides. However, when not linked to the ectodomain polypeptides, excess oligomerization domains, typically with each domain present in a separate polypeptide can be used, and in practice 2-fold molar excess, 3-fold molar excess, 4-fold molar excess, 5-fold molar excess, 6-fold molar excess, 7-fold molar excess, 8-fold molar excess, 9-fold molar excess, 10-fold molar excess, or more of oligomerization domains can be used. The RSVF ectodomain polypeptides, each further comprising an inserted C-terminal 6-helix bundle forming moiety, and three oligomerization domains, typically in three polypeptides, are combined under suitable conditions for the formation of the RSVF complex. In certain embodiments, the RSVF ectodomain polypeptides and oligomerization polypeptides are combined in a buffered aqueous solution (e.g., pH about 5 to about 9). If desired, mild denaturing conditions can be used, such as, by including urea, small amounts of organic solvents or heat to mildly denature the RSVF ectodomain polypeptides and denaturing conditions removed (e.g., by dialysis or dilution) to promote refolding and assembly of the complexes.

Any suitable preparation of RSVF ectodomain polypeptides and oligomerization polypeptides can be used in the method.

The use of uncleaved RSVF ectodomain polypeptides in the method provides advantages. As described herein, it has been discovered that cleavage of RSVF polypeptides *in vivo* of native RSVF ectodomains results in production of post-fusion ectodomains that are hydrophobic and frequently aggregatedmaking them difficult to purify. Cleavage *in vivo* of RSVF polypeptides with engineered features designed to stabilize the pre-fusion form often results in poor yields or unprocessed/misfolded RSVF proteins. However, RSVF ectodomain polypeptides that are not cleaved *in vivo* are produced in good yield as monomers and when the fusion peptide is altered in these ectodomain polypeptides the protein can be soluble and not aggregated (see, e.g., WO2011008974, incorporated herein by reference). The uncleaved monomers can be conveniently purified and used in the method to produce RSVF complexes. Thus, the purified RSVF ectodomain polypeptide monomers provided herein comprise an inserted C-terminal 6-helix bundle forming moiety for use in the complex assembly methods provided herein. The RSVF ectodomain polypeptides that are provided and used in the method are preferably uncleaved RSVF ectodomain polypeptides further comprising an inserted C-terminal 6-helix bundle forming moiety, and in certain embodiments, the uncleaved RSVF ectodomain polypeptides contain altered (i.e., inactivated) furin cleavage sites. In certain embodiments, the ectodomains include furin cleavage sites. In certain embodiments, the furin cleavage site is an optimized furin cleavage site. In certain embodiments, the amino acid sequence of the RSVF ectodomain polypeptides that are provided and used in the method comprises a sequence selected from the group consisting of: SEQ ID NO:8 (Del21 Furx), SEQ ID NO: 3 (Furmt), SEQ ID NO: 4 (Furdel), SEQ ID NO: 5 (Furx), SEQ ID NO: 6 (Furx R113Q, K123N, K124N), SEQ ID NO: 7 (Furx R113Q, K123Q, K124Q), SEQ ID NO:9 (Delp23Furx), SEQ ID NO: 10 (Delp21 furdel), SEQ ID NO: 11 (Delp23 furdel), SEQ ID NO: 12 (N-term Furin), SEQ ID NO: 13 (C-term Furin), SEQ ID NO: 14 (Factor Xa), SEQ ID NO: 15, SEQ ID NO:26 (Fusion Peptide Deletion 1), and any of the foregoing in which the signal peptide and/or HIS tag and/or fusion peptide, is altered or omitted.

In certain embodiments, the amino acid sequence of the RSVF ectodomain polypeptides that are provided and used in the method is selected from the group consisting of: SEQ ID NO:8 (Del21 Furx), SEQ ID NO: 3 (Furmt), SEQ ID NO: 4 (Furdel), SEQ ID NO: 5 (Furx), SEQ ID NO: 6 (Furx R113Q, K123N, K124N), SEQ ID NO: 7 (Furx R113Q, K123Q, K124Q), SEQ ID NO: 9 (Delp23Furx), SEQ ID NO: 10 (Delp21 furdel), SEQ ID NO: 11 (Delp23 furdel), and any of the foregoing in which the signal peptide and/or HIS tag and/or fusion peptide, is altered or omitted.

In certain embodiments, the amino acid sequence of the RSVF ectodomain polypeptides (that are provided and used in the method) corresponding to residues 100 - 150 of the wild-type RSVF polypeptide, such as SEQ ID NO:1 or SEQ ID NO:2, is selected from the group consisting of: SEQ ID NO:8 (Del21 Furx), SEQ ID NO: 3 (Furmt), SEQ ID NO: 4 (Furdel), SEQ ID NO: 5 (Furx), SEQ ID NO: 6 (Furx R113Q, K123N, K124N), SEQ ID NO: 7 (Furx R113Q, K123Q, K124Q), SEQ ID NO:9 (Delp23Furx), SEQ ID NO: 10 (Delp21 furdel), SEQ ID NO: 11 (Delp23 furdel), and any of the foregoing in which the signal peptide and/or HIS tag and/or fusion peptide, is altered or omitted.

In certain embodiments, the RSVF ectodomain polypeptides further comprises S155C and S290C substitutions at positions corresponding to SEQ ID NO: 1. In certain embodiments, the RSVF ectodomain polypeptides comprise at least one an F substitution at amino acid 190 and/or an L substitution at amino acid 207. In certain embodiments, the amino acid sequence of the RSVF ectodomain polypeptides comprise S155C, S290C, S190F, and V207L substitutions. In certain embodiments, the RSVF ectodomain polypeptide has a deletion of most or all of the p27 domain. In certain embodiments, the RSVF ectodomain polypeptide has a deletion of about amino acid 101 to about amino acid 161. In certain embodiments, the RSVF ectodomain polypeptide as a deletion of about amino acids 106 to about amino acid 130, or about amino acids 103-130, or about amino acids 103-136.

### Assembly of complexes in cells

The RSVF ectodomain polypeptides (e.g., uncleaved RSVF ectodomain polypeptides) are typically prepared by expression in a recombinant host system by expression of recombinant constructs that encode the ectodomains in suitable recombinant host cells, although any suitable methods can be used. Preferable recombinant host cells are mammalian culture cells that can readily be transfected and/or in which stable lines can be generated for expression of the peptides of the invention. The specific transfection method is selected based on general considerations known in the art, for example, the type of cells to be transfected and the volume in which the transfection is performed. Preferred cell lines for use include 293 cells, CHO cells, and HEK cells. Other suitable recombinant host cells include, for example, insect cells (e.g., *Aedes aegypti, Autographa californica, Bombyx mori, Drosophila melanogaster, Spodoptera frugiperda,* and *Trichoplusia ni),* mammalian cells (e.g., human, non-human primate, horse, cow, sheep, dog, cat, and rodent (e.g., hamster), avian cells (e.g., chicken, duck, and geese), bacteria (e.g., *E. coli, Bacillus subtilis,* and *Streptococcus spp*.), yeast cells (e.g., *Saccharomyces cerevisiae, Candida albicans, Candida maltosa, Hansenualpolymorpha, Kluyveromyces fragilis, Kluyveromyces lactis, Pichia guillerimondii, Pichia pastoris, Schizosaccharomyces pombe* and *Yarrowia lipolytica*), Tetrahymena cells *(e.g., Tetrahymena thermophila)* or combinations thereof. Many suitable insect cells and mammalian cells are well known in the art. Suitable insect cells include, for example, Sf9 cells, Sf21 cells, Tn5 cells, Schneider S2 cells, and High Five cells (a clonal isolate derived from the parental Trichoplusia ni BTI-TN-5B1-4 cell line (Invitrogen® )). Suitable mammalian cells include, for example, Chinese hamster ovary (CHO) cells, human embryonic kidney cells (HEK293 cells, typically transformed by sheared adenovirus type 5 DNA), NIH-3T3 cells, 293-T cells, Vero cells, HeLa cells, PERC.6 cells (ECACC deposit number 96022940), Hep G2 cells, MRC-5 (ATCC® CCL-171), WI-38 (ATCC® CCL-75), fetal rhesus lung cells (ATCC® CL-160), Madin-Darby bovine kidney ("MDBK") cells, Madin-Darby canine kidney ("MDCK") cells (e.g., MDCK (NBL2), ATCC® CCL34; or MDCK 33016, DSM ACC 2219), baby hamster kidney (BHK) cells, such as BHK21-F, HKCC cells, and the like. Suitable avian cells include, for example, chicken embryonic stem cells (e.g., EBx® cells), chicken embryonic fibroblasts, chicken embryonic germ cells, duck cells (e.g., AGE1.CR and AGE1.CR.pIX cell lines (ProBioGen®) which are described, for example, in Vaccine 27:4975-4982 (2009) and WO2005/042728), EB66 cells, and the like.

Suitable insect cell expression systems, such as baculovirus systems, are known to those of skill in the art and described in, *e.g.,* Summers and Smith, *Texas Agricultural Experiment Station Bulletin* No. 1555 (1987). Materials and methods for baculovirus/insert cell expression systems are commercially available in kit form from, *inter alia,* Invitrogen®, San Diego CA. Avian cell expression systems are also known to those of skill in the art and described in, *e.g.,* U.S. Patent Nos. 5,340,740; 5,656,479; 5,830,510; 6,114,168; and 6,500,668; European Patent No. EP 0787180B; European Patent Application No. EP03291813.8 ;WO 03/043415; and WO 03/076601. Similarly, bacterial and mammalian cell expression systems are also known in the art and described in, *e.g.,* Yeast Genetic Engineering (Barr et al., eds., 1989) Butterworths, London.

Recombinant constructs encoding RSVF protein ectodomains can be prepared in suitable vectors using conventional methods. A number of suitable vectors for expression of recombinant proteins in insect or mammalian cells are well known and conventional in the art. Suitable vectors can contain a number of components, including, but not limited to one or more of the following: an origin of replication; a selectable marker gene; one or more expression control elements, such as a transcriptional control element (e.g., a promoter, an enhancer, a terminator), and/or one or more translation signals; and a signal sequence or leader sequence for targeting to the secretory pathway in a selected host cell (e.g., of mammalian origin or from a heterologous mammalian or non-mammalian species). For example, for expression in insect cells a suitable baculovirus expression vector, such as pFastBac® (Invitrogen®), is used to produce recombinant baculovirus particles. The baculovirus particles are amplified and used to infect insect cells to express recombinant protein. For expression in mammalian cells, a vector that will drive expression of the construct in the desired mammalian host cell (e.g., Chinese hamster ovary cells) is used.

The selection of expression constructs and promoters for use in making the complexes of the invention depend, for example, if the complex is to be assembled from two different components, e.g., an ectodomain polypeptide including one of an inserted HRA or HRB domain and a separate oligomerization polypeptide; or if the complex is to be assembled from a pool of ectodomain polypeptides that include, for example, both an inserted HRA domain and an attached oligomerization domain comprising an HRB domain. Expression constructs are known in the art. Expression constructs typically include, for example, promoter sequences to drive the expression of the coding sequence, origins of replication to allow for replication of the plasmid (either in bacteria or in mammalian cells), and optionally sequences to promote integration of the expression construct stably into a cell for expression. Expression vectors including sequences for integration into the host genome will typically include sequences for selection, e.g., antibiotic resistance genes.

### Transfection of coding sequences for expression of ectodomain polypeptides linked to oligomerization domains

In certain embodiments, the invention includes expression of ectodomain polypeptides including an endogenous RSVF HRA domain, an endogenous RSVF HRB domain, and an inserted HRA domain or an inserted HRB domain, wherein the ectodomain polypeptide is further operably linked to an oligomerization domain, i.e., an HRB domain when an inserted HRA domain is present in the ectodomain peptide, or an HRA domain when an inserted HRB domain is present in the ectodomain peptide. In a preferred embodiment, when the oligomerization domain is operalbly linked to the ectodomain polypeptide it is linked by co-translation of the ectodomain polypeptide and the oligomerization domain. Expression constructs containing such coding sequences can be generated for expression in the cell type of interest using methods and expression vectors known in the art.

### Co-transfection of coding sequences for ectodomain polypeptides and oligomerization polypeptides

Strategies for expression of two distinct polypeptides in a single cell are known in the art. For example, expression vectors encoding two distinct polypeptides can be co-transfected into cells. Co-transfection of expression vectors allows for manipulation of the ratio of expression constructs in a cell. As used herein, it is assumed that the ratio of the expression constructs transfected into the cell reflects the ratio of the constructs taken up by the cell. For the sake of simplicity, an expression construct for expression of an ectodomain polypeptide is considered to have the same molecular weight as an expression vector for expression of an oligomerization domain. Therefore, transfection of 1 ug of an expression vector containing a sequence for expression of an ectodomain polypeptide with 1 ug of an expression vector containing a sequence for expression of a single oligomerization domain would be understood as a ratio of 1:1 ectodomain to oligomerization domain. Transfection of 1 ug of an expression vector containing a sequence for expression of an ectodomain polypeptide with 4 ug of an expression vector containing a sequence for expression of a single oligomerization domain would be understood as a ratio of 1:4 ectodomain to oligomerization domain. Other ratios for transfection of ectodomain polypeptide coding sequences to oligomerization polypeptide coding sequences include, but are not limited to 2:1 to 1:8. In preferred embodiments, the coding sequences for the oligomerization peptide are present in at least the same amount as the coding sequence for ectodomain polypeptides. In certain embodiments, the coding sequences for the oligomerization polypeptide are present in at least twice the amount as the coding sequence for ectodomain polypeptides. In certain embodiments, the coding sequences for the oligomerization polypeptide are present in at least three times the amount as the coding sequence for ectodomain polypeptides. In certain embodiments, the coding sequences for the oligomerization polypeptide are present in at least four times the amount as the coding sequence for ectodomain polypeptides. In certain embodiments, the coding sequences for the oligomerization polypeptide are present in at least five times the amount as the coding sequence for ectodomain polypeptides. In certain embodiments, the coding sequences for the oligomerization peptide are present in at least six times the amount as the coding sequence for ectodomain polypeptides. In certain embodiments, the coding sequences for the oligomerization polypeptide are present in at least seven times the amount as the coding sequence for ectodomain polypeptides. In certain embodiments, the coding sequences for the oligomerization peptide are present in at least eight times the amount as the coding sequence for ectodomain polypeptides.

In such a strategy where the ratio of the expression vectors containing the coding sequences for the different peptides is something other than 1:1, the expression vectors will typically include the same regulatory sequences for expression. Expression levels can also be modulated by selection of promoter sequences or other transcriptional translational regulatory sequences. Stronger promoter, enhancer, and/or replication sequences to increase the relative number of transcripts from one expression construct as compared another expression construct initially introduced into the cell at the same level. Such considerations are understood by those of skill in the art.

Coding sequences for two distinct polypeptides can be included in a single expression construct, for example, a construct including an IRES sequence. Bicistronic expression vectors for expression of two coding sequences are available, for example, from Clontech®. The use of such bicistronic vectors can facilitate the production of stable cell lines for expression of the complexes of the invention.

Without being bound by theory, it is proposed that the presence of an excess of oligomerization domains (when not operably linked to the ectodomain polypeptides) would favor the formation of complexes containing ectodomain polypeptides and oligomerization domains rather than complexes containing ectodomains alone.

### Leader sequences

Assembly of the RSVF complexes within cells takes place in the endoplasmic reticulum (ER) so that the complexes can be secreted from the cell. Therefore, coding sequences for the ectodomain polypeptide and separate oligomerization domain, when present, need to include an N-terminal leader sequence (also known as a signal sequence), preferably a cleavable N-terminal leader sequence, to promote transduction of the peptides into the ER. Known leader sequences include, but are not limited to, the native RSVF leader, the IgK leader, the serum albumin protein (SAP) leader, and the CD33 leader. An appropriate leader sequence can be selected based on routine considerations in the art such as the cell type in which the proteins are to be expressed.

In a preferred embodiment, the native, cleavable leader sequence of the RSVF ectodomain polypeptide is used. However, it is understood that other leader sequences, preferably cleavable leader sequences can be used.

When the oligomerization peptide is provided as a peptide separate from the ectodomain peptide, it also includes an N-terminal leader sequence, preferably a cleaved N-terminal leader sequence, to promote transduction into the ER. Leader sequences to promote transduction of the oligomerization peptide include, but are not limited to, the native RSVF leader, the IgK leader (SEQ ID NO: 107), the serum albumin protein (SAP) leader, and the CD33 leader. Commercial expression vectors can be purchased that contain leader sequences for directing proteins to the ER and secretion from the cell (e.g., pDisplay™ and pSecTag2 A, B, & C from Invitrogen®).

### Linker sequences

The ectodomain constructs of the invention typically include at least one linker, preferably a peptide linker, between the endogenous RSVF HRB sequence and the inserted HRA or HRB sequence, where the linker is preferably attached C-terminal to the endogenous RSVF HRB sequence and between the endogenous RSVF HRB sequence and the inserted six-helix bundle forming domain, e.g., an HRA or HRB domain (e.g., N-endogenous HRA-endogenous HRB-first linker-inserted HRA or HRB-C). The linker sequence can be of any length, preferably the linker is about 3-30 amino acids in length. In certain embodiments, the linker is about 5 to about 21 amino acids in length. In certain embodiments, the linker is about 10 to about 21 amino acids in length. In certain embodiments, this first linker sequence in the ectodomain polypeptide is an RSV sequence, preferably an RSVF sequence. Preferably, the linker is selected to favor interaction of the inserted HRA or HRB domain with the oligomerization peptide and disfavor interaction of the inserted HRA or HRB domain with the endogenous RSVF HRA and the endogenous RSVF HRB domains. Further, the linker sequence does not include sequences that would favor interaction of the endogenous HRA and HRB domains with the inserted domains or the oligomerization domains.

The ectodomain constructs of the invention may include a second linker sequence through which the oligomerization domain is operably linked C-terminally to the ectodomain polypeptide, preferably adjacent, and C-terminal to the inserted six-helix bundle forming moiety, e.g., an inserted HRA or HRB domain (e.g., N-endogenous HRA-endogenous HRB-first linker-inserted HRA or HRB-linker 2-oligomerization domain-C). The linker sequence can be of any length, preferably the linker is about 3-30 amino acids in length. In certain embodiments, the linker is about 5 to about 21 amino acids in length, and in certain embodiments, about 4-8 amino acids in length or 5-7 amino acids in length. In certain embodiments, this second linker sequence in the ectodomain polypeptide is an RSV sequence, preferably an RSVF sequence. Preferably, the linker is selected to favor interaction of the oligomerization domain with the inserted six-helix bundle forming moiety, e.g., an inserted HRA domain or the inserted HRB domain, and disfavor interaction of the oligomerization domain with the endogenous RSVF HRA and the endogenous RSVF HRB domains. Further, the linker sequence does not include sequences that would favor interaction of the inserted domain or the oligomerization domain with the endogenous RSVF HRA or the endogenous RSVF HRB domains. For example, the second linker, when present, the second linker can be a structurally constrained linker, for example a proline-glycine linker which promotes interaction between the inserted six-helix bundle forming moiety, e.g., an inserted HRA or inserted HRB sequence with the adjacent oligomerization domain.

### Purification tags

Purification tags can be used to facilitate purification of complexes of the invention and, in certain embodiments, for selection of appropriately assembled complexes of the invention. In ectodomain polypeptides, the purification tag is C-terminal to the inserted HRA or the inserted HRB domain when no oligomerization domain is linked to the ectodomain polypeptide. When the oligomerization peptide is not attached to the ectodomain polypeptide, the purification tag is N-terminal to the oligomerization domain sequence. In preferred embodiments with a separable oligomerization domain, if purification tags are present on both the ectodomain and the oligomerization domain, the purification tags are different. In a preferred embodiment with a separate oligomerization polypeptide, a purification tag is present only on the oligomerization polypeptide and not on the ectodomain polypeptide. Therefore, affinity purification selects for assembled complexes and single oligomerization domains which are substantially different in size and can be readily removed using known purification methods, e.g., SEC, to provide a population of assembled trimers.

In certain embodiments, the purification tags are relatively small, e.g., less than 10 kDa, preferably less than 5 kDa. Preferred tags include, but are not limited to, strep tags, including tandem strep tags; 6 X His tags, myc-tags, and HAI-tags. Multiple copies of smaller tags can be inserted into the RSVF peptides of the invention. For example, tags can be expressed in tandem adjacent or nearly adjacent to each other. In certain embodiments, tags can be inserted N-terminal and C-terminal to a six helix bundle forming domain, e.g., N-terminal and C-terminal to an inserted domain, or N-terminal and C-terminal to an oligomerization domain, that is either linked or not linked to an ectodomain. Tags can also include larger tags, e.g., GST tags. However, larger tags are typically used as single copies.

In preferred embodiments, the purification tags can be removed by protease cleavage. Removal of the purification tags by protease cleavage preferably does not result in substantial cleavage of the other portions of the RSVF complex. Methods to selecte appropriate protease cleavage sites and conditions are well known in the art.

### Complex purification

RSVF protein ecto-domain polypeptides, oligomerization peptides, and complexes can be purified using any suitable method. For example, methods for purifying RSVF ecto-domain polypeptides by immunoaffinity chromatography are known in the art. Ruiz-Arguello et al., J. Gen. Virol., 85:3677-3687 (2004). Suitable methods for purifying desired proteins including precipitation and various types of chromatography, such as hydrophobic interaction, ion exchange, affinity, chelating and size exclusion are well known in the art. Suitable purification schemes can be created using two or more of these or other suitable methods. If desired, the RSVF protein ecto-domain polypeptides and oligomerization petpides can include a "tag" that facilitates purification, such as an epitope tag or a HIS tag. Such tagged polypeptides can conveniently be purified, for example from conditioned media, by chelating chromatography or affinity chromatography.

The use of purification tags can facilitate purification of properly assembled trimers. For example, if two ectodomain polypeptides comprising endogenous RSVF HRA and HRB domain and an inserted HRA domain formed a complex in which the inserted HRA complexed with the endogenous HRB, the affinity tags would likely be buried in the complex such that the complex would not be captured by the affinity purification method. Similarly, disordered multimerization of peptides would likely mask the purification tags. In certain embodiments, only the non-linked oligomerization peptide includes an affinity tag. Therefore, only complexes containing at least one oligomerization polypeptide with an exposed purification tag is captured by the affinity purification methods. Due to the substantial difference in size between the assembled complexes and the oligomerization peptides, the complexes are easily separated from the oligomerization peptides providing a population of well formed trimers.

Affinity purification methods can be used in combination with other purification methods, for example, size exclusion chromatography is useful for separation of well formed trimers from monomers and other complexes. In certain embodiments, purification tags are cleaved from the assembled complexes prior to further purification steps.

In preferred embodiments, the RSVF complexes are assembled in cells and secreted into the growth media to facilitate purification. It is understood that the number and types of purification steps to be performed is dependent, at least in part, on the method of expression or production of the peptides for use in the complexes of the invention.

### Additional Peptide Sequences

Oligomerization polypeptides contain an oligomerization region and if desired can further contain a functional region as described herein. Suitable amino acid sequences for the oligomerization regions (e.g., the six-helix bundle forming moiety, e.g., the amino acid sequence of the HRA domain or the HRB domain of RSVF) are well known in the art as are suitable functional regions. The oligomerization polypeptide can be prepared using any suitable method, such as by chemical synthesis, recombinant expression in a suitable host cell, chemical conjugation and the like.

### Assembly and Cleavage of RSVF Complexes

### Complexes with attached oligomerization domains

In certain aspects, the invention relates to a method for producing a RSVF complex that contains three RSVF ectodomain polypeptides each further comprising a first C-terminal 6-helix bundle forming moiety and a second C-terminal 6-helix bundle forming moiety (i.e., an operably linked oligomerization domain), and therefore does not include a separate oligomerization polypeptide. The method for producing such complexes containing an attached oligomerization domain is substantially the same as the method for producing complexes that contain an oligomerization polypeptide except that the ratio of the attached oligomerization domains to the ectodomain polypeptides is fixed, and no separate oligomerization polypeptide is added to the ectodomain polypeptides during assembly of the trimers. In particular, the method includes a) providing RSVF ectodomain polypeptides comprising an endogenous HRA region and an endogenous HRB region further comprising a first inserted (i.e., non-endogenous) C-terminal 6-helix bundle forming moiety and a second non-endogenous C-terminal 6-helix bundle forming moiety (i.e., the oligomerization domain), and b) combining the RSVF ectodomain polypeptides under conditions suitable for the formation of an RSVF complex, whereby a RSVF complex is produced that comprises three RSVF ectodomain polypeptides and is characterized by a six-helix bundle formed by the first and second non-endogenous C-terminal 6-helix bundle forming moieties (e.g., inserted six-helix bundle forming moieties and oligomerization domains). It is understood that these steps can take place within a cell or outside of a cell. That is, it is understood that combining can include co-expression of the polypeptides within a cell. Further, it is understood that conditions suitable for the formation of an RSVF complex can include conditions within a cell or outside of a cell. Conditions suitable outside of the cell can include a series of conditions to promote partial unfolding and refolding of the ectodomain monomers to produce the trimers of the invention.

When RSVF complexes that contain cleaved RSVF ectodomain polypeptides are desired, the optional step c) cleaving the RSVF protein ectodomain polypeptides in the produced complex with a suitable protease. Suitable proteases include any protease that can cleave the RSVF ectodomain polypeptide (preferably an uncleaved RSVF ectodomain polypeptide) to form F1 and F2 subunits. Usually, the protease will cleave a natural or inserted cleavage site between about position 101 to about position 161. Proteases that can be used include, but are not limited to, trypsin, chymotrypsin, TEV protease, Rhinovirus 3c protease, and thrombin. if trypsin is used, trypsin digestion of the RSVF complex is performed using 1:1000 trypsin:RSVF complex by weight, or 10-15 BAEE units of trypsin for 1 mg of RSVF complex. In a typical reaction, trypsin from bovine plasma (Sigma Aldrich, T8802: 10,000-15,000 BAEE units/mg trypsin) is diluted to a 1 mg/ml concentration in 25 mM Tris pH 7.5, 300 mM NaCl and RSVF protein ecto-domain polypeptide (in 25 mM Tris pH 7.5, 300 mM NaCl) is digested for 1 hour at 37 °C. The cleavage reaction can be stopped using a trypsin inhibitor. In certain embodiments, the cleavage site comprises a furin cleavage site, optionally an optimized furin cleavage site. When a furin cleavage site is used, the complex is preferably assembled within cells and the furin cleavage takes place within the cells.

In certain embodiments, the ectodomain polypeptides or the oligomerization polypeptides can include purification tags that are removed by protease cleavage. Removal of a purification tag does not produce a cleaved ectodomain polypeptide as the purification tag is not present in the ectodomain polypeptide. Removal of purification tags by protease cleavage occurs outside of the cell, after purification of the protein complex. Proteases for removal of the purification tags are selected such that no significant cleavage of the RSVF complex occurs within the RSVF sequences. Considerations related to selection of appropriate proteases is within the ability of those of skill in the art.

### Complexes with separate oligomerization domains

In some embodiments, the method comprises a) providing RSVF ectodomain polypeptides further comprising at least one inserted C-terminal 6-helix bundle forming moiety and at least one oligomerization polypeptide (which can include more than one oligomerization domain) not linked to he ectodomain polypeptide, and b) combining the RSVF ectodomain polypeptides and at least one oligomerization polypeptide under conditions suitable for the formation of an RSVF complex, whereby a RSVF complex is produced that comprises three of the RSVF ectodomain polypeptides, and the at least one oligomerization polypeptide, and is characterized by a six-helix bundle. The six-helix bundle comprises inserted C-terminal 6-helix bundle forming moiety of each RSVF ectodomain polypeptide (e.g.,inserted RSVF HRA or inserted RSVF HRB) and three oligomerization domains, e.g., each oligomerization domain of three oligomerization polypeptides (e.g., RSVF HRB or RSVF HRA, respectively). In certain embodiments, three oligomerization domains of the oligomerization peptide comprise the amino acid sequence of the HRB region of RSVF, and the six-helix bundle forming moiety comprises the inserted HRA region of each of the three RSVF ectodomain polypeptides. It is understood that these steps can take place within a cell or outside of a cell. That is, it is understood that combining can include co-expression of the polypeptides within a cell. Further, it is understood that conditions suitable for the formation of an RSVF complex can include conditions within a cell or outside of a cell. Conditions suitable outside of the cell can include a series of conditions to promote partial unfolding and refolding of the ectodomain monomers to produce the trimers of the invention. Further, it is understood that RSVF ectodomain polypeptides can include cleavage sites that can be cleaved outside the cell or inside the cell. Moreover, it is understood that RSVF ectodomain polypeptides or oligomerization polypeptides can include purification tags that can optionally be removed by protease cleavage. Purification tags are removed outside of the cell after purification.

In other embodiments, the method comprises a) providing recombinant RSVF ectodomain polypeptides each further comprising a C-terminal 6-helix bundle forming moiety and at least one oligomerization polypeptide (wherein the oligomerization peptide may include more than one oligomerization domain), and b) combining the recombinant RSVF ectodomain polypeptides each further comprising a C-terminal 6-helix bundle forming moiety and at least one oligomerization polypeptide under conditions suitable for the formation of an RSVF complex, whereby an RSVF complex is produced that comprises three of the RSVF ectodomain polypeptides each further comprising a C-terminal 6-helix bundle forming moiety, the at least one oligomerization polypeptide, preferably three of the oligomerization polypeptides, and is characterized by a six-helix bundle. The six-helix bundle comprises the inserted C-terminal 6-helix bundle forming moiety of each recombinant RSVF ectodomain polypeptide and three oligomerization domains, preferably an oligomerization domain in each of three oligomerization polypeptides. In certain embodiments, the C-terminal 6-helix bundle forming moiety is the HRB region of RSVF or HR2 of HIV gp41, and the three oligomerization domains of the oligomerization peptide(s) comprises the amino acid sequence of the HRA region of RSVF or HR1 HIV gp41, respectively, or other six helix bundle forming moieties include, but are not limited to, class 1 fusion viral proteins that contain 6-helix bundle forming moieties. In such embodiments, the six-helix bundle comprises the inserted C-terminal 6-helix bundle forming moiety (i.e., the inserted HRA region) of each RSVF ectodomain polypeptide and the HRB region of each oligomerization peptide. It is understood that these steps can take place within a cell or outside of a cell. That is, it is understood that combining can include co-expression of the polypeptides within a cell. Further, it is understood that conditions suitable for the formation of an RSVF complex can include conditions within a cell or outside of a cell. Conditions suitable outside of the cell can include a series of conditions to promote partial unfolding and refolding of the ectodomain monomers to produce the trimers of the invention. Further, it is understood that RSVF ectodomain polypeptides can include cleavage sites that can be cleaved outside the cell or inside the cell. Moreover, it is understood that RSVF ectodomain polypeptides or oligomerization polypeptides can include purification tags that can optionally be removed by protease cleavage. Purification tags are removed outside of the cell after purification.

The invention also includes RSVF complexes produced using the methods described herein.

### Immunogenic compositions

The invention provides immunogenic compositions that comprise the RSVF complexes disclosed herein. The compositions are preferably suitable for administration to a mammalian subject, such as a human, and include one or more pharmaceutically acceptable carrier(s) and/or excipient(s), including adjuvants. A thorough discussion of such components is available in Gennaro (2000) Remington: The Science and Practice ofPharmacy. 20th edition, ISBN: 0683306472. Compositions will generally be in aqueous form. When the composition is an immunogenic composition, it will elicit an immune response when administered to a mammal, such as a human. In certain embodiments, the immune response is a neutralizing immune response. The immunogenic composition can be used to prepare a vaccine formulation for immunizing a mammal.

The immunogenic compositions may include a single active immunogenic agent, or several immunogenic agents. For example, the compositions can contain an RSVF complex and one or more other RSV proteins *(e.g.,* a G protein and/or an M protein) and/or one or more immunogens from other pathogens. The immunogenic composition can comprise a monovalent RSVF complex that contains RSVF complexes provided herein and, if desired, can contain one or more additional antigens from RSVF or another pathogen, e.g., influenza In one example, the immunogenic composition is divalent and comprises an RSVF complex that also contains another RSVF antigen, such as RSV G protein. As described herein, such multivalent complexes can be produced using an oligomerization polypeptide that contains an oligomerization region that is operably linked to an amino acid sequence from RSV G, such as an amino acid sequence from the central domain of RSV G.

The composition may include preservatives such as thiomersal or 2-phenoxyethanol. It is preferred, however, that the vaccine should be substantially free from *(i.e.,* less than 5µg/ml) mercurial material, *e.g.,* thiomersal-free. Immunogenic compositions containing no mercury are more preferred. Preservative-free immunogenic compositions are particularly preferred.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, and the like.

Compositions will generally have an osmolality of between 200 mOsm/kg and 400 mOsm/kg, preferably between 240-360 mOsm/kg, and will more preferably fall within the range of 290-310 mOsm/kg.

Compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range. The pH of a composition will generally be between 5.0 and 8.1, and more typically between 6.0 and 8.0, e.g., between 6.5 and 7.5, or between 7.0 and 7.8. A process of the invention may therefore include a step of adjusting the pH of the bulk vaccine prior to packaging.

The composition is preferably sterile. The composition is preferably non-pyrogenic, e.g., containing <1 EU (endotoxin unit, a standard measure) per dose, and preferably <0.1 EU per dose. The composition is preferably gluten free. Human vaccines are typically administered in a dosage volume of about 0.5ml, although a half dose (*i.e*., about 0.25ml) may be administered to children.

Immunogenic compositions of the invention may also comprise one or more immunoregulatory agents. Preferably, one or more of the immunoregulatory agents include one or more adjuvants, for example two, three, four or more adjuvants. The adjuvants may include a TH1 adjuvant and/or a TH2 adjuvant, further discussed below.

Preferably, the immunogenic composition comprises a RSVF complex that displays an epitope present in a prefusion conformation of RSVF glycoprotein. An exemplary composition comprises an RSVF complex that contains cleaved RSVF ectodomain polypeptides. Another exemplary composition comprises an RSVF complex that contains uncleaved RSVF ectodomain polypeptides.

### Methods of treatment and administration

Compositions of the invention are suitable for administration to mammals, and the invention provides a method of inducing an immune response in a mammal, comprising the step of administering a composition (e.g., an immunogenic composition) of the invention to the mammal. In certain embodiments, the immune response is a neutralizing immune response. The compositions (e.g., an immunogenic composition) can be used to produce a vaccine formulation for immunizing a mammal. The mammal is typically a human, and the RSVF complex typically contains human RSVF ectodomain polypeptides. However, the mammal can be any other mammal that is susceptible to infection with RSV, such as a cow that can be infected with bovine RSV.

The invention also provides a composition for use as a medicament, e.g., for use in immunizing a patient against RSV infection, e.g., for use in raising a neutralizing immune response in a patient.

The invention also provides the use of a RSVF complex as described above in the manufacture of a medicament for raising an immune response in a patient.

The immune response raised by these methods and uses will generally include an antibody response, preferably a protective antibody response (i.e., a neutralizing response). Methods for assessing antibody responses after RSV vaccination are well known in the art.

Compositions of the invention can be administered in a number of suitable ways, such as intramuscular injection *(e.g.,* into the arm or leg), subcutaneous injection, intranasal administration, oral administration, intradermal administration, transcutaneous administration, transdermal administration, and the like. The appropriate route of administration will be dependent upon the age, health and other characteristics of the mammal. A clinician will be able to determine an appropriate route of administration based on these and other factors.

Immunogenic compositions, and vaccine formulations, may be used to treat children and adults, including pregnant women. Thus a subject may be less than 1 year old, 1-5 years old, 5-15 years old, 15-55 years old, or at least 55 years old. Preferred subjects for receiving the vaccines are the elderly *(e.g.,* >50 years old, >60 years old, and preferably >65 years) and pregnant women. The vaccines are not suitable solely for these groups, however, and may be used more generally in a population.

Treatment can be by a single dose schedule or a multiple dose schedule. Multiple doses may be used in a primary immunization schedule and/or in a booster immunization schedule. In a multiple dose schedule the various doses may be given by the same or different routes, *e.g.,* a parenteral prime and mucosal boost, a mucosal prime and parenteral boost, *etc.* Administration of more than one dose (typically two doses) is particularly useful in immunologically naive patients. Multiple doses will typically be administered at least 1 week apart (e.g., about 2 weeks, about 3 weeks, about 4 weeks, about 6 weeks, about 8 weeks, about 10 weeks, about 12 weeks, about 16 weeks, and the like.).

Vaccine formulations produced using a composition of the invention may be administered to patients at substantially the same time as (*e.g.,* during the same medical consultation or visit to a healthcare professional or vaccination centre) other vaccines.

### Other viruses

As well as being used with human RSV, the invention may be used with other members of the *Pneumoviridae* and *Paramyxoviridae,* including, but not limited to, bovine respiratory syncytial virus, parainfluenzavirus 1, parainflueznavirus 2, parainfluenzavirus 3, and parainfluenzavirus 5.

Thus the invention provides an immunogenic composition comprising a F glycoprotein from a *Pneumoviridae* or *Paramyxoviridae,* wherein the F glycoprotein is in pre-fusion conformation.

The invention also provides an immunogenic composition comprising a polypeptide that displays an epitope present in a pre-fusion conformation of the F glycoprotein of a *Pneumoviridae* or *Paramyxoviridae,* but absent the glycoprotein's post fusion conformation.

The invention also provides these polypeptides and compositions for use in immunization, *etc.*

### General

As used herein, the transitional term "comprising", which is synonymous with "including," "containing," or "characterized by," is inclusive or open-ended and does not exclude additional, unrecited elements or method steps. As used herein, the transitional phrase "consisting of" excludes any element, step, or ingredient not specified in the claim. Finally, as used herein, the transitional phrase "consisting essentially of" limits the scope of a claim to the specified materials or steps "and those that do not materially affect the basic and novel characteristic(s)" of the claimed invention as understood under United States patent law.

The word "substantially" does not exclude "completely" e.g., a composition which is "substantially free" from Y may be completely free from Y. Substantially, for example, can include at least about 80%, at least about 85%, at least about 90%, at least about 95%, at least about 96%, at least about 97%, at least about 98%, or at least about 99%. Where necessary, the word "substantially" may be omitted from the definition of the invention.

The term "about" in relation to a numerical value x means, for example, ± two standard deviations of the value. In certain embodiments, "about" is understood as acceptable variation and tolerances within the specific art. It is understood that tolerances within SEC and western blot are higher than, for example, tolerances in BIACore® measurements. Methods and controls are known in the art to provide more quantitative or at least semi-quantitative results from certain methods. Moreover, it is understood that some methods are more useful to detect relative amounts of components rather than absolute amounts of components (e.g., western blotting and SEC). For western blotting and SEC when comparing relative levels of components, about is preferably understood as less than or equal to 5%, preferably less than or equal to 3%. Unless clear from context, all numerical terms herein are understood to be modified by about.

As use herein, "or" is understood to be inclusive and is interchangeable with "and/or" unless otherwise clearly indicated by context. For example, when referring to a population of RSVF complexes that has a certain percent of complexes that are properly folded or a certain percent that are cleaved, "or" is understood to be interchangeable with "and/or." For example a population of polypeptides in which a first percent are trimers or a second percent are cleaved, or is understood as and/or. However, when referring to a polypeptide that includes one of an inserted HRA or an inserted HRB, "or" is understood from context to be exclusive, i.e., either an inserted HRA is present or an inserted HRB is present for that element, not both.

As used herein, "a" and "the" are understood to include both singular and plural unless otherwise clearly indicated by context.

Unless specifically stated, a process comprising a step of mixing two or more components does not require any specific order of mixing. Thus components can be mixed in any order. Where there are three components then two components can be combined with each other, and then the combination may be combined with the third component, etc.

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

Where a cell substrate is used for reassortment or reverse genetics procedures, it is preferably one that has been approved for use in human vaccine production *e.g*., as in Ph Eur general chapter 5.2.3.

Identity between polypeptide sequences is preferably determined by the Smith-Waterman homology search algorithm as implemented in the MPSRCH program (Oxford Molecular), using an affine gap search with parameters gap open penalty=12 and gap extension penalty=1. Identity can be determined over the full length of the sequence, over one or more domains of a sequence, or within the overlapping portions of two sequences.

### Embodiments of the Invention

The invention includes the following embodiments
Embodiment 1. A respiratory syncytial virus F (RSVF) complex, comprising a six helix bundle, comprising:
   three RSVF ectodomain polypeptides each comprising an endogenous HRA region, an endogenous HRB region, further comprising one of an inserted six-helix bundle forming domain, preferably an inserted RSVF HRA region or an inserted RSVF HRB region and
   at least one oligomerization polypeptide, wherein the three ectodomain polypeptides and the at least one oligomerization polypeptide form a six-helix bundle, with the proviso that the six-helix bundle does not include the endogenous HRA regions and endogenous HRB regions of the RSVF polypeptides.
Embodiment 2. The RSVF complex of embodiment 1, wherein each RSVF ectodomain polypeptide comprises an inserted HRA region.
Embodiment 3. The RSVF complex of embodiment 1, wherein each RSVF ectodomain polypeptide comprises an inserted HRB region.
Embodiment 4. The RSVF complex of any of embodiments 1-3, wherein the six-helix bundle is detected by a method selected from the group consisting of size exclusion chromatography, western blot, and electron microscopy.
Embodiment 5. The RSVF complex of any one of embodiments 1-4, wherein the inserted RSVF HRA region or inserted RSVF HRB region is attached to the ectodomain by a polypeptide linker.
Embodiment 6. The RSVF complex of embodiment 5, wherein the polypeptide linker is 3-30, 3-21, 3-15, 3-10, 4-8, or 5-7 amino acids in length.
Embodiment 7. The RSVF complex of embodiment 5 or 6, wherein the inserted RSVF HRA region or inserted RSVF HRB region is C-terminal to the ectodomain in the RSVF ectodomain polypeptide.
Embodiment 8. The RSVF complex of any of embodiments 1-7, wherein the six helix bundle comprises the inserted HRA region of each RSVF ectodomain polypeptide and the oligomerization polypeptide comprises an HRB region.
Embodiment 9. The RSVF complex of embodiment 5, wherein the HRB region is operably linked to at least one RSVF ectodomain polypeptide.
Embodiment 10. The RSVF complex of embodiment 6, wherein the HRB region is linked to at least one RSVF ectodomain polypeptide by a covalent linker.
Embodiment 11. The RSVF complex of any of embodiments 1-7, wherein the six helix bundle comprises the inserted HRB region of each RSVF ectodomain polypeptide and the oligomerization polypeptide comprises an HRA region.
Embodiment 12. The RSVF complex of embodiment 11, wherein the HRA region is operably linked to at least one RSVF ectodomain polypeptide.
Embodiment 13. The RSVF complex of embodiment 12, wherein the HRA region is linked to at least one RSVF ectodomain polypeptide by a covalent linker.
Embodiment 14. The RSVF complex of embodiment 10 or 13, wherein the covalent linker comprises a polypeptide linker.
Embodiment 15. The RSVF complex of embodiment 13 or 14, wherein the covalent linker comprises a structurally constrained linker.
Embodiment 16. The RSVF complex of embodiment 14 or 15, wherein the polypeptide linker is 3-30, 3-21, 3-15, 3-10, 4-8, or 5-7 amino acids in length.
Embodiment 17. The RSV complex of any of embodiments 10 or 13-16, wherein the oligomerization domain is C-terminal to the inserted HRA region or the inserted HRB region.
Embodiment 18. The RSVF complex of any of embodiments 1-8 and 11, wherein the oligomerization polypeptide is not linked to at least one RSVF ectodomain polypeptide.
Embodiment 19. The RSVF complex of any one of embodiments 1-18, wherein the oligomerization polypeptide comprises a heptad repeat region of a fusion protein of an enveloped virus.
Embodiment 20. The RSVF complex of embodiment 19, wherein the heptad repeat region of a fusion protein of an enveloped virus is selected from the group consisting of an HIV gp41 HR1 region, an HIV gp41 HR2 region, Newcastle disease virus (NDV) HR1/HR2, human metapneumovirus HR1/HR2, other class 1 fusion viral proteins that contain 6-helix bundles in their postfusion state.
Embodiment 21. The RSVF complex of any of embodiments 1-20, wherein the at least one oligomerization polypeptide comprises two polypeptides.
Embodiment 22. The RSVF complex of any of embodiments 1-20, wherein the at least one oligomerization polypeptide comprises three polypeptides.
Embodiment 23. The RSVF complex of any of embodiments 1-22, wherein the oligomerization polypeptide comprises a purification tag.
Embodiment 24. The RSVF complex of embodiment 23, wherein the purification tag comprises at least one tag selected from the group consisting of 6X His tag, strep tag, myc tag.
Embodiment 25. The RSVF complex of embodiment 23 or 24, wherein the purification tag comprises 2 tags in tandem.
Embodiment 26. The RSVF complex of embodiment 23 or 24, wherein the purification tag comprises a cleavable purification tag.
Embodiment 27. The RSVF complex of any one of embodiments 1-26, wherein one or more of said oligomerization polypeptides further comprises a functional region that is operably linked to the oligomerization polypeptide.
Embodiment 28. The RSVF complex of embodiment 27, wherein the functional regions is selected from the group consisting of an immunogenic carrier protein, an antigen, a particle-forming polypeptide, a lipid, and a polypeptide that can associate the oligomerization polypeptide with a liposome or particle; or any combination thereof.
Embodiment 29. The RSVF complex of embodiment 28, wherein the functional region is a therapeutically relevant antigen.
Embodiment 30. The RSVF complex of embodiment 28 or 29, wherein the antigen is not present in RSVF.
Embodiment 31. The RSVF complex of any of embodiments 28-30, wherein the antigen is an artificial sequence.
Embodiment 32. The RSVF complex of embodiment 28-30, wherein the antigen is RSV G.
Embodiment 33. The RSVF complex of any one of embodiments 1-32, wherein one or more of the RSVF ectodomain polypeptides comprises an uncleaved RSVF ectodomain polypeptide.
Embodiment 34. The RSVF complex of any one of embodiments 1-32, wherein one or more of the RSVF ectodomain polypeptides comprises a cleaved RSVF ectodomain polypeptide.
Embodiment 35. The RSVF complex of any one of embodiments 1-34, wherein each of the RSVF ectodomain polypeptides comprises an altered furin cleavage site.
Embodiment 36. The RSVF complex of any one of embodiments 1-35, wherein each of the RSVF ectodomain polypeptides comprises a protease cleavage site.
Embodiment 37. The RSVF complex of embodiment 36, wherein the protease cleavage site is selected from the group consisting of furin cleavage site, trypsin cleavage site, chymotrypsin cleavage site, TEV protease cleavage site, thrombin cleavage site, and Rhinovirus 3c protease cleavage site.
Embodiment 38. The RSVF complex of any one of embodiments 1-37, wherein the amino acid sequence of the RSVF ectodomain polypeptides comprises a sequence selected from the group consisting of: SEQ ID NO: 1, SEQ ID NO: 8 (Del21 Furx), SEQ ID NO: 3 (Furmt), SEQ ID NO: 4 (Furdel), SEQ ID NO: 5 (Furx), SEQ ID NO: 6 (Furx R113Q, K123N, K124N), SEQ ID NO: 7 (Furx R113Q, K123Q, K124Q), SEQ ID NO:9 (Delp23Furx), SEQ ID NO: 10 (Delp21 furdel), SEQ ID NO: 11 (Delp23 furdel), SEQ ID NO: 12 (N-term Furin), SEQ ID NO: 13 (C-term Furin), SEQ ID NO: 14 (Factor Xa), SEQ ID NO: 15, SEQ ID NO: 26 (Fusion Peptide Deletion 1), an ectodomain sequence provided in the sequence listing; and any of the foregoing in which the signal peptide and/or HIS tag and/or fusion peptide, is omitted or altered.
Embodiment 39. The RSVF complex of any of embodiments 1-38, wherein the amino acid sequence of the RSVF ectodomain polypeptides further comprises S155C and S290C substitutions.
Embodiment 40. The RSVF complex of any of embodiments 1-39, wherein the amino acid sequence of the RSVF ectodomain polypeptides further comprises an F substitution at amino acid 190 and/or an L substitution at amino acid 207.
Embodiment 41. The RSVF complex any of embodiments 1-38, wherein the amino acid sequence of the RSVF ectodomain polypeptides further comprises S155C, S290C, S190F, and V207L substitutions.
Embodiment 42. The RSVF complex of embodiment 1, wherein the oligomerization polypeptide comprises a 6-helix bundle forming moiety.
Embodiment 43. The RSVF complex of embodiment 42, wherein the six-helix bundle forming moiety comprises a heptad repeat region of the fusion protein of an enveloped virus.
Embodiment 44. The RSVF complex of embodiment 43, wherein the heptad repeat region is selected from the group consisting of RSVF HRA domain, RSVF HRB domain, HIV gp41 HR1 region, an HIV gp41 HR2 region, Newcastle disease virus (NDV) HR1/HR2, human metapneumovirus HR1/HR2, other class 1 fusion viral proteins that contain 6-helix bundles in their postfusion state.
Embodiment 45. The RSVF complex of any one of embodiments 42-44, wherein the six-helix bundle comprises the inserted RSVF HRA domain or inserted RSVF HRB domain of three recombinant RSVF ectodomain polypeptides and the oligomerization region of each oligomerization polypeptide.
Embodiment 46. The RSVF complex of any one of embodiments 1-45, wherein the RSVF ectodomain polypeptides are in the pre-fusion conformation.
Embodiment 47. The RSVF complex of any one of embodiments 1-46, wherein the complex is characterized by a rounded shape when viewed in negatively stained electron micrographs.
Embodiment 48. The RSVF complex of any one of embodiments 1-47, wherein the complex is characterized by antibody binding.
Embodiment 49. The RSVF complex of embodiment 48, wherein binding by the D25 antibody is indicative of a pre-fusion conformation.
Embodiment 50. The RSVF complex of any one of embodiments 1-49, wherein the complex comprises pre-fusion epitopes that are not present on post-fusion forms of RSVF.
Embodiment 51. The RSVF complex of any one of embodiments 1-37 and 42-45, wherein the RSVF ectodomain polypeptides are in the post-fusion conformation.
Embodiment 52. The RSVF complex of any of embodiments 1-51, wherein the inserted HRA region or the inserted HRB region is C-terminal to the endogenous HRB region in the RSVF ectodomain polypeptide.
Embodiment 53. The RSVF complex of embodiment 52, wherein the oligomerization polypeptide is linked to the RSVF ectodomain polypeptide C-terminal to the inserted HRA region or the inserted HRB region.
Embodiment 54. A respiratory syncytial virus F (RSVF) complex comprising a six-helix bundle, comprising three RSVF ectodomain polypeptides each comprising (a) an endogenous HRA region and an endogenous HRB region, each further comprising (b) one of an inserted RSVF HRA region or an inserted RSVF HRB region, further comprising (c) a C-terminal 6-helix bundle forming moiety, the six-helix bundle is formed by the C-terminal 6-helix bundle forming moiety and the inserted HRA region or the inserted HRB region, with the proviso that the six-helix bundle does not include the endogenous HRA regions and endogenous HRB regions of the RSVF polypeptides.
Embodiment 55. The complex of embodiment 54, wherein the ectodomain polypeptides each comprise an inserted RSVF HRA domain and the C-terminal 6-helix bundle forming moiety comprises an RSVF HRB domain.
Embodiment 56. The complex of embodiment 54, wherein the ectodomain polypeptides each comprise an inserted RSVF HRB domain and the C-terminal 6-helix bundle forming moiety comprises an RSVF HRA domain.
Embodiment 57. A method for producing a respiratory syncytial virus F (RSVF) complex comprising a six-helix bundle, comprising:
   a) providing RSVF protein ectodomain polypeptides comprising an endogenous HRA region and an endogenous HRB region, further comprising one of an inserted RSVF HRA region or an inserted RSVF HRB region; and at least one oligomerization polypeptide, and
   b) combining the RSVF ectodomain polypeptides and the at least one oligomerization polypeptide under conditions suitable for the formation of an RSVF complex, whereby an RSVF complex is produced in which three of the RSVF ectodomain polypeptides and at least one of the oligomerization polypeptides form a six-helix bundle, with the proviso that the endogenous HRA regions and the endogenous HRB regions of the RSVF ectodomain polypeptides are not part of the six-helix bundle.
Embodiment 58. The method of embodiment 57, wherein the RSVF protein ectodomain polypeptides and the oligomerization polypeptide are provided in a cell.
Embodiment 59. The method of embodiment 57, wherein the RSVF protein ectodomain polypeptides and the oligomerization polypeptide are provided in conditioned cell culture media.
Embodiment 60. The method of embodiment 58, wherein the RSVF protein ectodomain polypeptides and the oligomerization polypeptide are provided by expression from an expression construct.
Embodiment 61. The method of embodiment 60, wherein the expression construct comprises a DNA construct or an RNA construct.
Embodiment 62. The method of embodiment 60 or 61 wherein the RSVF protein ectodomain polypeptides and the oligomerization polypeptide are expressed from a single construct.
Embodiment 63. The method of embodiment 62 wherein the RSVF protein ectodomain polypeptides and the oligomerization polypeptide are expressed under the control of a single promoter.
Embodiment 64. The method of embodiment 62 wherein the RSVF protein ectodomain polypeptides and the oligomerization polypeptide are expressed under the control of separate promoters.
Embodiment 65. The method of embodiment 64, wherein the promoters are the same.
Embodiment 66. The method of embodiment 64, wherein the promoters are different.
Embodiment 67. The method of embodiment 60 or 61 wherein the RSVF protein ectodomain polypeptides and the oligomerization polypeptide are expressed from two separate constructs.
Embodiment 68. The method of embodiment 67, wherein the two separate constructs have same promoter sequences.
Embodiment 69. The method of embodiment 67, wherein the two separate constructs have different promoter sequences.
Embodiment 70. The method of any of embodiments 67-69, wherein the number of coding sequences for the ectodomain and number of coding sequences for the oligomerization domain are present in a cell at a ratio of 2:1 to 1:8.
Embodiment 71. The method of any of embodiments 67-69, wherein the number of coding sequences for the ectodomain and the number of coding sequences for the oligomerization domain are present in a cell at a ratio of 1:1 to 1:8.
Embodiment 72. The method of any of embodiments 67-69, wherein the number of coding sequences for the ectodomain and the number of coding sequences for the oligomerization domain are present in a cell at a ratio of 1:1 to 1:4.
Embodiment 73. The method of any of embodiments 67-69, wherein the number of coding sequences for the ectodomain and the number of coding sequences for the oligomerization domain are present in a cell at a ratio of 1:1 to 1:2.
Embodiment 74. The method of any of embodiments 59-73, wherein the ectodomain polypeptide and the oligomerization polypeptide are provided at a ratio of 2:1 to 1:8.
Embodiment 75. The method of any of embodiments 59-73, wherein the ectodomain polypeptide and the oligomerization polypeptide are provided at a ratio of 1:1 to 1:8.
Embodiment 76. The method of any of embodiments 59-73, wherein the ectodomain polypeptide and the oligomerization polypeptide are provided at a ratio of 1:1 to 1:4.
Embodiment 77. The method of any of embodiments 59-73, wherein the ectodomain polypeptide and the oligomerization polypeptide are provided at a ratio of 1:1 to 1:2.
Embodiment 78. The method of embodiment 57, wherein the RSVF protein ectodomain polypeptides are provided as substantially purified polypeptides.
Embodiment 79. The method of embodiment 57 or 78, wherein the oligomerization polypeptide is provided as a substantially purified polypeptide.
Embodiment 80. The method of embodiment 57-63, wherein the ectodomain polypeptide is operably linked to the oligomerization polypeptide.
Embodiment 81. The method of embodiment 57-79, wherein the ectodomain polypeptide is not operably linked to the oligomerization polypeptide.
Embodiment 82. The method of any one of embodiments 57-81, wherein the ectodomain polypeptide comprises an RSVF HRA region and the oligomerization polypeptide comprises an RSVF HRB region.
Embodiment 83. The method of any one of embodiments 57-81, wherein the ectodomain polypeptide comprises an RSVF HRB region and the oligomerization polypeptide comprises an RSVF HRA region.
Embodiment 84. The method of embodiment 57-83, wherein the RSVF ectodomain polypeptides provided in a) comprise uncleaved RSVF ectodomain polypeptides.
Embodiment 85. The method of embodiment 57-84, wherein the RSVF ectodomain polypeptides provided in a) each comprise one or more altered furin cleavage sites.
Embodiment 86. The method of embodiment 57-85, wherein the RSVF ectodomain polypeptides comprise at least one protease cleavage site.
Embodiment 87. The method of embodiment 86, wherein protease cleavage site is selected from the group consisting of furin cleavage site, trypsin cleavage site, TEV protease cleavage site, thrombin cleavage site, and Rhinovirus 3c protease cleavage site.
Embodiment 88. The method of any one of embodiments 57-87, wherein the RSVF ectodomain polypeptides provided in a) comprise purified monomers.
Embodiment 89. The method of any one of embodiments 57-88, further comprising c) cleaving the RSVF protein ectodomain polypeptides in the produced complex with a protease.
Embodiment 90. The method of any one of embodiments 57-89, wherein each RSVF ectodomain polypeptide comprises an HRB region and each exogenous oligomerization polypeptide comprises an oligomerization region.
Embodiment 91. The method of any one of embodiments 57-90, wherein the inserted HRA region or the inserted HRB region is C-terminal to the endogenous HRA region and the endogenous HRB region.
Embodiment 92. The method of embodiment 80, wherein the oligomerization peptide is operably linked to the RSVF ectodomain polypeptide C-terminal to the inserted HRA region or inserted HRB region.
Embodiment 93. The method of any one of embodiments 57-92, wherein the complex consists of the three RSVF ectodomain polypeptides and three oligomerization polypeptides.
Embodiment 94. The method of any one of embodiments 57-93, wherein one or more of said oligomerization polypeptides further comprise a functional region that is operably linked to the oligomerization region.
Embodiment 95. The method of any one of embodiments 57-94, wherein the amino acid sequence of the RSVF ectodomain polypeptides provided in step a) comprises a sequence selected from the group consisting of: SEQ ID NO: 1, SEQ ID NO:8 (Del21 Furx), SEQ ID NO: 3 (Furmt), SEQ ID NO: 4 (Furdel), SEQ ID NO: 5 (Furx), SEQ ID NO: 6 (Furx R113Q, K123N, K124N), SEQ ID NO: 7 (Furx R113Q, K123Q, K124Q), SEQ ID NO: 9 (Delp23Furx), SEQ ID NO: 10 (Delp21 furdel), SEQ ID NO: 11 (Delp23 furdel), SEQ ID NO: 12 (N-term Furin), SEQ ID NO: 13 (C-term Furin), SEQ ID NO: 14 (Factor Xa), SEQ ID NO: 15, SEQ ID NO: 26 (Fusion Peptide Deletion 1), an ectodomain sequence provided in the sequence listing; and any of the foregoing in which the signal peptide and/or HIS tag and/or fusion peptide, is omitted or altered.
Embodiment 96. The method of embodiment 95, wherein the amino acid sequence of the RSVF ectodomain polypeptides further comprises S155C and S290C substitutions.
Embodiment 97. The method of embodiment 95 or 96, wherein the amino acid sequence of the RSVF ectodomain polypeptides further comprises an F substitution at amino acid 190 and/or an L substitution at amino acid 207.
Embodiment 98. The method of embodiment 95, wherein the amino acid sequence of the RSVF ectodomain polypeptides further comprises S155C, S290C, S190F, and V207L substitutions.
Embodiment 99. The method of embodiment 57, wherein the oligomerization polypeptide that comprises a 6-helix bundle forming moiety.
Embodiment 100. The method of embodiment 99, wherein the C-terminal 6-helix bundle forming moiety comprises a heptad repeat region of the fusion protein of an enveloped virus.
Embodiment 101. The method of embodiment 100, wherein the heptad repeat region is selected from the group consisting of RSVF HRA domain, RSVF HRB domain, HIV gp41 HRA domain, and HIV gp41 HRB domain.
Embodiment 102. The method of any one of embodiments 99-101, wherein the six-helix bundle comprises the inserted RSVF HRA domain or RSVF HRB domain of three recombinant RSVF ectodomain polypeptides and the oligomerization region of each oligomerization polypeptide.
Embodiment 103. The method of any one of embodiments 57-102, wherein the RSVF ectodomain polypeptides in the complex that is produced are in the pre-fusion conformation.
Embodiment 104. The method of any one of embodiments 57-103, wherein the RSVF ectodomain polypeptides in the complex that is produced are characterized by a rounded shape when viewed in negatively stained electron micrographs.
Embodiment 105. The method of embodiment 57-104, wherein wherein the RSVF ectodomain polypeptides in the complex that is produced are characterized by D25 antibody binding.
Embodiment 106. The method of any one of embodiments 57-105, wherein the RSVF ectodomain polypeptides in the complex that is produced comprise prefusion epitopes that are not present on post-fusion forms of RSVF.
Embodiment 107. A method for producing a respiratory syncytial virus F (RSVF) complex comprising a six-helix bundle, comprising:
   a) providing RSVF protein ectodomain polypeptides comprising an endogenous RSVF HRA domain and an endogenous RSVF HRB domain that contain a C-terminal 6-helix bundle forming moiety comprising an inserted RSVF HRA domain and an inserted RSVF HRB domain, and
   b) combining the RSVF ectodomain polypeptides under conditions suitable for the formation of a RSVF complex, whereby a RSVF complex is produced that comprises three RSVF ectodomain polypeptides and is characterized by a six-helix bundle formed by the C-terminal 6-helix bundle forming moiety comprising the inserted RSVF HRA domain and an inserted RSVF HRB domain.
Embodiment 108. A respiratory syncytial virus F (RSVF) complex produced by the method of any one of embodiments 57-107.
Embodiment 109. A respiratory syncytial virus F (RSVF) complex of any one of embodiments 1-56 produced by the method of any of embodiments 57-107.
Embodiment 110. A population of respiratory syncytial virus F (RSVF) complexes of any one of embodiments 1-56 and 108.
Embodiment 111. The population of RSVF complexes of embodiment 110, wherein at least 60% of the complexes in the population are trimers.
Embodiment 112. The population of RSVF complexes of embodiment 110, wherein at least 70% of the complexes in the population are trimers.
Embodiment 113. The population of RSVF complexes of embodiment 110, wherein at least 80% of the complexes in the population are trimers.
Embodiment 114. The population of RSVF complexes of embodiment 110, wherein at least 90% of the complexes in the population are trimers.
Embodiment 115. The population of RSVF complexes of any one of embodiments 110-114, wherein at least 60% of the complexes in the population bind D25 antibody.
Embodiment 116. The population of RSVF complexes of any one of embodiments 110-114, wherein at least 70% of the complexes in the population bind D25 antibody.
Embodiment 117. The population of RSVF complexes of any one of embodiments 110-114, wherein at least 80% of the complexes in the population bind D25 antibody.
Embodiment 118. The population of RSVF complexes of any one of embodiments 110-114, wherein at least 90% of the complexes in the population bind D25 antibody.
Embodiment 119. The population of RSVF complexes of any one of embodiments 110-118, wherein at least 60% of the complexes in the population comprise a cleaved ectodomain polypeptide.
Embodiment 120. The population of RSVF complexes of any one of embodiments 110-118, wherein at least 70% of the complexes in the population comprise a cleaved ectodomain polypeptide.
Embodiment 121. The population of RSVF complexes of any one of embodiments 110-118, wherein at least 80% of the complexes in the population comprise a cleaved ectodomain polypeptide.
Embodiment 122. The population of RSVF complexes of any one of embodiments 110-118, wherein at least 90% of the complexes in the population comprise a cleaved ectodomain polypeptide.
Embodiment 123. The population of RSVF complexes of any one of embodiments 110-122, wherein the population of complexes is substantially free of lipids and lipoproteins.
Embodiment 124. An immunogenic composition comprising a respiratory syncytial virus F (RSVF) complex according to any one of embodiments 1-57 and 108-109, or a population of RSVF complexes of any of clams 110-123.
Embodiment 125. A method of inducing an immune response in a subject to RSVF comprising administering an immunogenic composition of embodiment 124 to the subject.
Embodiment 126. An RSVF ectodomain polypeptide comprising an endogenous HRA region, an endogenous HRB region, an inserted RSVF HRA region, and an inserted RSVF HRB region.
Embodiment 127. The RSVF ectodomain polypeptide of embodiment 126, wherein the inserted RSVF HRA region is C-terminal to the endogenous HRA region and the endogenous HRB region.
Embodiment 128. The RSVF ectodomain polypeptide of embodiment 126 or 127, wherein the inserted RSVF HRB region is C-terminal to the endogenous HRA region and the endogenous HRB region.
Embodiment 129. The RSVF ectodomain polypeptide of any of embodiments 126-128, wherein the inserted RSVF HRB region is C-terminal to the inserted RSVF HRA region.
Embodiment 128. The RSVF ectodomain polypeptide of any one of embodiments 126-128, further comprising a linker between the endogenous HRB region and the inserted RSVF HRA region or the inserted RSVF HRB region.
Embodiment 130. The RSVF ectodomain polypeptide of embodiment 129, wherein the linker limits pairing of the endogenous HRB region and the inserted RSVF HRA region.
Embodiment 131. The RSVF ectodomain polypeptide of any one of embodiments 126-128,further comprising a linker between the inserted RSVF HRA region and the inserted RSVF HRB region.
Embodiment 132. The RSVF ectodomain polypeptide of embodiment 131, wherein the linker promotes pairing of the inserted pairing of the inserted RSVF HRA region with the inserted RSVF HRB region.
Embodiment 133. The RSVF ectodomain polypeptide of embodiment 132, wherein the linker is a structurally constrained linker.
Embodiment 134. A polypeptide comprising an RSVF peptide having at least 90% sequence identity to an RSVF peptide sequence in the sequence listing wherein the peptide comprises a sequence that can form a part of a six helix bundle.
Embodiment 135. The polypeptide of embodiment 134, comprising an RSVF peptide sequence in the sequence listing.
Embodiment 136. A nucleic acid encoding a polypeptide of embodiment 134 or 135.
Embodiment 137. An expression vector comprising the nucleic acid of any one of embodiments 60-69 or 136.
Embodiment 138. An expression construct for use in the method of any one of embodiments 60-69.
Embodiment 139. A composition comprising a pair of expression constructs for use in the method of any one of embodiments 60-61 and 63-69.
Embodiment 140. A cell containing the nucleic acid of embodiment 136 or the expression vectors of embodiment 137 or 138.

### EXEMPLIFICATION

The following examples are merely illustrative of the scope of the present invention and therefore are not intended to limit the scope in any way.

### Example 1 -Purification Protocol for RSVF proteins from insect cells

Methods for protein expression in a number of cell systems are known in the art. Growth and purification of the proteins and complexes provided herein can be readily adapted to various cell types. Baculoviruses expressing RSVF constructs can be propagated and 6XHis (SEQ ID NO: 39) tagged RSVF proteins can be purified as follows:

One hundred microliters of P1 stock virus are added to 50 mls of SF9 cells (Invitrogen®) diluted to 0.8 x 106/ml (grown in Sf500 media) and allowed to infect/grow for approximately 5-6 days. The infection is monitored using the Cedex® instrument. Baculovirus growth is considered complete when cell viability was <50%, while cell diameter predominantly increases from ∼13 nm to ∼16nm.

One ml of P2 stock is added to 1 liter of Sf9 cells diluted to 0.8 x 10⁶/ml and was allowed to grow for 5-6 days. The infection is monitored using the Cedex® instrument. Baculovirus growth is considered complete when cell viability is <50%, while cell diameter predominantly increased from ∼13 nm to ∼16nm.

Expression is carried out in cultures of either Sf9 cellsor HiFive® cells (Invitrogen®) in which, unless a test expression is done to determine an appropriate m.o.i., 10 mls of P3 (passage 3) baculovirus stock is added to every liter of cells at 2 x 10⁶/ml. Expression is allowed to continue for ∼72 hours.

Cells are harvested, after taking an aliquot of cell/media suspension for SDS-PAGE analysis, by pelleting the cells from the media by centrifuging the cells at 3000 r.p.m. for ∼30 minutes.

Copper (II) sulfate is added to the media to a final concentration of 500 micromolar and 1 liter of media with copper is added to ∼15 mls of chelating IMAC resin (BioRad® Profinity).

Protein-bound resin is then separated from flow-through using a gravity column. The resin is washed with at least 10 resin volumes of equilibration buffer (25 mM Tris pH 7.5, 300 mM NaCl), and protein is eluted with at least 10 resin volumes of elution buffer (25 mM Tris pH 7.5, 300 mM NaCl, 250 mM imidazole).

The elution solution is spiked with EDTA-free complete protease inhibitor (Pierce®) and EDTA to a final concentration of 1 mM. The elution solution is then dialyzed at least twice at 4° C against 16 volumes of equilibration buffer. The elution solution is loaded onto one or two HiTrap® Chelating columns preloaded with Ni⁺⁺. (A single 5 ml column is typically sufficient for 10 liters of expression.) Protein is eluted from the column using an FPLC capable of delivering a gradient of elution buffer with the following gradient profile (2 ml/min flow rate)
a. 0 to 5% Elution buffer over 60 mls
b. 5 to 40% Elution buffer over 120 mls
c. 40 to 100% Elution buffer over 60 mls

Fractions containing RSVF protein are evaluated by SDS-PAGE analysis using Coomassie staining and/or western blotting (typically, RSVF elutes off ∼170 mls into the gradient): the material is concentrated to approximately 0.5-1 mg/ml; and EDTA is added to 1 mM final concentration

Using an FPLC, 1 ml fractions are collected. The RSVF material (retention volume approximately 75 ml) is resolved from the insect protein contaminates (retention time approximately 60 ml) by size exclusion chromatography (SEC) with a 16/60 Superdex column (GE® Healthcare) using with equilibration buffer as the mobile phase,.

Fractions are analyzed using SDS-PAGE with Coomassie staining and sufficiently pure RSVF material is pooled and concentrated to approximately 1 mg/ml.

### Example 2 - Chemical Synthesis of Oligomerization Peptides

Chemcial synthesis can be used for the generation of oligomerization peptides that are not expressed in cells, e.g., as a portion of the ectodomain peptide. HRA peptide or HRB peptide (the oligomerization polypeptide) is synthesized by Anaspec® (RSVF HRA peptide, RSV residues 160-207; RSVF HRB, RSV residues 474-525) is resuspended into SEC buffer (25 mM Tris pH 7.5, 300 mM NaCl) and UV absorbance at 280 nm (1 AU per 1 mg/ml: estimated) is used to estimate protein concentration.

### Example 3 - Formation of RSVF Complexes

An exemplary method of formation of the RSVF trimer complexes with a separate oligomerization domain are provided below. RSVF ectodomain peptides further comprising an inserted HRA domain are recombinantly expressed and purified using methods such as those provided above. Oligomerization domains containing HRB domains are synthesized chemically by a commercial provider.

To assemble the complexes, 0.5 mls of ∼0.75 mg/ml RSVF ectodomain polypeptide monomer containing an inserted HRA domain is added to 0.5 mls HRB containing oligomerization peptide solution, and 1 ml of the complex solution is separated on a SEC column according to the RSVF purification protocol. The principle peak migrates similarly to the RSVF trimer region. In general, the RSVF trimer protein (RSVF fusion peptide deletion) elutes in the trimer peak, the unlceaved RSV monomer elutes in the monomer peak. The principle peak for the RSVF monomer + HRA is more consistent with a trimer elution than a monomer. This shift in elution volume suggests peptide-F protein interaction and formation of a trimer of complexes between the HRA oligomerization peptides and the RSVF ectodomains (that is, a hetero-hexamer with three HRB peptides and three F uncleaved ectodomain polypeptides including an inserted HRA domain).

This uncleavable ectodomain F:HRA peptide complex is evaluated by electron microscopy (EM) to determine if a three-lobed species or a prefusion globular head is formed. Alternatively, the complex is analyzed by antibody binding and SEC as described below using D25 Fab to detect RSVF proteins with prefusion structures and the Motavisumab Fab to detect total RSVF protein (i.e., prefusion and post-fusion). Additionally, the peptide complex formation is repeated with cleavable RSVF ectodomain that can be protease digested to F 1/F2 species. If the prefusion F globular head is formed, and this prefusion RSVF behaves similarly to parainfluenza F, we expect that stabilizing the prefusion form will prevent rosette formation.

### Example 4 - Addition of a C-terminal 6-helix bundle forming sequence

A sequence, such as an additional RSV HRB or HIV gp41 HRB is added to the complex described in Example 2 to form a C-terminal 6-helix bundle, thus permitting trimerization with inserted of RSV HRA or HIV gp41 HRA, respectively. This may have an additional advantage of constraining native RSV HR from the monomer into its native prefusion HRB trimer stalk, instead of the postfusion-like 6-helix bundle.

### Example 5 - Addition of HRB disulfides

Disulfides are added to the synthetic oligomerization domain described in Example 2. Thus, when trimerization of the monomer occurs, the cysteine additions are in appropriate positions to form the desired disulfides between the oligomerization domains and the subunits, providing an additional level of prefusion stability.

### Example 6 - Addition of conjugated proteins fused to peptides

Instead of adding HRA, HRB, or gp41 peptides alone (Example 3), conjugated proteins fused with functional peptides are added, such as RSV G, albumin, or KLF conjugate protein. For example, an HRA peptide-RSV G central domain construct is added to the F ectodomain protein containing an inserted HRB. Upon trimerization induced by the HRA peptide, the RSV G central domain protein is bound to F making an F/G complex, which may provide further immunogenicity upon vaccination.

### Example 7 - Trimerization of RSVF monomers with HRA peptides.

In this example, RSVF monomers (a Delp23 Furdel Truncated HIS construct) containing an inserted RSV HRB domain are mixed with 5-fold mass of RSV HRA synthetically produced peptides (SEQ ID NO: 40), using the same method as described in Example 2.

A MALS analysis is performed using Wyatt® SEC column and Waters® HPLC with PBS as mobile phase of RSVF monomers prior to and after mixing with RSV HRA peptides. The retention time of the major peak of RSVF monomers is shifted from to an earlier time upon addition of HRA peptides. This shift is roughly consistant with a monomer being transformed to a trimer upon addition of the peptide, indicating that HRA peptides trimerized the RSVF monomers.

### Example 8 - RSVF monomer binding to prefusion-specific antibody D25 Fab demonstrated with BIAcore™ analysis

McLellan, J.S. et al. (Science, 340:1113-7 (2013)) disclosed the crystal structure of RSVF in its prefusion conformation bound to the Fab of an antibody D25 which binds to epitopes unique to the prefusion conformation but not present on the postfusion structure.

One can test if the RSVF monomer is a prefusion precursor (i.e. a folded protein able to bind prefusion-specific antibodies or Fabs) by binding the protein to D25 Fab, e.g., by ELISA, surface plasmon resonance analysis such as BIAcore™, ITC, size exclusion chromatography (SEC), shift by native gel electrophoresis, AUC, Western or dot blot, etc.

The D25 Fab was generated for this study using the sequence disclosed in McLellan, J.S. et al., 2013 harboring a HIS-tag and Strep-tag used for purification in E. coli cells using conventional laboratory methods.

In this example a BIAcore™ analysis is carried out which demonstrated specific binding of RSVF monomer (uncleaved F) by D25 Fab. D25 Fab is immobilized on a CM5 chip using standard amide chemistry as described by the operator's manual (BIAcore™/GE Healthscience). D25 Fab is loaded to ∼75 RU on the chip surface. RSVF monomer (uncleaved RSVF Delp23 Furdel) is diluted in BIAcore™ mobile phase (PBS with 0.05% N20 detergent) to 30 nM, 20 nM, 15 nM, 10 nM, 7.5 nM, 5 nM 3.75 nM, 0.5 nM and 0 nM concentrations. Sensorgrams of binding are recorded against a double blank (Initial sensorgrams represent F2-F1 channel where F2 is D25 immobilized channel and F1 is no protein channel treated with amine coupling. The 0 nM initial sensorgram is immediately subtracted from each of the other sensorgrams to generate the final concentration sensorgrams used for fitting.) The binding constant and error are determined by fitting to a 1:1 binding model using the BIAcore™ Evaluation software.

BIAcore™ analysis demonstrates that RSVF monomer is able to bind the prefusion-specific antibody D25. In addition, the tight binding data suggests that the prefusion epitope is preformed on the protein surface. D25 is known to bind tightly to prefusion RSVF (McLellan, J.S. *et al*., 2013). If RSVF monomer is in the prefusion conformation, one would expect the binding affinity to be very tight, in the uM range or tighter.

RSVF uncleaved subunit antigen (F monomer) is first demonstrated to be in the prefusion conformation in this work. This antigen should elicit a supperior immune response to the previously published postfusion antigens.

### Example 9 - RSVF monomer binding to D25 Fab demonstrated with SEC

Size exclusion chromatography is useful for demonstrating binding of antigens and antibodies. This is typically done either with preparatory chromatography (i.e. Superdex P200 or Superdex 200 PC 3.2/30) or on analytical HPLC such as Wyatt MALS SEC column.

An analytical HPLC-SEC is performed on RSVF monomer with or without addition of a 1:1 molar amount of D25 Fab. SEC can be run on a Waters® MALS system as described in Example 6 with PBS as the mobile phase to detect a shift in the RSVF monomer to a new retention time which is consistent with a mass of RSVF monomer bound to a D25 Fab as demonstrated by Stokes radius and MALS analysis. In particular, analysis of RSVF monomer (Delp23 Furdel) without Fab using analytical sizing chromatography HPLC shows a specific retention time that is deceased upon the addition of D25 Fab at a 1:1 molar ratio. A (near-)complete shift of RSVF monomer to a new retention time indicates that nearly all the RSVF monomer is competent to bind prefusion-specific D25 Fab, suggesting that the RSVF monomer is fairly homogeneous in its prefusion conformation.

### Example 10 - RSVF monomer and trimer complex binding to D25 Fab demonstrated with size exclusion chromatography (SEC)

In this example a preparatory SEC is performed to demonstrate that RSVF monomer and trimer are prefusion antigens. The experiment is performed with a microFPLC (GE® Healthcare) using 25 mM Tris pH 7.5 and 50 mM NaCl as mobile phase. RSVF monomer (uncleaved RSVF Delp23 Furdel) is run on SEC preparatory column (micro Superdex® 200 by GE® Healthcare) and the retention time of the elution peak is noted. Adding D25 Fab to RSV monomer at 1:1 molar ratio and running on SEC column results in a shift of the principle peak to a lower retention volume consistent with RSVF monomer:D25 Fab at 1:1 ratio, with an additional double peak at at higer retention volume which is consistent with the retention volume of unbound. Such results would show that RSVF monomer is able to bind prefusion-specific Fab and is likely prefusion antigen.

### Example 11 - Formation of RSVF complexes with linked oligomerization domains

Exemplary RSVF constructs comprising ectodomains comprising an endogenous HRA domain and an endogenous HRB domain further comprising an inserted HRA domain and an inserted HRB domain are shown in Figure 4. The Arm 1, 2, and 3 groups of proteins contain an RSVF ectodomain sequence with S155C, S290C, S190F, and V207L substitutions within the ectodomain, and the indicated inserted and oligomerization domains shown. The peptides were expressed in 293 cells and purified from conditioned media. Trimerization was assayed by western blot and size exclusion chromatography (SEC) of antibody bound complexes. The structure of the RSVF domain in the trimers was assayed using the D25 antibody, which recognizes RSVF in the pre-fusion conformation, and the motavizumab antibody, which recognizes RSVF in both the pre- and the post-fusion conformations, in ELISA and in SEC methods.

Exemplary constructs were demonstrated to trimerize. Further, the trimers were recognized by both the D25 and motavizumab antibodies.

These results demonstrate that RSVF trimers are formed by RSVF ectodomain polypeptides further comprising an inserted HRA domain and HRB oligomerization domain. Further, the results demonstrate that the RSVF ectodomain has a pre-fusion structure in the trimerized form.

### Example 12-- Coexpression and assembly of RSVF ectodomain and oligomerization domains in cells

RSVF ectodomains provided herein were expressed in 293 cells from various expression constructs containing the the S155C, S290C, S190F, and V207L substitutions in the endogenous ectodomain sequence.. Plasmids encoding RSVF constructs comprising ectodomains with an endogenous HRA and endogenous HRB and an inserted HRA were co-transfected with a separate plasmid expressing the RSVF HRB oligomerization domain peptide sequence. The transfections were carried out with ratios of DNA encoding RSVF ectodomain with inserted HRA to DNA encoding the HRB peptide format ratios of 1:1, 1:2, 1:3 or 1:4 while keeping the total amount of transfected DNA constant. IRES constructs were also generated for expression of the ectodomain peptides and the oligomerization domain peptides from a single construct. The total amount of transfected DNA was the same as in the co-transfected wells. For constructs containing an endogenous HRA, an endogenous HRB, an inserted HRA, and an attached HRB oligomerization domain, the total amount of transfected DNA was the same as in the co-transfected wells.

Purification tags such as single or double strep or 6-His tags were present on the RSVF ectodomain constructs C-terminal to the inserted HRA (or the inserted HRB when both an inserted HRA and an HRB oligomerization domain were present in the construct), or on the HRB oligomerization peptide construct N-terminal to the HRB sequence. Proteolytic cleavage sites such as those recognized by thrombin or TEV protease were present between the purification tag (if present) and the HRB peptide in the oligomerization domain to allow for removal of the tag after purification using routine methods. Purification tags were preferably removed prior to assessing trimerization status of the complexes

The constructs for the RSVF ectodomain polypeptides included the native RSVF leader sequence. IgK leader sequences were included in the oligomerization domain HRB peptide oligomerization domain constructs. These sequences ensure the proper targeting of the peptide into the endoplasmic reticulum where it can assemble into trimers with the RSVF ectodomain construct and promote secretion of the assembled complex into the growth medium to facilitate production and purification. The leader sequences were encoded N-terminal to the purification tag, if present.

Protein expression from various constructs was assessed using western blotting with an anti-RSV antibody. When the six helix bundle containing the inserted HRA in the RSVF ectodomain and the HRB oligomerization domain peptide assembles, the trimerized molecule runs as a discrete band on SDS-PAGE at the size of a trimer (∼200 kDa) with minimal protein running at the size of the monomer. Under boiled and reduced conditions, the band in western blot is detected at the size of a monomer of the RSVF ectodomain (∼60-70 kDa).

The oligomerization status of the expressed protein was also assessed in expression media using a fluorescence HPLC technique. D25 Fab was labeled with a fluorescent dye such as Alexa® Fluor 488 and 1ug of labeled Fab was incubated with 100uL of expression media. The complex was then resolved over a HPLC column (Waters® 2695, Separations Module; Column Type: Agilent® Bio SEC-3, 300A, 7.8x300mm; cat: 5190-2511; Serial #: USDLV01746) in DPBS (1X) buffer (Cat No: 14190-144, Invitrogen®), detected using an appropriate detector (Waters® 996, Photodiode Array Detector), and the retention time was noted. Retention times were compared to the elution time of control monomer and trimer complexes. The retention time for a well-folded trimer was determined based on the DS-CAV1 construct of McClellan et al., 2013 which contains a foldon trimerization domain (in this example, around 6.3 minutes) and for a well-folded prefusion monomer (delta p23 furdel, around 7.1 minutes). Presence of higher molecular weight aggregates, when present, are indicated by peak(s) eluting before 6.3 minutes. The shape of the elution curve was also considered with a smooth, well-formed peak being indicative of a homogenous population of well-formed trimers.

The assay simultaneously confirmed the presence of trimers in the prefusion conformation. The assay was repeated with similarly-labeled motavizumab Fab, which recognizes both prefusion and postfusion F.

Cells expressing the antigen stably can be made for this strategy by selecting for clones that express the RSVF ectodomain with inserted HRA and the HRB peptide constructs either from two separate plasmids with different mammalian selection markers such as DHFR, or stable clones transfected with a single vector encoding for the RSVF ectodomain and the HRB peptide from two separate promoters on the same plasmid.

The presence of a purification tag such as a single or double Strep or 6-His tag on the separately expressed HRB peptide allows for easy purification of the fully-formed prefusion F trimer with the six helix bundle trimerization domain being composed of the inserted HRA and the HRB oligomerization domain peptide. Any excess HRB oligomerization domain peptide alone that copurifies is easily removed in SEC due to the vast difference in size between the two species. This purification scheme also ensures that any trimer that forms due to the interaction of the inserted HRA with the upstream endogenous HRB is not captured (due to lack of a purification tag on such a construct). This in turn ensures that the purified protein is a homogenous population.

The constructs are designed with a proteolytic cleavage site such as a thrombin site between the purification tag and the protein or HRB peptide. Once the tag is cleaved, the protein contains substantially no additional heterologous sequences and hence the immune response can be focused solely on the sites present on RSVF.

### Example 13-- Expression of RSVF complexes with various linker lengths and attached oligomerization domains

The RSV F ectodomain constructs described in Example 11 and in Figure 4 contain two inserted linker sequences, the first between the endogenous HRB and the inserted HRA (linker 1), and the second between the inserted HRA and the HRB oligomerization domain (linker 2), if present. The length and composition of the linkers can have an effect on the correct assembly of the trimer complex. For example, linker 2 can have an effect by favoring the folding back of the HRB oligomerization domain on the upstream inserted HRA of the same monomer, thereby preventing formation of aggregates. Similarly, linker 1 can also have an effect by disfavoring the inserted HRA from interating with the upstream endogenous HRB. Various linker lengths were tested in the two positions, with lengths varying from 7 to 21 residues. The constructs included the the S155C, S290C, S190F, and V207L substitutions in the endogenous ectodomain sequence.

Based on the levels of trimers secreted into growth media, a longer sequence was favored for linker 1 as assessed by fluorescence HPLC detailed in Example 12. Without wishing to be bound by theory, it is suggested that by providing enough distance between the endogenous HRB and the inserted HRA, the longer linker favors interaction between the inserted HRA domain and the HRB oligomerization domain. However, a shorter sequence was favored for linker 2. Longer linkers in this position had a higher proportion of an earlier eluting peak in fluorescence HPLC, indicative of potential formation of higher order oligomers.

The linker 2 sequence can be manipulated to further favor interaction between the inserted HRA domain and the HRB oligomerization domain. For example, the linker sequence can be shortened and/or altered introduce a bend in the protein chain through the use of Pro-Gly sequences and/or disulfide bonds to favor interaction of the HRB-containing oligomerization domain with the upstream inserted HRA domain.

### Example 14-- Expression of RSV F complexes with various linker lengths with detached oligomerization domains

As demonstrated above, linker 2 can play a role in determining the proportion of the expressed protein that forms a well-folded trimer versus higher order oligomers due to possibility of the inserted HRA domain and the oligomerization domain HRB from forming complexes other than properly folded trimers. In order to eliminate that possibility, a second strategy was employed where the HRB oligomerization domain was expressed as a separate polypeptide, either from a separate plasmid, or from the same mRNA with an IRES between the RSV F and the HRB peptide. The constructs included the the S155C, S290C, S190F, and V207L substitutions in the endogenous ectodomain sequence.

A longer sequence for linker 1 again favored higher expression for the trimer with a separate HRB oligomerization domain, however, the effects of the length of linker 1 on the expression of constructs with detached oligomerization domains was less striking than on the expression of constructs with the oligomerization domain attached.

### Example 15-- Expression of RSV F complexes with various ratios of detached oligomerization domains

Assays were preformed to assess the effect of changes in the ratio of ectodomain polypeptides to oligomerization polypeptides on expression levels (ratios of 1:1 to 1:4 of ectodomain:oligomerization domain). Various ratios of plasmid expression constructs containing either a coding sequence for ectodomain polypeptides containing the S155C, S290C, S190F, and V207L substitutions in the context of an endogenous RSV F HRA, an endogenous RSV F HRB, and an inserted RSV F HRA domain; and a coding sequence oligomerization polypeptide containing an RSV F HRB domain were cotransfected into 293 cells as described above, maintaining the total DNA constant. IRES constructs were also used to co-express the ectodomain polypeptides and oligomerization peptides in a cell wherein the coding sequences were present at a 1:1 ratio.

Expression level of assembled trimers in growth media were assayed by western blot. A 1:2 ratio of ectodomain to oligomerization domain was found to provide somewhat higher levels of expression of the RSV F complex. However, expression of the complexes was detectable at all ratios (1:1-1:4).

### Example 16 - Expression of RSV F Complexes with Internal Ectodomain Furin Cleavage Sites

The sequence of the wild-type RSV F protein contains two internal furin cleavage sites separated by a 27-residue stretch, designated p27. This p27 peptide is excised during protein folding in the ER by the cellular protease furin resulting in a mature protein made up of two distinct polypeptide chains, F1 and F2, linked together by disulfide bonds. Cleavage at both sites to remove the p27 peptide is required for the secretion of the mature protein into cell culture media as uncleaved F ectodomains containing the p27 sequence remain trapped inside the cell. A set of constructs where F1 is linked to F2 by a single furin cleavage site in between, with the p27 sequence deleted were generated to optimize the furin cleavage site. The constructs were designed such that the mature processed protein would be virtually identical to the final processed RSV F wild-type protein. The constructs included the the S155C, S290C, S190F, and V207L substitutions in the endogenous ectodomain sequence.

The two furin cleavage sites in wild-type RSV F have different sequences. Furin cleavage site 1, which has the sequence RARR (SEQ ID NO: 109), results in cleavage at amino acid 109 at SEQ ID NO: 1 and furin cleavage site 2, which has the sequence RKRR (SEQ ID NO: 110), results in cleavage at amino acid 136 of SEQ ID NO: 1. Constructs were generated in which the p27 sequence was deleted and replaced with the sequence of either the furin cleavage site 1 or furin cleavage site 2. Upon expression in cells, furin cleavage site 2 was much more efficiently cleaved than furin cleavage site 1. Although uncleaved, p27 containing RSV F polypeptides are not secreted from the cell, an uncleaved population of p27 deleted constructs with single furin sites were secreted into cell culture media. Without wishing to be bound by theory, it is suggested that secretion of the peptides was possible due to the ability of the protein to still fold correctly in the absence of the p27 sequence.

Having determined that the RKRR (SEQ ID NO: 110) protease cleavage site was more efficiently cleaved than the RARR (SEQ ID NO: 109) cleavage site, constructs were generated to further optimize the cleavage site. Specifically, mutations were made in which a serine and optionally a glycine were placed immediately C-terminal to the RKRR cleavage site, either by mutation of the position or insertion of amino acids next to the cleavage site. This Ser either replaced Phe137 (start of fusion peptide) or was an insertion N-terminal to it. Additional amino acids were inserted either before or after the furin site to ensure that the protein chain would not be constrained in length for proper folding if cleavage were to not occur. Mutants with a combination of furin cleavage site 2 where Phe137 was replaced with a Ser or where a Ser was inserted between the cleavage site and Phe137 had higher levels of expression as compared to the parental DS-CAV1 foldon construct with the two endogenous furin cleavage sites flanking the p27 sequence. An advantage of the inclusion of a single furin cleavage site is that the final processed RSV F polypeptide is nearly identical to the parental protein with the exception of A107K in the improved furin site, and a Ser replacing the Phe within the buried fusion peptide.

A summary of the experimental results from Examples 9-16 is provided in the table below.

| **Construct** | **Appearance in western blot with anti-RSV polyclonal antibody** | **Fluorescence HPLC profile** |
|---|---|---|
| RSV F DS-CAV1 with Foldon (after removal of purification tag with Thrombin) (SEQ ID NO: 105) | Single band at size of monomer (F1+F2; 60kDa) in non-boiled / non-reduced conditions. Single band at size of F 1 in boiled/reduced conditions | Single clean peak eluting at 6.3 minutes when complexed with labeled D25 or Motavizumab Fab (after cleaving of tag) |
| HRA short linker NO strep HIS (SEQ ID NO: 68) + Cleavable 2xStrep HRB 1:2 (SEQ ID NO: 89) | Single band at size of trimer in NB/NR conditions. Single band at size of F1 in B/R conditions | Major peak eluting at 6.4 minutes with a minor peak at 5.7 minutes when complexed with labeled D25 Fab |
| HRA long linker NO strep HIS (SEQ ID NO: 70) + Cleavable 2xStrep HRB 1:2 (SEQ ID NO: 89) | Single band at size of trimer in NB/NR conditions. Single band at size of F1 (∼50kDa) in B/R conditions | Major peak eluting at 6.4 minutes with a minor peak at 5.7 minutes when complexed with labeled D25 Fab. Higher peak than R209+R240. |
| HRA-HRB short linker short linker strep HIS (SEQ ID NO: 71) | Mixed population of two major bands, one around size of trimer, one at size of monomer (F1+F2) in NB/NR conditions. Single band at ∼70kDa in B/R conditions | Two peaks at 5.3 minutes and 6.0 minutes when complexed with labeled D25 Fab. |
| HRA-HRB short linker long linker strep HIS (SEQ ID NO: 73) | Mixed population of two major bands, one around size of trimer, one at size of monomer (F1+F2) in NB/NR conditions. Single band at ∼70kDa in B/R conditions. | Two peaks at 5.3 minutes and 6.0 minutes when complexed with labeled D25 Fab. |
| HRA-HRB long linker short linker strep HIS (SEQ ID NO: 75) | Mixed population of two major bands, one around size of trimer, one at size of monomer (F1+F2) in NB/NR conditions. Single band at ∼70kDa in B/R conditions | Two peaks at 5.3 minutes and 6.0 minutes when complexed with labeled D25 Fab. |
| RSV DSCAV1 HRA-HRB long linker long linker strep HIS (SEQ ID NO: 57) | Mixed population of two major bands, one around size of trimer, one at size of monomer (F1+F2) in NB/NR conditions. Single band at ∼70kDa in B/R conditions. | Two peaks at 5.3 minutes and 6.0 minutes when complexed with labeled D25 Fab. |
| Single furin sitel delta p27 DS CAV1 (SEQ ID NO: 100) | Two bands around with a major band at 70kDa (uncleaved F1+F2) and a minor band at 50kDa (cleaved F1) under B/R conditions (highlighting incomplete cleavage) | Not tested |
| Single furin site2 delta p27 DS CAV1 (SEQ ID NO: 101) | Two bands around with a minor band at 70kDa (uncleaved F1+F2) and a major band at 50kDa (cleaved F1) under B/R conditions (highlighting incomplete cleavage, but better than R236) | Not tested |
| Single furin site2 plus S delta p27 DS CAV1 (SEQ ID NO: 102) | Two bands at 70 kDa (F1+F2) and 50 kDa (F1) of equal intensity in B/R conditions highlighting incomplete cleavage. | Single clean peak eluting at 6.3 minutes when complexed with labeled D25 Fab (after cleaving purification tag with Thrombin in expression media) |
| Single furin site2 F137S delta p27 DS CAV1 (SEQ ID NO: 103) | Single band at 50kDa (F1) under B/R conditions showing complete cleavage of F1 and F2. Single band at ∼70kDa under NB/NR conditions. Higher expression than R196. | Single clean peak eluting at 6.3 minutes when complexed with labeled D25 Fab (after cleaving purification tag with Thrombin in expression media) |
| FLD006 Single furin site2 plus SG delta p27 DS CAV1 (SEQ ID NO: 104) | Two bands at 70 kDa (F1+F2) and 50 kDa (F1) of equal intensity in B/R conditions highlighting incomplete cleavage. | Single clean peak eluting at 6.3 minutes when complexed with labeled D25 Fab (after cleaving purification tag with Thrombin in expression media) |
| Single furin site2 with G before site F137S after site delta p27 DS CAV1 (SEQ ID NO: 105) | Single band at 50kDa (F1) under B/R conditions showing complete cleavage of F1 and F2. Single band at ∼70kDa under NB/NR conditions. Higher expression than R196. | Single clean peak eluting at 6.3 minutes when complexed with labeled D25 Fab (after cleaving purification tag with Thrombin in expression media) |

A brief description of the sequences provided in the sequence listing with their corresponding SESQ ID NO is provided in the table below.

**SEQUENCE TABLE**

| **SEQ ID** | **Description** |
|---|---|
| 1 | RSVF A2 strain; GenBank GI: 138251; Swiss Prot P03420 |
| 2 | RSVF 18537 strain; GI: 138250; Swiss Prot P13843 |
| 3 | Modified RSVF fragment, Furmt |
| 4 | Modified RSVF fragment, Furdel |
| 5 | Modified RSVF fragment, Furx |
| 6 | Modified RSVF fragment, Furx R113Q, K123N, K124N |
| 7 | Modified RSVF fragment, Furx R113Q, K123Q, K124Q |
| 8 | Modified RSVF fragment, Del21 Furx |
| 9 | Modified RSVF fragment, Delp23Furx |
| 10 | Modified RSVF fragment, Delp21 furdel |
| 11 | Modified RSVF fragment, Delp23 furdel |
| 12 | Modified RSVF fragment, N-term Furin |
| 13 | Modified RSVF fragment, C-term Furin |
| 14 | Modified RSVF fragment, inserted Factor Xa site |
| 15 | Modified RSVF fragment (aa 100-150) |
| 16 | GST fusion - RSV HRA, oligmerization peptide of HRA |
| 17 | GST fusion - RSV truncated HRA, oligmerization peptide of HRA |
| 18 | GST fusion - HRA oligomerization sequence - RSV G from strain b (RSV Gb CC HRA short) |
| 19 | HRA oligomerization sequence- RSV G from strain a (RSV Ga CC HRA short) |
| 20 | HRB oligomerization sequence - RSV G from strain b (RSV Gb CC HRB) |
| 21 | HRB oligomerization sequence - RSV G from strain a (RSV Ga CC HRB) |
| 22 | RSV F ectodomain sequence - inserted C-terminal HRA sequence - linker - HIS tag (RSV F delP23 furdel Trunc HRA HIS) |
| 23 | RSV F ectodomain sequence - inserted C-terminal HRA sequence - linker - HIS tag (RSV F delP23 furdel C509C510 C481C489 HRA HIS) |
| 24 | Consensus of F protein sequences |
| 25 | RSVF fragment, wild-type |
| 26 | Modified RSVF fragment, fusion peptide deleted |
| 27 | A2, F protein of the strain A2 (accession number AF035006) |
| 28 | CP52, F protein of the CP52 strain (accession number AF013255) |
| 29 | B, F protein of the B strain (accession number AF013254) |
| 30 | Long, F protein of the long strain (accession number AY911262) strain |
| 31 | 18537strain, F protein of the 18537 strain |
| 32 | Altered furin cleavage site |
| 33 | Altered furin cleavage site |
| 34 | Altered furin cleavage site |
| 35 | Altered furin cleavage site |
| 36 | Altered furin cleavage site |
| 37 | Altered furin cleavage site |
| 38 | Altered furin cleavage site |
| 39 | 6X His affinity tag |
| 40 | HRA oligomerization peptide |
| 41 | Truncated HRB |
| 42 | HRA |
| 43 | HRB |
| 44 | RSV DS-CAV1 |
| 45 | Short linker |
| 46 | Long linker |
| 47 | RSV DSCAV1 HRA short linker strep HIS (Arm 1) |
| 48 | RSV DSCAV1 HRA short linker NO strep HIS |
| 49 | RSV DSCAV1 HRA long linker strep HIS |
| 50 | RSV DSCAV1 HRA long linker NO strep HIS |
| 51 | RSV DSCAV1 HRAHRB short linker short linker strep HIS (Arm 2) |
| 52 | RSV DSCAV1 HRAHRB short linker short linker NO strep HIS |
| 53 | RSV DSCAV1 HRAHRB short linker long linker strep HIS |
| 54 | RSV DSCAV1 HRAHRB short linker long linker NO strep HIS |
| 55 | RSV DSCAV1 HRAHRB long linker short linker strep HIS |
| 56 | RSV DSCAV1 HRAHRB long linker short linker NO strep HIS |
| 57 | RSV DSCAV1 HRAHRB long linker long linker strep HIS |
| 58 | RSV DSCAV1 HRAHRB long linker long linker NO strep HIS |
| 59 | RSV DSCAV1 shHRB HRAHRB short linker short linker strep HIS (Arm 3) |
| 60 | RSV DSCAV1 shHRB HRAHRB short linker short linker NO strep HIS |
| 61 | RSV DSCAV1 shHRB HRAHRB short linker long linker strep HIS |
| 62 | RSV DSCAV1 shHRB HRAHRB short linker long linker NO strep HIS |
| 63 | RSV DSCAV1 shHRB HRAHRB long linker short linker strep HIS |
| 64 | RSV DSCAV1 shHRB HRAHRB long linker short linker NO strep HIS |
| 65 | RSV DSCAV1 shHRB HRAHRB long linker long linker strep HIS |
| 66 | RSV DSCAV1 HRAHRB long linker long linker NO strep HIS |
| 67 | HRA short linker strep HIS |
| 68 | HRA short linker NO strep HIS |
| 69 | HRA long linker strep HIS |
| 70 | HRA long linker NO strep HIS |
| 71 | HRAHRB short linker short linker strep HIS |
| 72 | HRAHRB short linker short linker NO strep HIS |
| 73 | HRAHRB short linker long linker strep HIS |
| 74 | HRAHRB short linker long linker NO strep HIS |
| 75 | HRAHRB long linker short linker strep HIS |
| 76 | HRAHRB long linker short linker NO strep HIS |
| 77 | HRAHRB long linker long linker strep HIS |
| 78 | HRAHRB long linker long linker NO strep HIS |
| 79 | Encoded upstream sequence in IRES constructs SEQ ID NO: 80-83 and downstream sequence in IRES constructs SEQ ID NO: 84-87 |
| 80 | Encoded downstream sequence in Cleavable Strep HRB IRES RSVF DS-CAV1 short linker HRA |
| 81 | Encoded downstream sequence in Cleavable Strep HRB IRES RSVF DS-CAV1 long linker HRA |
| 82 | Encoded downstream sequence in Cleavable Strep HRB IRES RSVF DS-CAV1- shHRB short linker HRA |
| 83 | Encoded downstream sequence in Cleavable Strep HRB IRES RSVF DS-CAV1- shHRB long linker HRA |
| 84 | Upstream sequence in RSVF DS-CAV1 short linker HRA IRES Cleavable Strep HRB |
| 85 | Encoded downstream sequence in RSVF DS-CAV1 long linker HRA IRES Cleavable Strep HRB |
| 86 | Encoded downstream sequence in RSVF DS-CAV1 shHRB short linker HRA IRES Cleavable Strep HRB |
| 87 | Encoded downstream sequence in RSVF DS-CAV1 shHRB long linker HRA IRES Cleavable Strep HRB |
| 88 | Thrombin cleavage site |
| 89 | Cleavable 2xStrep HRB |
| 90 | Cleavable His HRB |
| 91 | Single furin site2 F137S delta p27 R208 (DS-CAV1 short linker HRA Strep His) |
| 92 | Single furin site2 F137S delta p27 R209 (DS-CAV1 short linker HRA no tag) |
| 93 | Single furin site2 F137S delta p27 R210 (DS-CAV1 long linker HRA Strep His) |
| 94 | Single furin site2 F137S delta p27 R211 (DS-CAV1 long linker HRA no tag) |
| 95 | Single chain R208 (DS-CAV1 short linker HRA Strep His) |
| 96 | Single chain R209 (DS-CAV1 short linker HRA no tag) |
| 97 | Single chain R210 (DSCAV1 long linker HRA strep HIS) |
| 98 | Single chain R211 (DSCAV1 long linker HRA no tag) |
| 99 | R196 DS-CAV1 with A107K, E110S, F137S |
| 100 | Single furin site1 deltap27 DS CAV1 |
| 101 | Single furin site2 delta p27 DS CAV1 |
| 102 | Single furin site2 plus S delta p27 DS CAV1 |
| 103 | Single furin site2 F137S delta p27 DS CAV1 |
| 104 | Single furin site2 plus SG delta p27 DS CAV1 |
| 105 | Single furin site2 with G before site F137S after site delta p27 DS CAV1 |
| 106 | DS-CAV1-with foldon |
| 107 | IgKappa leader sequence |
| 108 | Strep tag |
| 109 | Furin cleavage site 1 |
| 110 | Furin cleavage site 2 |
| 111 | Pro-gly sequence |

## Claims

1. A respiratory syncytial virus F (RSVF) complex, comprising a six helix bundle, comprising:
three RSVF ectodomain polypeptides each comprising an endogenous HRA region, an endogenous HRB region, further comprising one of an inserted RSVF HRA region or an inserted RSVF HRB region and
at least one oligomerization polypeptide, wherein the three ectodomain polypeptides and the at least one oligomerization polypeptide form a six-helix bundle, with the proviso that the six-helix bundle does not include the endogenous HRA regions and endogenous HRB regions of the RSVF polypeptides, wherein the inserted HRA region or inserted HRB region is optionally operably linked C-terminal to the ectodomain endogenous RSVF HRB region in the RSVF ectodomain polypeptide.

2. The RSVF complex of claim 1, wherein the six helix bundle comprises the inserted HRA region of each RSVF ectodomain polypeptide and the oligomerization polypeptide comprises an HRB region; or the the six helix bundle comprises the inserted HRB region of each RSVF ectodomain polypeptide and the oligomerization polypeptide comprises an HRA region.

3. The RSVF complex of claim 1 or 2, wherein the oligomerization polypeptide is not operably linked to an RSVF ectodomain polypeptide.

4. The RSVF complex of claim 1 or 2, wherein the oligomerization polypeptide is operably linked to at least one RSVF ectodomain polypeptide.

5. The RSVF complex of any of claims 1-4, wherein at least one operable link comprises a polypeptide linker and/or a structurally constrained linker.

6. The RSVF complex of any one of claims 1-6, wherein the inserted six-helix bundle forming moiety and the oligomerization polypeptide comprise complementary heptad repeat regions of a fusion protein of an enveloped virus, optionally selected from the group consisting of an RSVF HRA and an RSVF HRB; an HIV gp41 HR1 region and an HIV gp41 HR2 region; Newcastle disease virus (NDV) HR1 and NVD HR2, human metapneumovirus HR1 and human metapneumovirus HR; and other class 1 fusion viral proteins that contain 6-helix bundles in their postfusion state.

7. The RSVF complex of any of claims 1-6, wherein the at least one oligomerization polypeptide comprises three oligomerization polypeptides.

8. The RSVF complex of any of claims 1-7, wherein the oligomerization polypeptide comprises a purification tag.

9. The RSVF complex of any one of claims 1-8, wherein one or more of the RSVF ectodomain polypeptides comprises an uncleaved RSVF ectodomain polypeptide or a cleaved RSVF ectodomain polypeptide.

10. The RSVF complex of any one of claims 1-9, wherein each of the RSVF ectodomain polypeptides comprises a protease cleavage site.

11. The RSVF complex of claim 10, wherein the protease cleavage site comprises a furin cleavage site.

12. The RSVF complex of any of claims 1-11, wherein the amino acid sequence of the RSVF ectodomain polypeptides further comprises substitutions selected from the group consisting of S155C and S290C substitutions; S190F, and V207L substitutions; and S155C, S290C, S190F, and V207L substitutions.

13. A method for producing a respiratory syncytial virus F (RSVF) complex comprising a six-helix bundle, comprising:
a) providing RSVF protein ectodomain polypeptides comprising an endogenous HRA region and an endogenous HRB region, further comprising a six helix bundle forming moiety; and at least one oligomerization polypeptide, and
b) combining the RSVF ectodomain polypeptides and the at least one oligomerization polypeptide under conditions suitable for the formation of an RSVF complex, whereby an RSVF complex is produced in which three of the RSVF ectodomain polypeptides and at least one of the oligomerization polypeptides form a six-helix bundle, with the proviso that the endogenous HRA regions and the endogenous HRB regions of the RSVF ectodomain polypeptides are not part of the six-helix bundle.

14. The method of claim 13, wherein the inserted six-helix bundle forming moiety and the oligomerization polypeptide comprise complementary heptad repeat regions of a fusion protein of an enveloped virus, selected from the group consisting of an RSVF HRA and an RSVF HRB; an HIV gp41 HR1 region and an HIV gp41 HR2 region; Newcastle disease virus (NDV) HR1 and NVD HR2, human metapneumovirus HR1 and human metapneumovirus HR; and other class 1 fusion viral proteins that contain 6-helix bundles in their postfusion state.

15. The method of claim 13 or 14, wherein the RSVF protein ectodomain polypeptides and the oligomerization polypeptide are provided in a way selected from the group consisting of in a cell, in conditioned cell culture media, as expression constructs, and as purified components.


SEQUENCE LISTING

<110> NOVARTIS AG

<120> RSV F TRIMERIZATION DOMAINS

<130> PAT056288-EP-EPA

<140>
<141>

<160> 111

<170> PatentIn version 3.5

<210> 1
<211> 574
<212> PRT
<213> Respiratory syncytial virus

<400> 1
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Pro Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Ser Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asp Lys Gln Leu Leu Pro Ile Val Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Ile Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile

420                 425                 430

Lys Thr Phe Ser Asn Gly Cys Asp Tyr Val Ser Asn Lys Gly Met Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu His Asn Val Asn Ala Gly Lys Ser Thr Thr Asn Ile Met Ile Thr
        515                 520                 525

Thr Ile Ile Ile Val Ile Ile Val Ile Leu Leu Ser Leu Ile Ala Val
    530                 535                 540

Gly Leu Leu Leu Tyr Cys Lys Ala Arg Ser Thr Pro Val Thr Leu Ser
545                 550                 555                 560

Lys Asp Gln Leu Ser Gly Ile Asn Asn Ile Ala Phe Ser Asn
                565                 570

<210> 2
<211> 574
<212> PRT
<213> Respiratory syncytial virus

<400> 2
Met Glu Leu Leu Ile His Arg Ser Ser Ala Ile Phe Leu Thr Leu Ala
1               5                   10                  15

Val Asn Ala Leu Tyr Leu Thr Ser Ser Gln Asn Ile Thr Glu Glu Phe
            20                  25                  30

Tyr Gln Ser Thr Cys Ser Ala Val Ser Arg Gly Tyr Phe Ser Ala Leu
        35                  40                  45

Arg Thr Gly Trp Tyr Thr Ser Val Ile Thr Ile Glu Leu Ser Asn Ile
    50                  55                  60

Lys Glu Thr Lys Cys Asn Gly Thr Asp Thr Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Asn Thr Pro Ala Ala Asn Asn Arg Ala Arg Arg Glu Ala Pro
            100                 105                 110

Gln Tyr Met Asn Tyr Thr Ile Asn Thr Thr Lys Asn Leu Asn Val Ser
        115                 120                 125

Ile Ser Lys Lys Arg Lys Arg Arg Phe Leu Gly Phe Leu Leu Gly Val
    130                 135                 140

Gly Ser Ala Ile Ala Ser Gly Ile Ala Val Ser Lys Val Leu His Leu
145                 150                 155                 160

Glu Gly Glu Val Asn Lys Ile Lys Asn Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asn Asn Arg Leu Leu Pro Ile Val Asn
        195                 200                 205

Gln Gln Ser Cys Arg Ile Ser Asn Ile Glu Thr Val Ile Glu Phe Gln
    210                 215                 220

Gln Met Asn Ser Arg Leu Leu Glu Ile Thr Arg Glu Phe Ser Val Asn
225                 230                 235                 240

Ala Gly Val Thr Thr Pro Leu Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Ser Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Ile Tyr Gly Val Ile Asp Thr Pro Cys Trp Lys Leu His Thr Ser Pro
305                 310                 315                 320

Leu Cys Thr Thr Asn Ile Lys Glu Gly Ser Asn Ile Cys Leu Thr Arg

325                 330                 335

Thr Asp Arg Gly Trp Tyr Cys Asp Asn Ala Gly Ser Val Ser Phe Phe
            340                 345                 350

Pro Gln Ala Asp Thr Cys Lys Val Gln Ser Asn Arg Val Phe Cys Asp
        355                 360                 365

Thr Met Asn Ser Leu Thr Leu Pro Ser Glu Val Ser Leu Cys Asn Thr
    370                 375                 380

Asp Ile Phe Asn Ser Lys Tyr Asp Cys Lys Ile Met Thr Ser Lys Thr
385                 390                 395                 400

Asp Ile Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Leu Glu Gly
    450                 455                 460

Lys Asn Leu Tyr Val Lys Gly Glu Pro Ile Ile Asn Tyr Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Arg Ser Asp Glu Leu
            500                 505                 510

Leu His Asn Val Asn Thr Gly Lys Ser Thr Thr Asn Ile Met Ile Thr
        515                 520                 525

Thr Ile Ile Ile Val Ile Ile Val Val Leu Leu Ser Leu Ile Ala Ile
    530                 535                 540

Gly Leu Leu Leu Tyr Cys Lys Ala Lys Asn Thr Pro Val Thr Leu Ser
545                 550                 555                 560

Lys Asp Gln Leu Ser Gly Ile Asn Asn Ile Ala Phe Ser Lys
                565                 570

<210> 3
<211> 51
<212> PRT
<213> Respiratory syncytial virus

<400> 3
Thr Pro Ala Thr Asn Asn Arg Ala Arg Lys Glu Leu Pro Arg Phe Met
1               5                   10                  15

Asn Tyr Thr Leu Asn Asn Ala Lys Lys Thr Asn Val Thr Leu Ser Lys
            20                  25                  30

Lys Arg Lys Lys Lys Phe Leu Gly Phe Leu Leu Gly Val Gly Ser Ala
        35                  40                  45

Ile Ala Ser
    50

<210> 4
<211> 48
<212> PRT
<213> Respiratory syncytial virus

<400> 4
Thr Pro Ala Thr Asn Asn Arg Ala Arg Gln Glu Leu Pro Arg Phe Met
1               5                   10                  15

Asn Tyr Thr Leu Asn Asn Ala Lys Lys Thr Asn Val Thr Leu Ser Lys
            20                  25                  30

Lys Arg Phe Leu Gly Phe Leu Leu Gly Val Gly Ser Ala Ile Ala Ser
        35                  40                  45

<210> 5
<211> 51
<212> PRT
<213> Respiratory syncytial virus

<400> 5
Thr Pro Ala Thr Asn Asn Gln Ala Gln Asn Glu Leu Pro Arg Phe Met
1               5                   10                  15

Asn Tyr Thr Leu Asn Asn Ala Lys Lys Thr Asn Val Thr Leu Ser Gln
            20                  25                  30

Asn Gln Asn Gln Asn Phe Leu Gly Phe Leu Leu Gly Val Gly Ser Ala
        35                  40                  45

Ile Ala Ser
    50

<210> 6

<211> 51
<212> PRT
<213> Respiratory syncytial virus

<400> 6
Thr Pro Ala Thr Asn Asn Gln Ala Gln Asn Glu Leu Pro Gln Phe Met
1               5                   10                  15

Asn Tyr Thr Leu Asn Asn Ala Asn Asn Thr Asn Val Thr Leu Ser Gln
            20                  25                  30

Asn Gln Asn Gln Asn Phe Leu Gly Phe Leu Leu Gly Val Gly Ser Ala
        35                  40                  45

Ile Ala Ser
    50

<210> 7
<211> 51
<212> PRT
<213> Respiratory syncytial virus

<400> 7
Thr Pro Ala Thr Asn Asn Gln Ala Gln Asn Glu Leu Pro Gln Phe Met
1               5                   10                  15

Asn Tyr Thr Leu Asn Asn Ala Gln Gln Thr Asn Val Thr Leu Ser Gln
            20                  25                  30

Asn Gln Asn Gln Asn Phe Leu Gly Phe Leu Leu Gly Val Gly Ser Ala
        35                  40                  45

Ile Ala Ser
    50

<210> 8
<211> 30
<212> PRT
<213> Respiratory syncytial virus

<400> 8
Thr Pro Ala Thr Asn Asn Gln Ala Gln Asn Gln Asn Gln Asn Gln Asn
1               5                   10                  15

Phe Leu Gly Phe Leu Leu Gly Val Gly Ser Ala Ile Ala Ser
            20                  25                  30

<210> 9
<211> 28
<212> PRT
<213> Respiratory syncytial virus

<400> 9

Thr Pro Ala Thr Asn Asn Gln Ala Gln Asn Gln Asn Gln Asn Phe Leu
1               5                   10                  15

Gly Phe Leu Leu Gly Val Gly Ser Ala Ile Ala Ser
            20                  25

<210> 10
<211> 30
<212> PRT
<213> Respiratory syncytial virus

<400> 10
Thr Pro Ala Thr Asn Asn Arg Ala Arg Gln Gln Asn Gln Gln Gln Arg
1               5                   10                  15

Phe Leu Gly Phe Leu Leu Gly Val Gly Ser Ala Ile Ala Ser
            20                  25                  30

<210> 11
<211> 28
<212> PRT
<213> Respiratory syncytial virus

<400> 11
Thr Pro Ala Thr Asn Asn Arg Ala Arg Gln Gln Gln Gln Arg Phe Leu
1               5                   10                  15

Gly Phe Leu Leu Gly Val Gly Ser Ala Ile Ala Ser
            20                  25

<210> 12
<211> 51
<212> PRT
<213> Respiratory syncytial virus

<400> 12
Thr Pro Ala Thr Asn Asn Arg Ala Arg Arg Glu Leu Pro Gln Phe Met
1               5                   10                  15

Asn Tyr Thr Leu Asn Asn Ala Gln Gln Thr Asn Val Thr Leu Ser Gln
            20                  25                  30

Asn Gln Asn Gln Asn Phe Leu Gly Phe Leu Leu Gly Val Gly Ser Ala
        35                  40                  45

Ile Ala Ser
    50

<210> 13
<211> 51
<212> PRT
<213> Respiratory syncytial virus

<400> 13
Thr Pro Ala Thr Asn Asn Gln Ala Gln Asn Glu Leu Pro Gln Phe Met
1               5                   10                  15

Asn Tyr Thr Leu Asn Asn Ala Gln Gln Thr Asn Val Thr Leu Ser Lys
            20                  25                  30

Lys Arg Lys Arg Arg Phe Leu Gly Phe Leu Leu Gly Val Gly Ser Ala
        35                  40                  45

Ile Ala Ser
    50

<210> 14
<211> 51
<212> PRT
<213> Respiratory syncytial virus

<400> 14
Thr Pro Ala Thr Asn Asn Ile Glu Gly Arg Glu Leu Pro Arg Phe Met
1               5                   10                  15

Asn Tyr Thr Leu Asn Asn Ala Lys Lys Thr Asn Val Thr Leu Ser Lys
            20                  25                  30

Lys Ile Glu Gly Arg Phe Leu Gly Phe Leu Leu Gly Val Gly Ser Ala
        35                  40                  45

Ile Ala Ser
    50

<210> 15
<211> 41
<212> PRT
<213> Respiratory syncytial virus

<400> 15
Thr Pro Pro Thr Asn Asn Arg Ala Arg Arg Glu Leu Pro Arg Phe Met
1               5                   10                  15

Asn Tyr Thr Leu Asn Asn Ala Lys Lys Thr Asn Val Thr Leu Ser Lys
            20                  25                  30

Lys Arg Lys Arg Arg Ala Ile Ala Ser
        35                  40

<210> 16
<211> 300
<212> PRT
<213> Artificial Sequence

<220>

<221> source
<223> /note="Description of Artificial Sequence: Synthetic
      polypeptide"

<400> 16
Met His His His His His His Gly Ser Met Ser Pro Ile Leu Gly Tyr
1               5                   10                  15

Trp Lys Ile Lys Gly Leu Val Gln Pro Thr Arg Leu Leu Leu Glu Tyr
            20                  25                  30

Leu Glu Glu Lys Tyr Glu Glu His Leu Tyr Glu Arg Asp Glu Gly Asp
        35                  40                  45

Lys Trp Arg Asn Lys Lys Phe Glu Leu Gly Leu Glu Phe Pro Asn Leu
    50                  55                  60

Pro Tyr Tyr Ile Asp Gly Asp Val Lys Leu Thr Gln Ser Met Ala Ile
65                  70                  75                  80

Ile Arg Tyr Ile Ala Asp Lys His Asn Met Leu Gly Gly Cys Pro Lys
                85                  90                  95

Glu Arg Ala Glu Ile Ser Met Leu Glu Gly Ala Val Leu Asp Ile Arg
            100                 105                 110

Tyr Gly Val Ser Arg Ile Ala Tyr Ser Lys Asp Phe Glu Thr Leu Lys
        115                 120                 125

Val Asp Phe Leu Ser Lys Leu Pro Glu Met Leu Lys Met Phe Glu Asp
    130                 135                 140

Arg Leu Cys His Lys Thr Tyr Leu Asn Gly Asp His Val Thr His Pro
145                 150                 155                 160

Asp Phe Met Leu Tyr Asp Ala Leu Asp Val Val Leu Tyr Met Asp Pro
                165                 170                 175

Met Cys Leu Asp Ala Phe Pro Lys Leu Val Cys Phe Lys Lys Arg Ile
            180                 185                 190

Glu Ala Ile Pro Gln Ile Asp Lys Tyr Leu Lys Ser Ser Lys Tyr Ile
        195                 200                 205

Ala Trp Pro Leu Gln Gly Trp Gln Ala Thr Phe Gly Gly Gly Asp His
    210                 215                 220

Pro Pro Lys Ser Asp Leu Val Pro Arg Gly Ser Gly Ser Leu Glu Val
225                 230                 235                 240

Leu Phe Gln Gly Pro Gly Gly Ser Ala Gly Ser Gly Leu Glu Gly Glu
                245                 250                 255

Val Asn Lys Ile Lys Ser Ala Leu Leu Ser Thr Asn Lys Ala Val Val
            260                 265                 270

Ser Leu Ser Asn Gly Val Ser Val Leu Thr Ser Lys Val Leu Asp Leu
        275                 280                 285

Lys Asn Tyr Ile Asp Lys Gln Leu Leu Pro Ile Val
    290                 295                 300

<210> 17
<211> 290
<212> PRT
<213> Artificial Sequence

<220>
<221> source
<223> /note="Description of Artificial Sequence: Synthetic
      polypeptide"

<400> 17
Met His His His His His His Gly Ser Met Ser Pro Ile Leu Gly Tyr
1               5                   10                  15

Trp Lys Ile Lys Gly Leu Val Gln Pro Thr Arg Leu Leu Leu Glu Tyr
            20                  25                  30

Leu Glu Glu Lys Tyr Glu Glu His Leu Tyr Glu Arg Asp Glu Gly Asp
        35                  40                  45

Lys Trp Arg Asn Lys Lys Phe Glu Leu Gly Leu Glu Phe Pro Asn Leu
    50                  55                  60

Pro Tyr Tyr Ile Asp Gly Asp Val Lys Leu Thr Gln Ser Met Ala Ile
65                  70                  75                  80

Ile Arg Tyr Ile Ala Asp Lys His Asn Met Leu Gly Gly Cys Pro Lys
                85                  90                  95

Glu Arg Ala Glu Ile Ser Met Leu Glu Gly Ala Val Leu Asp Ile Arg
            100                 105                 110

Tyr Gly Val Ser Arg Ile Ala Tyr Ser Lys Asp Phe Glu Thr Leu Lys
        115                 120                 125

Val Asp Phe Leu Ser Lys Leu Pro Glu Met Leu Lys Met Phe Glu Asp
    130                 135                 140

Arg Leu Cys His Lys Thr Tyr Leu Asn Gly Asp His Val Thr His Pro
145                 150                 155                 160

Asp Phe Met Leu Tyr Asp Ala Leu Asp Val Val Leu Tyr Met Asp Pro
                165                 170                 175

Met Cys Leu Asp Ala Phe Pro Lys Leu Val Cys Phe Lys Lys Arg Ile
            180                 185                 190

Glu Ala Ile Pro Gln Ile Asp Lys Tyr Leu Lys Ser Ser Lys Tyr Ile
        195                 200                 205

Ala Trp Pro Leu Gln Gly Trp Gln Ala Thr Phe Gly Gly Gly Asp His
    210                 215                 220

Pro Pro Lys Ser Asp Leu Val Pro Arg Gly Ser Gly Ser Leu Glu Val
225                 230                 235                 240

Leu Phe Gln Gly Pro Gly Gly Ser Ala Gly Ser Gly Leu Glu Gly Glu
                245                 250                 255

Val Asn Lys Ile Lys Ser Ala Leu Leu Ser Thr Asn Lys Ala Val Val
            260                 265                 270

Ser Leu Ser Asn Gly Val Ser Val Leu Thr Ser Lys Val Leu Asp Leu
        275                 280                 285

Lys Asn
    290

<210> 18
<211> 337
<212> PRT
<213> Artificial Sequence

<220>
<221> source
<223> /note="Description of Artificial Sequence: Synthetic
      polypeptide"

<400> 18
Met His His His His His His Gly Ser Met Ser Pro Ile Leu Gly Tyr
1               5                   10                  15

Trp Lys Ile Lys Gly Leu Val Gln Pro Thr Arg Leu Leu Leu Glu Tyr
            20                  25                  30

Leu Glu Glu Lys Tyr Glu Glu His Leu Tyr Glu Arg Asp Glu Gly Asp
        35                  40                  45

Lys Trp Arg Asn Lys Lys Phe Glu Leu Gly Leu Glu Phe Pro Asn Leu
    50                  55                  60

Pro Tyr Tyr Ile Asp Gly Asp Val Lys Leu Thr Gln Ser Met Ala Ile
65                  70                  75                  80

Ile Arg Tyr Ile Ala Asp Lys His Asn Met Leu Gly Gly Cys Pro Lys
                85                  90                  95

Glu Arg Ala Glu Ile Ser Met Leu Glu Gly Ala Val Leu Asp Ile Arg
            100                 105                 110

Tyr Gly Val Ser Arg Ile Ala Tyr Ser Lys Asp Phe Glu Thr Leu Lys
        115                 120                 125

Val Asp Phe Leu Ser Lys Leu Pro Glu Met Leu Lys Met Phe Glu Asp
    130                 135                 140

Arg Leu Cys His Lys Thr Tyr Leu Asn Gly Asp His Val Thr His Pro
145                 150                 155                 160

Asp Phe Met Leu Tyr Asp Ala Leu Asp Val Val Leu Tyr Met Asp Pro
                165                 170                 175

Met Cys Leu Asp Ala Phe Pro Lys Leu Val Cys Phe Lys Lys Arg Ile
            180                 185                 190

Glu Ala Ile Pro Gln Ile Asp Lys Tyr Leu Lys Ser Ser Lys Tyr Ile
        195                 200                 205

Ala Trp Pro Leu Gln Gly Trp Gln Ala Thr Phe Gly Gly Gly Asp His
    210                 215                 220

Pro Pro Lys Ser Asp Leu Val Pro Arg Gly Ser Gly Ser Leu Glu Val
225                 230                 235                 240

Leu Phe Gln Gly Pro Gly Gly Ser Ala Gly Ser Gly Arg Leu Lys Asn
                245                 250                 255

Pro Pro Lys Lys Pro Lys Asp Asp Tyr His Phe Glu Val Phe Asn Phe
            260                 265                 270

Val Pro Cys Ser Ile Cys Gly Asn Asn Gln Leu Cys Lys Ser Ile Cys
        275                 280                 285

Lys Thr Ile Pro Gly Gly Ser Ala Gly Ser Gly Leu Glu Gly Glu Val

290                 295                 300

Asn Lys Ile Lys Ser Ala Leu Leu Ser Thr Asn Lys Ala Val Val Ser
305                 310                 315                 320

Leu Ser Asn Gly Val Ser Val Leu Thr Ser Lys Val Leu Asp Leu Lys
                325                 330                 335

Asn

<210> 19
<211> 337
<212> PRT
<213> Artificial Sequence

<220>
<221> source
<223> /note="Description of Artificial Sequence: Synthetic
      polypeptide"

<400> 19
Met His His His His His His Gly Ser Met Ser Pro Ile Leu Gly Tyr
1               5                   10                  15

Trp Lys Ile Lys Gly Leu Val Gln Pro Thr Arg Leu Leu Leu Glu Tyr
            20                  25                  30

Leu Glu Glu Lys Tyr Glu Glu His Leu Tyr Glu Arg Asp Glu Gly Asp
        35                  40                  45

Lys Trp Arg Asn Lys Lys Phe Glu Leu Gly Leu Glu Phe Pro Asn Leu
    50                  55                  60

Pro Tyr Tyr Ile Asp Gly Asp Val Lys Leu Thr Gln Ser Met Ala Ile
65                  70                  75                  80

Ile Arg Tyr Ile Ala Asp Lys His Asn Met Leu Gly Gly Cys Pro Lys
                85                  90                  95

Glu Arg Ala Glu Ile Ser Met Leu Glu Gly Ala Val Leu Asp Ile Arg
            100                 105                 110

Tyr Gly Val Ser Arg Ile Ala Tyr Ser Lys Asp Phe Glu Thr Leu Lys
        115                 120                 125

Val Asp Phe Leu Ser Lys Leu Pro Glu Met Leu Lys Met Phe Glu Asp
    130                 135                 140

Arg Leu Cys His Lys Thr Tyr Leu Asn Gly Asp His Val Thr His Pro

145                 150                 155                 160

Asp Phe Met Leu Tyr Asp Ala Leu Asp Val Val Leu Tyr Met Asp Pro
                165                 170                 175

Met Cys Leu Asp Ala Phe Pro Lys Leu Val Cys Phe Lys Lys Arg Ile
            180                 185                 190

Glu Ala Ile Pro Gln Ile Asp Lys Tyr Leu Lys Ser Ser Lys Tyr Ile
        195                 200                 205

Ala Trp Pro Leu Gln Gly Trp Gln Ala Thr Phe Gly Gly Gly Asp His
    210                 215                 220

Pro Pro Lys Ser Asp Leu Val Pro Arg Gly Ser Gly Ser Leu Glu Val
225                 230                 235                 240

Leu Phe Gln Gly Pro Gly Gly Ser Ala Gly Ser Gly Arg Gln Asn Lys
                245                 250                 255

Pro Pro Ser Lys Pro Asn Asn Asp Phe His Phe Glu Val Phe Asn Phe
            260                 265                 270

Val Pro Cys Ser Ile Cys Ser Asn Asn Pro Thr Cys Trp Ala Ile Cys
        275                 280                 285

Lys Arg Ile Pro Gly Gly Ser Ala Gly Ser Gly Leu Glu Gly Glu Val
    290                 295                 300

Asn Lys Ile Lys Ser Ala Leu Leu Ser Thr Asn Lys Ala Val Val Ser
305                 310                 315                 320

Leu Ser Asn Gly Val Ser Val Leu Thr Ser Lys Val Leu Asp Leu Lys
                325                 330                 335

Asn

<210> 20
<211> 333
<212> PRT
<213> Artificial Sequence

<220>
<221> source
<223> /note="Description of Artificial Sequence: Synthetic
      polypeptide"

<400> 20
Met His His His His His His Gly Ser Met Ser Pro Ile Leu Gly Tyr

1               5                   10                  15

Trp Lys Ile Lys Gly Leu Val Gln Pro Thr Arg Leu Leu Leu Glu Tyr
            20                  25                  30

Leu Glu Glu Lys Tyr Glu Glu His Leu Tyr Glu Arg Asp Glu Gly Asp
        35                  40                  45

Lys Trp Arg Asn Lys Lys Phe Glu Leu Gly Leu Glu Phe Pro Asn Leu
    50                  55                  60

Pro Tyr Tyr Ile Asp Gly Asp Val Lys Leu Thr Gln Ser Met Ala Ile
65                  70                  75                  80

Ile Arg Tyr Ile Ala Asp Lys His Asn Met Leu Gly Gly Cys Pro Lys
                85                  90                  95

Glu Arg Ala Glu Ile Ser Met Leu Glu Gly Ala Val Leu Asp Ile Arg
            100                 105                 110

Tyr Gly Val Ser Arg Ile Ala Tyr Ser Lys Asp Phe Glu Thr Leu Lys
        115                 120                 125

Val Asp Phe Leu Ser Lys Leu Pro Glu Met Leu Lys Met Phe Glu Asp
    130                 135                 140

Arg Leu Cys His Lys Thr Tyr Leu Asn Gly Asp His Val Thr His Pro
145                 150                 155                 160

Asp Phe Met Leu Tyr Asp Ala Leu Asp Val Val Leu Tyr Met Asp Pro
                165                 170                 175

Met Cys Leu Asp Ala Phe Pro Lys Leu Val Cys Phe Lys Lys Arg Ile
            180                 185                 190

Glu Ala Ile Pro Gln Ile Asp Lys Tyr Leu Lys Ser Ser Lys Tyr Ile
        195                 200                 205

Ala Trp Pro Leu Gln Gly Trp Gln Ala Thr Phe Gly Gly Gly Asp His
    210                 215                 220

Pro Pro Lys Ser Asp Leu Val Pro Arg Gly Ser Gly Ser Leu Glu Val
225                 230                 235                 240

Leu Phe Gln Gly Pro Gly Gly Ser Ala Gly Ser Gly Arg Leu Lys Asn
                245                 250                 255

Pro Pro Lys Lys Pro Lys Asp Asp Tyr His Phe Glu Val Phe Asn Phe
            260                 265                 270

Val Pro Cys Ser Ile Cys Gly Asn Asn Gln Leu Cys Lys Ser Ile Cys
        275                 280                 285

Lys Thr Ile Pro Gly Gly Ser Ala Gly Ser Gly Pro Ser Asp Glu Phe
    290                 295                 300

Asp Ala Ser Ile Ser Gln Val Asn Glu Lys Ile Asn Gln Ser Leu Ala
305                 310                 315                 320

Phe Ile Arg Lys Ser Asp Glu Leu Leu His Asn Val Asn
                325                 330

<210> 21
<211> 333
<212> PRT
<213> Artificial Sequence

<220>
<221> source
<223> /note="Description of Artificial Sequence: Synthetic
      polypeptide"

<400> 21
Met His His His His His His Gly Ser Met Ser Pro Ile Leu Gly Tyr
1               5                   10                  15

Trp Lys Ile Lys Gly Leu Val Gln Pro Thr Arg Leu Leu Leu Glu Tyr
            20                  25                  30

Leu Glu Glu Lys Tyr Glu Glu His Leu Tyr Glu Arg Asp Glu Gly Asp
        35                  40                  45

Lys Trp Arg Asn Lys Lys Phe Glu Leu Gly Leu Glu Phe Pro Asn Leu
    50                  55                  60

Pro Tyr Tyr Ile Asp Gly Asp Val Lys Leu Thr Gln Ser Met Ala Ile
65                  70                  75                  80

Ile Arg Tyr Ile Ala Asp Lys His Asn Met Leu Gly Gly Cys Pro Lys
                85                  90                  95

Glu Arg Ala Glu Ile Ser Met Leu Glu Gly Ala Val Leu Asp Ile Arg
            100                 105                 110

Tyr Gly Val Ser Arg Ile Ala Tyr Ser Lys Asp Phe Glu Thr Leu Lys
        115                 120                 125

Val Asp Phe Leu Ser Lys Leu Pro Glu Met Leu Lys Met Phe Glu Asp
    130                 135                 140

Arg Leu Cys His Lys Thr Tyr Leu Asn Gly Asp His Val Thr His Pro
145                 150                 155                 160

Asp Phe Met Leu Tyr Asp Ala Leu Asp Val Val Leu Tyr Met Asp Pro
                165                 170                 175

Met Cys Leu Asp Ala Phe Pro Lys Leu Val Cys Phe Lys Lys Arg Ile
            180                 185                 190

Glu Ala Ile Pro Gln Ile Asp Lys Tyr Leu Lys Ser Ser Lys Tyr Ile
        195                 200                 205

Ala Trp Pro Leu Gln Gly Trp Gln Ala Thr Phe Gly Gly Gly Asp His
    210                 215                 220

Pro Pro Lys Ser Asp Leu Val Pro Arg Gly Ser Gly Ser Leu Glu Val
225                 230                 235                 240

Leu Phe Gln Gly Pro Gly Gly Ser Ala Gly Ser Gly Arg Gln Asn Lys
                245                 250                 255

Pro Pro Ser Lys Pro Asn Asn Asp Phe His Phe Glu Val Phe Asn Phe
            260                 265                 270

Val Pro Cys Ser Ile Cys Ser Asn Asn Pro Thr Cys Trp Ala Ile Cys
        275                 280                 285

Lys Arg Ile Pro Gly Gly Ser Ala Gly Ser Gly Pro Ser Asp Glu Phe
    290                 295                 300

Asp Ala Ser Ile Ser Gln Val Asn Glu Lys Ile Asn Gln Ser Leu Ala
305                 310                 315                 320

Phe Ile Arg Lys Ser Asp Glu Leu Leu His Asn Val Asn
                325                 330

<210> 22
<211> 543
<212> PRT
<213> Artificial Sequence

<220>
<221> source
<223> /note="Description of Artificial Sequence: Synthetic
      polypeptide"

<400> 22

Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Gln Gln Gln Gln
            100                 105                 110

Arg Phe Leu Gly Phe Leu Leu Gly Val Gly Ser Ala Ile Ala Ser Gly
        115                 120                 125

Val Ala Val Ser Lys Val Leu His Leu Glu Gly Glu Val Asn Lys Ile
    130                 135                 140

Lys Ser Ala Leu Leu Ser Thr Asn Lys Ala Val Val Ser Leu Ser Asn
145                 150                 155                 160

Gly Val Ser Val Leu Thr Ser Lys Val Leu Asp Leu Lys Asn Tyr Ile
                165                 170                 175

Asp Lys Gln Leu Leu Pro Ile Val Asn Lys Gln Ser Cys Ser Ile Ser
            180                 185                 190

Asn Ile Glu Thr Val Ile Glu Phe Gln Gln Lys Asn Asn Arg Leu Leu
        195                 200                 205

Glu Ile Thr Arg Glu Phe Ser Val Asn Ala Gly Val Thr Thr Pro Val
    210                 215                 220

Ser Thr Tyr Met Leu Thr Asn Ser Glu Leu Leu Ser Leu Ile Asn Asp
225                 230                 235                 240

Met Pro Ile Thr Asn Asp Gln Lys Lys Leu Met Ser Asn Asn Val Gln
                245                 250                 255

Ile Val Arg Gln Gln Ser Tyr Ser Ile Met Ser Ile Ile Lys Glu Glu
            260                 265                 270

Val Leu Ala Tyr Val Val Gln Leu Pro Leu Tyr Gly Val Ile Asp Thr
        275                 280                 285

Pro Cys Trp Lys Leu His Thr Ser Pro Leu Cys Thr Thr Asn Thr Lys
    290                 295                 300

Glu Gly Ser Asn Ile Cys Leu Thr Arg Thr Asp Arg Gly Trp Tyr Cys
305                 310                 315                 320

Asp Asn Ala Gly Ser Val Ser Phe Phe Pro Gln Ala Glu Thr Cys Lys
                325                 330                 335

Val Gln Ser Asn Arg Val Phe Cys Asp Thr Met Asn Ser Leu Thr Leu
            340                 345                 350

Pro Ser Glu Val Asn Leu Cys Asn Val Asp Ile Phe Asn Pro Lys Tyr
        355                 360                 365

Asp Cys Lys Ile Met Thr Ser Lys Thr Asp Val Ser Ser Ser Val Ile
    370                 375                 380

Thr Ser Leu Gly Ala Ile Val Ser Cys Tyr Gly Lys Thr Lys Cys Thr
385                 390                 395                 400

Ala Ser Asn Lys Asn Arg Gly Ile Ile Lys Thr Phe Ser Asn Gly Cys
                405                 410                 415

Asp Tyr Val Ser Asn Lys Gly Val Asp Thr Val Ser Val Gly Asn Thr
            420                 425                 430

Leu Tyr Tyr Val Asn Lys Gln Glu Gly Lys Ser Leu Tyr Val Lys Gly
        435                 440                 445

Glu Pro Ile Ile Asn Phe Tyr Asp Pro Leu Val Phe Pro Ser Asp Glu
    450                 455                 460

Phe Asp Ala Ser Ile Ser Gln Val Asn Glu Lys Ile Asn Gln Ser Leu
465                 470                 475                 480

Ala Phe Ile Arg Lys Ser Asp Glu Leu Leu His Asn Leu Glu Gly Glu
                485                 490                 495

Val Asn Lys Ile Lys Ser Ala Leu Leu Ser Thr Asn Lys Ala Val Val
            500                 505                 510

Ser Leu Ser Asn Gly Val Ser Val Leu Thr Ser Lys Val Leu Asp Leu
        515                 520                 525

Lys Asn Gly Gly Ser Ala Gly Ser Gly His His His His His His
    530                 535                 540

<210> 23
<211> 543
<212> PRT
<213> Artificial Sequence

<220>
<221> source
<223> /note="Description of Artificial Sequence: Synthetic
      polypeptide"

<400> 23
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Gln Gln Gln Gln
            100                 105                 110

Arg Phe Leu Gly Phe Leu Leu Gly Val Gly Ser Ala Ile Ala Ser Gly
        115                 120                 125

Val Ala Val Ser Lys Val Leu His Leu Glu Gly Glu Val Asn Lys Ile
    130                 135                 140

Lys Ser Ala Leu Leu Ser Thr Asn Lys Ala Val Val Ser Leu Ser Asn
145                 150                 155                 160

Gly Val Ser Val Leu Thr Ser Lys Val Leu Asp Leu Lys Asn Tyr Ile
                165                 170                 175

Asp Lys Gln Leu Leu Pro Ile Val Asn Lys Gln Ser Cys Ser Ile Ser
            180                 185                 190

Asn Ile Glu Thr Val Ile Glu Phe Gln Gln Lys Asn Asn Arg Leu Leu
        195                 200                 205

Glu Ile Thr Arg Glu Phe Ser Val Asn Ala Gly Val Thr Thr Pro Val
    210                 215                 220

Ser Thr Tyr Met Leu Thr Asn Ser Glu Leu Leu Ser Leu Ile Asn Asp
225                 230                 235                 240

Met Pro Ile Thr Asn Asp Gln Lys Lys Leu Met Ser Asn Asn Val Gln
                245                 250                 255

Ile Val Arg Gln Gln Ser Tyr Ser Ile Met Ser Ile Ile Lys Glu Glu
            260                 265                 270

Val Leu Ala Tyr Val Val Gln Leu Pro Leu Tyr Gly Val Ile Asp Thr
        275                 280                 285

Pro Cys Trp Lys Leu His Thr Ser Pro Leu Cys Thr Thr Asn Thr Lys
    290                 295                 300

Glu Gly Ser Asn Ile Cys Leu Thr Arg Thr Asp Arg Gly Trp Tyr Cys
305                 310                 315                 320

Asp Asn Ala Gly Ser Val Ser Phe Phe Pro Gln Ala Glu Thr Cys Lys
                325                 330                 335

Val Gln Ser Asn Arg Val Phe Cys Asp Thr Met Asn Ser Leu Thr Leu
            340                 345                 350

Pro Ser Glu Val Asn Leu Cys Asn Val Asp Ile Phe Asn Pro Lys Tyr
        355                 360                 365

Asp Cys Lys Ile Met Thr Ser Lys Thr Asp Val Ser Ser Ser Val Ile
    370                 375                 380

Thr Ser Leu Gly Ala Ile Val Ser Cys Tyr Gly Lys Thr Lys Cys Thr
385                 390                 395                 400

Ala Ser Asn Lys Asn Arg Gly Ile Ile Lys Thr Phe Ser Asn Gly Cys
                405                 410                 415

Asp Tyr Val Ser Asn Lys Gly Val Asp Thr Val Ser Val Gly Asn Thr

420                 425                 430

Leu Tyr Tyr Val Asn Lys Gln Glu Gly Lys Ser Leu Tyr Val Lys Gly
        435                 440                 445

Glu Pro Ile Ile Asn Phe Tyr Asp Pro Leu Cys Phe Pro Ser Asp Glu
    450                 455                 460

Phe Cys Ala Ser Ile Ser Gln Val Asn Glu Lys Ile Asn Gln Ser Leu
465                 470                 475                 480

Ala Phe Ile Arg Lys Cys Cys Glu Leu Leu His Asn Leu Glu Gly Glu
                485                 490                 495

Val Asn Lys Ile Lys Ser Ala Leu Leu Ser Thr Asn Lys Ala Val Val
            500                 505                 510

Ser Leu Ser Asn Gly Val Ser Val Leu Thr Ser Lys Val Leu Asp Leu
        515                 520                 525

Lys Asn Gly Gly Ser Ala Gly Ser Gly His His His His His His
    530                 535                 540

<210> 24
<211> 574
<212> PRT
<213> Artificial Sequence

<220>
<221> source
<223> /note="Description of Artificial Sequence: Synthetic
      consensus polypeptide"

<220>
<221> VARIANT
<222> (114)..(114)
<223> /replace="Phe"

<220>
<221> VARIANT
<222> (152)..(152)
<223> /replace="Val"

<220>
<221> VARIANT
<222> (200)..(200)
<223> /replace="Asp"

<220>
<221> VARIANT
<222> (356)..(356)
<223> /replace="Glu"

<220>
<221> VARIANT

<222> (402)..(402)
<223> /replace="Val"

<220>
<221> VARIANT
<222> (477)..(477)
<223> /replace="Phe"

<220>
<221> VARIANT
<222> (537)..(537)
<223> /replace="Ile"

<220>
<221> VARIANT
<222> (544)..(544)
<223> /replace="Val"

<220>
<221> MISC_FEATURE
<222> (1)..(574)
<223> /note="Variant residues given in the sequence have no
      preference with respect to those in the annotations for
      variant positions"

<400> 24
Met Glu Leu Leu Ile Leu Lys Ala Asn Ala Ile Thr Thr Ile Leu Ala
1               5                   10                  15

Ala Val Thr Phe Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Ala Asn Asn Arg Ala Arg Arg Glu Leu Pro
            100                 105                 110

Arg Tyr Met Asn Tyr Thr Leu Asn Asn Thr Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Ile Ala Val Ser Lys Val Leu His Leu

145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asn Lys Gln Leu Leu Pro Ile Val Asn
        195                 200                 205

Lys Gln Ser Cys Arg Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Asp Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Ile Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Cys Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Tyr Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu His Asn Val Asn Ala Gly Lys Ser Thr Thr Asn Ile Met Ile Thr
        515                 520                 525

Thr Ile Ile Ile Val Ile Ile Val Val Leu Leu Ser Leu Ile Ala Ile
    530                 535                 540

Gly Leu Leu Leu Tyr Cys Lys Ala Arg Ser Thr Pro Val Thr Leu Ser
545                 550                 555                 560

Lys Asp Gln Leu Ser Gly Ile Asn Asn Ile Ala Phe Ser Asn
                565                 570

<210> 25
<211> 51
<212> PRT
<213> Respiratory syncytial virus

<400> 25
Thr Pro Ala Thr Asn Asn Arg Ala Arg Arg Glu Leu Pro Arg Phe Met
1               5                   10                  15

Asn Tyr Thr Leu Asn Asn Ala Lys Lys Thr Asn Val Thr Leu Ser Lys
            20                  25                  30

Lys Arg Lys Arg Arg Phe Leu Gly Phe Leu Leu Gly Val Gly Ser Ala
        35                  40                  45

Ile Ala Ser

    50

<210> 26
<211> 42
<212> PRT
<213> Respiratory syncytial virus

<400> 26
Thr Pro Ala Thr Asn Asn Arg Ala Arg Arg Glu Leu Pro Arg Phe Met
1               5                   10                  15

Asn Tyr Thr Leu Asn Asn Ala Lys Lys Thr Asn Val Thr Leu Ser Lys
            20                  25                  30

Lys Arg Lys Arg Arg Ser Ala Ile Ala Ser
        35                  40

<210> 27
<211> 574
<212> PRT
<213> Respiratory syncytial virus

<400> 27
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Ser Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asp Lys Gln Leu Leu Pro Ile Val Asn
        195                 200                 205

Lys Gln Ser Cys Ser Ile Ser Asn Ile Ala Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu His Asn Val Asn Ala Gly Lys Ser Thr Ile Asn Ile Met Ile Thr
        515                 520                 525

Thr Ile Ile Ile Val Ile Ile Val Ile Leu Leu Ser Leu Ile Ala Val
    530                 535                 540

Gly Leu Leu Leu Tyr Cys Lys Ala Arg Ser Thr Pro Val Thr Leu Ser
545                 550                 555                 560

Lys Asp Gln Leu Ser Gly Ile Asn Asn Ile Ala Phe Ser Asn
                565                 570

<210> 28
<211> 574
<212> PRT
<213> Respiratory syncytial virus

<400> 28
Met Glu Leu Leu Ile His Arg Leu Ser Ala Ile Phe Leu Thr Leu Ala
1               5                   10                  15

Ile Asn Ala Leu Tyr Leu Thr Ser Ser Gln Asn Ile Thr Glu Glu Phe
            20                  25                  30

Tyr Gln Ser Thr Cys Ser Ala Val Ser Arg Gly Tyr Phe Ser Ala Leu
        35                  40                  45

Arg Thr Gly Trp Tyr Thr Ser Val Ile Thr Ile Glu Leu Ser Asn Ile
    50                  55                  60

Lys Glu Thr Lys Cys Asn Gly Thr Asp Thr Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Asn Thr Pro Ala Ala Asn Asn Arg Ala Arg Arg Glu Ala Pro
            100                 105                 110

Gln Tyr Met Asn Tyr Thr Ile Asn Thr Thr Lys Asn Leu Asn Val Ser
        115                 120                 125

Ile Ser Lys Lys Arg Lys Arg Arg Phe Leu Gly Phe Leu Leu Gly Val
    130                 135                 140

Gly Ser Ala Ile Ala Ser Gly Ile Ala Val Ser Lys Val Leu His Leu
145                 150                 155                 160

Glu Gly Glu Val Asn Lys Ile Lys Asn Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asn Asn Gln Leu Leu Pro Ile Val Asn
        195                 200                 205

Gln Gln Ser Cys Arg Ile Ser Asn Ile Gly Thr Val Ile Glu Phe Gln
    210                 215                 220

Gln Lys Asn Ser Arg Leu Leu Glu Ile Asn Arg Glu Phe Ser Val Asn
225                 230                 235                 240

Ala Gly Val Thr Thr Pro Leu Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Ser Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Ile Tyr Gly Val Ile Asp Thr Pro Cys Trp Lys Leu His Thr Ser Pro
305                 310                 315                 320

Leu Cys Thr Thr Asn Ile Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Asp Thr Cys Lys Val Gln Ser Asn Arg Val Phe Cys Asp
        355                 360                 365

Thr Met Asn Ser Leu Thr Leu Pro Ser Glu Val Ser Leu Cys Asn Thr
    370                 375                 380

Asp Ile Phe Asn Ser Lys Tyr Asp Cys Lys Ile Met Thr Ser Lys Thr
385                 390                 395                 400

Asp Ile Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Leu Glu Gly
    450                 455                 460

Lys Asn Leu Tyr Val Lys Gly Glu Pro Ile Ile Asn Tyr Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Arg Ser Asp Glu Leu
            500                 505                 510

Leu His Asn Val Asn Thr Gly Lys Ser Thr Thr Asn Ile Met Ile Thr
        515                 520                 525

Thr Ile Ile Ile Val Ile Ile Val Val Leu Leu Ser Leu Ile Ala Ile
    530                 535                 540

Gly Leu Leu Leu Tyr Cys Lys Ala Lys Asn Thr Pro Val Thr Leu Ser
545                 550                 555                 560

Lys Asp Gln Leu Ser Gly Ile Asn Asn Ile Ala Phe Ser Lys
                565                 570

<210> 29
<211> 574
<212> PRT
<213> Respiratory syncytial virus

<400> 29
Met Glu Leu Leu Ile His Arg Leu Ser Ala Ile Phe Leu Thr Leu Ala
1               5                   10                  15

Ile Asn Ala Leu Tyr Leu Thr Ser Ser Gln Asn Ile Thr Glu Glu Phe
            20                  25                  30

Tyr Gln Ser Thr Cys Ser Ala Val Ser Arg Gly Tyr Phe Ser Ala Leu
        35                  40                  45

Arg Thr Gly Trp Tyr Thr Ser Val Ile Thr Ile Glu Leu Ser Asn Ile
    50                  55                  60

Lys Glu Thr Lys Cys Asn Gly Thr Asp Thr Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Asn Thr Pro Ala Ala Asn Asn Arg Ala Arg Arg Glu Ala Pro
            100                 105                 110

Gln Tyr Met Asn Tyr Thr Ile Asn Thr Thr Lys Asn Leu Asn Val Ser
        115                 120                 125

Ile Ser Lys Lys Arg Lys Arg Arg Phe Leu Gly Phe Leu Leu Gly Val
    130                 135                 140

Gly Ser Ala Ile Ala Ser Gly Ile Ala Val Ser Lys Val Leu His Leu
145                 150                 155                 160

Glu Gly Glu Val Asn Lys Ile Lys Asn Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asn Asn Gln Leu Leu Pro Ile Val Asn
        195                 200                 205

Gln Gln Ser Cys Arg Ile Ser Asn Ile Glu Thr Val Ile Glu Phe Gln
    210                 215                 220

Gln Lys Asn Ser Arg Leu Leu Glu Ile Asn Arg Glu Phe Ser Val Asn
225                 230                 235                 240

Ala Gly Val Thr Thr Pro Leu Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Ser Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Ile Tyr Gly Val Ile Asp Thr Pro Cys Trp Lys Leu His Thr Ser Pro
305                 310                 315                 320

Leu Cys Thr Thr Asn Ile Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Asp Thr Cys Lys Val Gln Ser Asn Arg Val Phe Cys Asp
        355                 360                 365

Thr Met Asn Ser Leu Thr Leu Pro Ser Glu Val Ser Leu Cys Asn Thr
    370                 375                 380

Asp Ile Phe Asn Ser Lys Tyr Asp Cys Lys Ile Met Thr Ser Lys Thr
385                 390                 395                 400

Asp Ile Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Leu Glu Gly
    450                 455                 460

Lys Asn Leu Tyr Val Lys Gly Glu Pro Ile Ile Asn Tyr Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Arg Ser Asp Glu Leu
            500                 505                 510

Leu His Asn Val Asn Thr Gly Lys Ser Thr Thr Asn Ile Met Ile Thr
        515                 520                 525

Thr Ile Ile Ile Val Ile Ile Val Val Leu Leu Ser Leu Ile Ala Ile
    530                 535                 540

Gly Leu Leu Leu Tyr Cys Lys Ala Lys Asn Thr Pro Val Thr Leu Ser
545                 550                 555                 560

Lys Asp Gln Leu Ser Gly Ile Asn Asn Ile Ala Phe Ser Lys
                565                 570

<210> 30
<211> 574
<212> PRT
<213> Respiratory syncytial virus

<400> 30
Met Glu Leu Pro Ile Leu Lys Ala Asn Ala Ile Thr Thr Ile Leu Ala
1               5                   10                  15

Ala Val Thr Phe Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Asn
65                  70                  75                  80

Gln Glu Leu Asp Lys Tyr Lys Asn Ala Val Thr Glu Leu Gln Leu Leu
                85                  90                  95

Met Gln Ser Thr Thr Ala Ala Asn Asn Arg Ala Arg Arg Glu Leu Pro
            100                 105                 110

Arg Phe Met Asn Tyr Thr Leu Asn Asn Thr Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Ile Ala Val Ser Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asp Lys Gln Leu Leu Pro Ile Val Asn
        195                 200                 205

Lys Gln Ser Cys Arg Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu His His Val Asn Ala Gly Lys Ser Thr Thr Asn Ile Met Ile Thr
        515                 520                 525

Thr Ile Ile Ile Val Ile Ile Val Ile Leu Leu Ser Leu Ile Ala Val
    530                 535                 540

Gly Leu Leu Leu Tyr Cys Lys Ala Arg Ser Thr Pro Val Thr Leu Ser
545                 550                 555                 560

Lys Asp Gln Leu Ser Gly Ile Asn Asn Ile Ala Phe Ser Asn
                565                 570


<210> 31
<211> 574
<212> PRT
<213> Respiratory syncytial virus

<400> 31
Met Glu Leu Leu Ile His Arg Ser Ser Ala Ile Phe Leu Thr Leu Ala
1               5                   10                  15

Val Asn Ala Leu Tyr Leu Thr Ser Ser Gln Asn Ile Thr Glu Glu Phe
            20                  25                  30

Tyr Gln Ser Thr Cys Ser Ala Val Ser Arg Gly Tyr Phe Ser Ala Leu
        35                  40                  45

Arg Thr Gly Trp Tyr Thr Ser Val Ile Thr Ile Glu Leu Ser Asn Ile
    50                  55                  60

Lys Glu Thr Lys Cys Asn Gly Thr Asp Thr Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Asn Thr Pro Ala Ala Asn Asn Arg Ala Arg Arg Glu Ala Pro
            100                 105                 110

Gln Tyr Met Asn Tyr Thr Ile Asn Thr Thr Lys Asn Leu Asn Val Ser
        115                 120                 125

Ile Ser Lys Lys Arg Lys Arg Arg Phe Leu Gly Phe Leu Leu Gly Val
    130                 135                 140

Gly Ser Ala Ile Ala Ser Gly Ile Ala Val Ser Lys Val Leu His Leu
145                 150                 155                 160

Glu Gly Glu Val Asn Lys Ile Lys Asn Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asn Asn Arg Leu Leu Pro Ile Val Asn
        195                 200                 205

Gln Gln Ser Cys Arg Ile Ser Asn Ile Glu Thr Val Ile Glu Phe Gln
    210                 215                 220

Gln Met Asn Ser Arg Leu Leu Glu Ile Thr Arg Glu Phe Ser Val Asn
225                 230                 235                 240

Ala Gly Val Thr Thr Pro Leu Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Ser Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Ile Tyr Gly Val Ile Asp Thr Pro Cys Trp Lys Leu His Thr Ser Pro
305                 310                 315                 320

Leu Cys Thr Thr Asn Ile Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Asp Thr Cys Lys Val Gln Ser Asn Arg Val Phe Cys Asp
        355                 360                 365

Thr Met Asn Ser Leu Thr Leu Pro Ser Glu Val Ser Leu Cys Asn Thr
    370                 375                 380

Asp Ile Phe Asn Ser Lys Tyr Asp Cys Lys Ile Met Thr Ser Lys Thr
385                 390                 395                 400

Asp Ile Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Leu Glu Gly
    450                 455                 460

Lys Asn Leu Tyr Val Lys Gly Glu Pro Ile Ile Asn Tyr Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Arg Ser Asp Glu Leu
            500                 505                 510

Leu His Asn Val Asn Thr Gly Lys Ser Thr Thr Asn Ile Met Ile Thr

515                 520                 525

Thr Ile Ile Ile Val Ile Ile Val Val Leu Leu Ser Leu Ile Ala Ile
    530                 535                 540

Gly Leu Leu Leu Tyr Cys Lys Ala Lys Asn Thr Pro Val Thr Leu Ser
545                 550                 555                 560

Lys Asp Gln Leu Ser Gly Ile Asn Asn Ile Ala Phe Ser Lys
                565                 570

<210> 32
<211> 4
<212> PRT
<213> Respiratory syncytial virus

<400> 32
Arg Ala Arg Lys
1

<210> 33
<211> 4
<212> PRT
<213> Respiratory syncytial virus

<400> 33
Arg Ala Arg Gln
1

<210> 34
<211> 4
<212> PRT
<213> Respiratory syncytial virus

<400> 34
Gln Ala Gln Asn
1

<210> 35
<211> 4
<212> PRT
<213> Respiratory syncytial virus

<400> 35
Ile Glu Gly Arg
1

<210> 36
<211> 4
<212> PRT
<213> Respiratory syncytial virus

<400> 36
Arg Lys Lys Lys
1

<210> 37
<211> 4
<212> PRT
<213> Respiratory syncytial virus

<400> 37
Gln Asn Gln Asn
1

<210> 38
<211> 4
<212> PRT
<213> Respiratory syncytial virus

<400> 38
Gln Gln Gln Arg
1

<210> 39
<211> 6
<212> PRT
<213> Artificial Sequence

<220>
<221> source
<223> /note="Description of Artificial Sequence: Synthetic
      6xHis tag"

<400> 39
His His His His His His
1               5

<210> 40
<211> 48
<212> PRT
<213> Artificial Sequence

<220>
<221> source
<223> /note="Description of Artificial Sequence: Synthetic
      polypeptide"

<400> 40
Leu Glu Gly Glu Val Asn Lys Ile Lys Ser Ala Leu Leu Ser Thr Asn
1               5                   10                  15

Lys Ala Val Val Ser Leu Ser Asn Gly Val Ser Val Leu Thr Ser Lys
            20                  25                  30

Val Leu Asp Leu Lys Asn Tyr Ile Asp Lys Gln Leu Leu Pro Ile Val
        35                  40                  45

<210> 41
<211> 26
<212> PRT

<213> Respiratory syncytial virus

<400> 41
Phe Tyr Asp Pro Leu Val Phe Pro Ser Asp Glu Phe Asp Ala Ser Ile
1               5                   10                  15

Ser Gln Val Asn Glu Lys Ile Asn Gln Ser
            20                  25

<210> 42
<211> 52
<212> PRT
<213> Respiratory syncytial virus

<400> 42
Leu His Leu Glu Gly Glu Val Asn Lys Ile Lys Ser Ala Leu Leu Ser
1               5                   10                  15

Thr Asn Lys Ala Val Val Ser Leu Ser Asn Gly Val Ser Val Leu Thr
            20                  25                  30

Ser Lys Val Leu Asp Leu Lys Asn Tyr Ile Asp Lys Gln Leu Leu Pro
        35                  40                  45

Ile Val Asn Lys
    50

<210> 43
<211> 43
<212> PRT
<213> Respiratory syncytial virus

<400> 43
Phe Tyr Asp Pro Leu Val Phe Pro Ser Asp Glu Phe Asp Ala Ser Ile
1               5                   10                  15

Ser Gln Val Asn Glu Lys Ile Asn Gln Ser Leu Ala Phe Ile Arg Lys
            20                  25                  30

Ser Asp Glu Leu Leu His Asn Val Asn Ala Gly
        35                  40

<210> 44
<211> 513
<212> PRT
<213> Respiratory syncytial virus

<400> 44
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile

275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu

<210> 45
<211> 7
<212> PRT
<213> Artificial Sequence

<220>
<221> source
<223> /note="Description of Artificial Sequence: Synthetic
      linker peptide"

<400> 45
Gly Gly Ser Ala Gly Ser Gly
1               5


<210> 46
<211> 21
<212> PRT
<213> Artificial Sequence

<220>
<221> source
<223> /note="Description of Artificial Sequence: Synthetic
      linker peptide"

<400> 46
Gly Gly Ser Ala Gly Ser Gly Gly Gly Ser Ala Gly Ser Gly Gly Gly
1               5                   10                  15


Ser Ala Gly Ser Gly
            20


<210> 47
<211> 595
<212> PRT
<213> Artificial Sequence

<220>
<221> source
<223> /note="Description of Artificial Sequence: Synthetic
      polypeptide"

<400> 47
Met Glu Leu Leu Ile Leu Lys Ala Asn Ala Ile Thr Thr Ile Leu Thr
1               5                   10                  15


Ala Val Thr Phe Cys Phe Ala Ser Gly Gln Asn Ile Thr Glu Glu Phe
            20                  25                  30


Tyr Gln Ser Thr Cys Ser Ala Val Ser Lys Gly Tyr Leu Ser Ala Leu
        35                  40                  45


Arg Thr Gly Trp Tyr Thr Ser Val Ile Thr Ile Glu Leu Ser Asn Ile
    50                  55                  60


Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg

325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Leu His Leu Glu Gly Glu Val Asn
        515                 520                 525

Lys Ile Lys Ser Ala Leu Leu Ser Thr Asn Lys Ala Val Val Ser Leu
    530                 535                 540

Ser Asn Gly Val Ser Val Leu Thr Ser Lys Val Leu Asp Leu Lys Asn
545                 550                 555                 560

Tyr Ile Asp Lys Gln Leu Leu Pro Ile Val Asn Lys Gly Gly Ser Ala
                565                 570                 575

Gly Ser Gly Ser Ala Trp Ser His Pro Gln Phe Glu Lys His His His
            580                 585                 590

His His His
        595

<210> 48
<211> 572
<212> PRT
<213> Artificial Sequence

<220>
<221> source
<223> /note="Description of Artificial Sequence: Synthetic
      polypeptide"

<400> 48
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Leu His Leu Glu Gly Glu Val Asn
        515                 520                 525

Lys Ile Lys Ser Ala Leu Leu Ser Thr Asn Lys Ala Val Val Ser Leu
    530                 535                 540

Ser Asn Gly Val Ser Val Leu Thr Ser Lys Val Leu Asp Leu Lys Asn
545                 550                 555                 560

Tyr Ile Asp Lys Gln Leu Leu Pro Ile Val Asn Lys
                565                 570

<210> 49
<211> 609
<212> PRT
<213> Artificial Sequence

<220>
<221> source
<223> /note="Description of Artificial Sequence: Synthetic
      polypeptide"

<400> 49
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Gly Gly Ser Ala Gly Ser Gly Gly
        515                 520                 525

Gly Ser Ala Gly Ser Gly Leu His Leu Glu Gly Glu Val Asn Lys Ile
    530                 535                 540

Lys Ser Ala Leu Leu Ser Thr Asn Lys Ala Val Val Ser Leu Ser Asn
545                 550                 555                 560

Gly Val Ser Val Leu Thr Ser Lys Val Leu Asp Leu Lys Asn Tyr Ile

565                 570                 575

Asp Lys Gln Leu Leu Pro Ile Val Asn Lys Gly Gly Ser Ala Gly Ser
            580                 585                 590

Gly Ser Ala Trp Ser His Pro Gln Phe Glu Lys His His His His His
        595                 600                 605

His

<210> 50
<211> 586
<212> PRT
<213> Artificial Sequence

<220>
<221> source
<223> /note="Description of Artificial Sequence: Synthetic
      polypeptide"

<400> 50
   Met Glu Leu Leu Ile Leu Lys Ala Asn Ala Ile Thr Thr Ile Leu Thr
   1               5                   10                  15
   Ala Val Thr Phe Cys Phe Ala Ser Gly Gln Asn Ile Thr Glu Glu Phe
               20                  25                  30
   Tyr Gln Ser Thr Cys Ser Ala Val Ser Lys Gly Tyr Leu Ser Ala Leu
           35                  40                  45
   Arg Thr Gly Trp Tyr Thr Ser Val Ile Thr Ile Glu Leu Ser Asn Ile
       50                  55                  60
   Lys Glu Asn Lys Cys Asn Gly Thr Asp Ala Lys Val Lys Leu Ile Lys
   65                  70                  75                  80
   Gln Glu Leu Asp Lys Tyr Lys Asn Ala Val Thr Glu Leu Gln Leu Leu
                   85                  90                  95
   Met Gln Ser Thr Pro Ala Thr Asn Asn Arg Ala Arg Arg Glu Leu Pro
               100                 105                 110
   Arg Phe Met Asn Tyr Thr Leu Asn Asn Ala Lys Lys Thr Asn Val Thr
           115                 120                 125
   Leu Ser Lys Lys Arg Lys Arg Arg Phe Leu Gly Phe Leu Leu Gly Val
       130                 135                 140
   Gly Ser Ala Ile Ala Ser Gly Val Ala Val Cys Lys Val Leu His Leu
   145                 150                 155                 160
   Glu Gly Glu Val Asn Lys Ile Lys Ser Ala Leu Leu Ser Thr Asn Lys
                   165                 170                 175
   Ala Val Val Ser Leu Ser Asn Gly Val Ser Val Leu Thr Phe Lys Val
               180                 185                 190
   Leu Asp Leu Lys Asn Tyr Ile Asp Lys Gln Leu Leu Pro Ile Leu Asn
           195                 200                 205
   Lys Gln Ser Cys Ser Ile Ser Asn Ile Glu Thr Val Ile Glu Phe Gln
       210                 215                 220
   Gln Lys Asn Asn Arg Leu Leu Glu Ile Thr Arg Glu Phe Ser Val Asn
   225                 230                 235                 240
   Ala Gly Val Thr Thr Pro Val Ser Thr Tyr Met Leu Thr Asn Ser Glu
                   245                 250                 255
   Leu Leu Ser Leu Ile Asn Asp Met Pro Ile Thr Asn Asp Gln Lys Lys
               260                 265                 270
   Leu Met Ser Asn Asn Val Gln Ile Val Arg Gln Gln Ser Tyr Ser Ile
           275                 280                 285
   Met Cys Ile Ile Lys Glu Glu Val Leu Ala Tyr Val Val Gln Leu Pro
       290                 295                 300
   Leu Tyr Gly Val Ile Asp Thr Pro Cys Trp Lys Leu His Thr Ser Pro
   305                 310                 315                 320
   Leu Cys Thr Thr Asn Thr Lys Glu Gly Ser Asn Ile Cys Leu Thr Arg
                   325                 330                 335
   Thr Asp Arg Gly Trp Tyr Cys Asp Asn Ala Gly Ser Val Ser Phe Phe
               340                 345                 350
   Pro Gln Ala Glu Thr Cys Lys Val Gln Ser Asn Arg Val Phe Cys Asp
           355                 360                 365
   Thr Met Asn Ser Leu Thr Leu Pro Ser Glu Val Asn Leu Cys Asn Val
       370                 375                 380
   Asp Ile Phe Asn Pro Lys Tyr Asp Cys Lys Ile Met Thr Ser Lys Thr
   385                 390                 395                 400
   Asp Val Ser Ser Ser Val Ile Thr Ser Leu Gly Ala Ile Val Ser Cys
                   405                 410                 415
   Tyr Gly Lys Thr Lys Cys Thr Ala Ser Asn Lys Asn Arg Gly Ile Ile
               420                 425                 430
   Lys Thr Phe Ser Asn Gly Cys Asp Tyr Val Ser Asn Lys Gly Val Asp
           435                 440                 445
   Thr Val Ser Val Gly Asn Thr Leu Tyr Tyr Val Asn Lys Gln Glu Gly
       450                 455                 460
   Lys Ser Leu Tyr Val Lys Gly Glu Pro Ile Ile Asn Phe Tyr Asp Pro
   465                 470                 475                 480
   Leu Val Phe Pro Ser Asp Glu Phe Asp Ala Ser Ile Ser Gln Val Asn
                   485                 490                 495
   Glu Lys Ile Asn Gln Ser Leu Ala Phe Ile Arg Lys Ser Asp Glu Leu
               500                 505                 510
   Leu Gly Gly Ser Ala Gly Ser Gly Gly Gly Ser Ala Gly Ser Gly Gly
           515                 520                 525
   Gly Ser Ala Gly Ser Gly Leu His Leu Glu Gly Glu Val Asn Lys Ile
       530                 535                 540
   Lys Ser Ala Leu Leu Ser Thr Asn Lys Ala Val Val Ser Leu Ser Asn
   545                 550                 555                 560
   Gly Val Ser Val Leu Thr Ser Lys Val Leu Asp Leu Lys Asn Tyr Ile
                   565                 570                 575
   Asp Lys Gln Leu Leu Pro Ile Val Asn Lys
               580                 585

<210> 51
<211> 645
<212> PRT
<213> Artificial Sequence

<220>
<221> source
<223> /note="Description of Artificial Sequence: Synthetic
      polypeptide"

<400> 51
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Leu His Leu Glu Gly Glu Val Asn
        515                 520                 525

Lys Ile Lys Ser Ala Leu Leu Ser Thr Asn Lys Ala Val Val Ser Leu
    530                 535                 540

Ser Asn Gly Val Ser Val Leu Thr Ser Lys Val Leu Asp Leu Lys Asn
545                 550                 555                 560

Tyr Ile Asp Lys Gln Leu Leu Pro Ile Val Asn Lys Gly Gly Ser Ala
                565                 570                 575

Gly Ser Gly Phe Tyr Asp Pro Leu Val Phe Pro Ser Asp Glu Phe Asp
            580                 585                 590

Ala Ser Ile Ser Gln Val Asn Glu Lys Ile Asn Gln Ser Leu Ala Phe
        595                 600                 605

Ile Arg Lys Ser Asp Glu Leu Leu His Asn Val Asn Ala Gly Gly Gly
    610                 615                 620

Ser Ala Gly Ser Gly Ser Ala Trp Ser His Pro Gln Phe Glu Lys His
625                 630                 635                 640

His His His His His
                645

<210> 52
<211> 622
<212> PRT
<213> Artificial Sequence

<220>
<221> source
<223> /note="Description of Artificial Sequence: Synthetic
polypeptide"

<400> 52
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg

325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Leu His Leu Glu Gly Glu Val Asn
        515                 520                 525

Lys Ile Lys Ser Ala Leu Leu Ser Thr Asn Lys Ala Val Val Ser Leu
    530                 535                 540

Ser Asn Gly Val Ser Val Leu Thr Ser Lys Val Leu Asp Leu Lys Asn
545                 550                 555                 560

Tyr Ile Asp Lys Gln Leu Leu Pro Ile Val Asn Lys Gly Gly Ser Ala
                565                 570                 575

Gly Ser Gly Phe Tyr Asp Pro Leu Val Phe Pro Ser Asp Glu Phe Asp
            580                 585                 590

Ala Ser Ile Ser Gln Val Asn Glu Lys Ile Asn Gln Ser Leu Ala Phe
        595                 600                 605

Ile Arg Lys Ser Asp Glu Leu Leu His Asn Val Asn Ala Gly
    610                 615                 620

<210> 53
<211> 659
<212> PRT
<213> Artificial Sequence

<220>
<221> source
<223> /note="Description of Artificial Sequence: Synthetic
      polypeptide"

<400> 53
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Leu His Leu Glu Gly Glu Val Asn
        515                 520                 525

Lys Ile Lys Ser Ala Leu Leu Ser Thr Asn Lys Ala Val Val Ser Leu
    530                 535                 540

Ser Asn Gly Val Ser Val Leu Thr Ser Lys Val Leu Asp Leu Lys Asn
545                 550                 555                 560

Tyr Ile Asp Lys Gln Leu Leu Pro Ile Val Asn Lys Gly Gly Ser Ala
                565                 570                 575

Gly Ser Gly Gly Gly Ser Ala Gly Ser Gly Gly Gly Ser Ala Gly Ser
            580                 585                 590

Gly Phe Tyr Asp Pro Leu Val Phe Pro Ser Asp Glu Phe Asp Ala Ser
        595                 600                 605

Ile Ser Gln Val Asn Glu Lys Ile Asn Gln Ser Leu Ala Phe Ile Arg
    610                 615                 620

Lys Ser Asp Glu Leu Leu His Asn Val Asn Ala Gly Gly Gly Ser Ala
625                 630                 635                 640

Gly Ser Gly Ser Ala Trp Ser His Pro Gln Phe Glu Lys His His His
                645                 650                 655

His His His

<210> 54
<211> 636
<212> PRT
<213> Artificial Sequence

<220>
<221> source
<223> /note="Description of Artificial Sequence: Synthetic
      polypeptide"

<400> 54
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly

450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Leu His Leu Glu Gly Glu Val Asn
        515                 520                 525

Lys Ile Lys Ser Ala Leu Leu Ser Thr Asn Lys Ala Val Val Ser Leu
    530                 535                 540

Ser Asn Gly Val Ser Val Leu Thr Ser Lys Val Leu Asp Leu Lys Asn
545                 550                 555                 560

Tyr Ile Asp Lys Gln Leu Leu Pro Ile Val Asn Lys Gly Gly Ser Ala
                565                 570                 575

Gly Ser Gly Gly Gly Ser Ala Gly Ser Gly Gly Gly Ser Ala Gly Ser
            580                 585                 590

Gly Phe Tyr Asp Pro Leu Val Phe Pro Ser Asp Glu Phe Asp Ala Ser
        595                 600                 605

Ile Ser Gln Val Asn Glu Lys Ile Asn Gln Ser Leu Ala Phe Ile Arg
    610                 615                 620

Lys Ser Asp Glu Leu Leu His Asn Val Asn Ala Gly
625                 630                 635

<210> 55
<211> 659
<212> PRT
<213> Artificial Sequence

<220>
<221> source
<223> /note="Description of Artificial Sequence: Synthetic
      polypeptide"

<400> 55
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe

20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Gly Gly Ser Ala Gly Ser Gly Gly
        515                 520                 525

Gly Ser Ala Gly Ser Gly Leu His Leu Glu Gly Glu Val Asn Lys Ile
    530                 535                 540

Lys Ser Ala Leu Leu Ser Thr Asn Lys Ala Val Val Ser Leu Ser Asn
545                 550                 555                 560

Gly Val Ser Val Leu Thr Ser Lys Val Leu Asp Leu Lys Asn Tyr Ile
                565                 570                 575

Asp Lys Gln Leu Leu Pro Ile Val Asn Lys Gly Gly Ser Ala Gly Ser
            580                 585                 590

Gly Phe Tyr Asp Pro Leu Val Phe Pro Ser Asp Glu Phe Asp Ala Ser
        595                 600                 605

Ile Ser Gln Val Asn Glu Lys Ile Asn Gln Ser Leu Ala Phe Ile Arg
    610                 615                 620

Lys Ser Asp Glu Leu Leu His Asn Val Asn Ala Gly Gly Gly Ser Ala
625                 630                 635                 640

Gly Ser Gly Ser Ala Trp Ser His Pro Gln Phe Glu Lys His His His
                645                 650                 655

His His His

<210> 56
<211> 636
<212> PRT
<213> Artificial Sequence

<220>
<221> source
<223> /note="Description of Artificial Sequence: Synthetic
      polypeptide"

<400> 56
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Gly Gly Ser Ala Gly Ser Gly Gly
        515                 520                 525

Gly Ser Ala Gly Ser Gly Leu His Leu Glu Gly Glu Val Asn Lys Ile
    530                 535                 540

Lys Ser Ala Leu Leu Ser Thr Asn Lys Ala Val Val Ser Leu Ser Asn
545                 550                 555                 560

Gly Val Ser Val Leu Thr Ser Lys Val Leu Asp Leu Lys Asn Tyr Ile

565                 570                 575

Asp Lys Gln Leu Leu Pro Ile Val Asn Lys Gly Gly Ser Ala Gly Ser
            580                 585                 590

Gly Phe Tyr Asp Pro Leu Val Phe Pro Ser Asp Glu Phe Asp Ala Ser
        595                 600                 605

Ile Ser Gln Val Asn Glu Lys Ile Asn Gln Ser Leu Ala Phe Ile Arg
    610                 615                 620

Lys Ser Asp Glu Leu Leu His Asn Val Asn Ala Gly
625                 630                 635

<210> 57
<211> 673
<212> PRT
<213> Artificial Sequence

<220>
<221> source
<223> /note="Description of Artificial Sequence: Synthetic
      polypeptide"

<400> 57
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val

130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Gly Gly Ser Ala Gly Ser Gly Gly
        515                 520                 525

Gly Ser Ala Gly Ser Gly Leu His Leu Glu Gly Glu Val Asn Lys Ile
    530                 535                 540

Lys Ser Ala Leu Leu Ser Thr Asn Lys Ala Val Val Ser Leu Ser Asn
545                 550                 555                 560

Gly Val Ser Val Leu Thr Ser Lys Val Leu Asp Leu Lys Asn Tyr Ile
                565                 570                 575

Asp Lys Gln Leu Leu Pro Ile Val Asn Lys Gly Gly Ser Ala Gly Ser
            580                 585                 590

Gly Gly Gly Ser Ala Gly Ser Gly Gly Gly Ser Ala Gly Ser Gly Phe
        595                 600                 605

Tyr Asp Pro Leu Val Phe Pro Ser Asp Glu Phe Asp Ala Ser Ile Ser
    610                 615                 620

Gln Val Asn Glu Lys Ile Asn Gln Ser Leu Ala Phe Ile Arg Lys Ser
625                 630                 635                 640

Asp Glu Leu Leu His Asn Val Asn Ala Gly Gly Gly Ser Ala Gly Ser
                645                 650                 655

Gly Ser Ala Trp Ser His Pro Gln Phe Glu Lys His His His His His
            660                 665                 670

His

<210> 58
<211> 650
<212> PRT
<213> Artificial Sequence

<220>
<221> source
<223> /note="Description of Artificial Sequence: Synthetic
      polypeptide"

<400> 58
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Gly Gly Ser Ala Gly Ser Gly Gly
        515                 520                 525

Gly Ser Ala Gly Ser Gly Leu His Leu Glu Gly Glu Val Asn Lys Ile
    530                 535                 540

Lys Ser Ala Leu Leu Ser Thr Asn Lys Ala Val Val Ser Leu Ser Asn
545                 550                 555                 560

Gly Val Ser Val Leu Thr Ser Lys Val Leu Asp Leu Lys Asn Tyr Ile
                565                 570                 575

Asp Lys Gln Leu Leu Pro Ile Val Asn Lys Gly Gly Ser Ala Gly Ser
            580                 585                 590

Gly Gly Gly Ser Ala Gly Ser Gly Gly Gly Ser Ala Gly Ser Gly Phe
        595                 600                 605

Tyr Asp Pro Leu Val Phe Pro Ser Asp Glu Phe Asp Ala Ser Ile Ser
    610                 615                 620

Gln Val Asn Glu Lys Ile Asn Gln Ser Leu Ala Phe Ile Arg Lys Ser
625                 630                 635                 640

Asp Glu Leu Leu His Asn Val Asn Ala Gly
                645                 650

<210> 59

<211> 634
<212> PRT
<213> Artificial Sequence

<220>
<221> source
<223> /note="Description of Artificial Sequence: Synthetic
      polypeptide"

<400> 59
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln

210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Leu His Leu
            500                 505                 510

Glu Gly Glu Val Asn Lys Ile Lys Ser Ala Leu Leu Ser Thr Asn Lys
        515                 520                 525

Ala Val Val Ser Leu Ser Asn Gly Val Ser Val Leu Thr Ser Lys Val
    530                 535                 540

Leu Asp Leu Lys Asn Tyr Ile Asp Lys Gln Leu Leu Pro Ile Val Asn
545                 550                 555                 560

Lys Gly Gly Ser Ala Gly Ser Gly Phe Tyr Asp Pro Leu Val Phe Pro
                565                 570                 575

Ser Asp Glu Phe Asp Ala Ser Ile Ser Gln Val Asn Glu Lys Ile Asn
            580                 585                 590

Gln Ser Leu Ala Phe Ile Arg Lys Ser Asp Glu Leu Leu His Asn Val
        595                 600                 605

Asn Ala Gly Gly Gly Ser Ala Gly Ser Gly Ser Ala Trp Ser His Pro
    610                 615                 620

Gln Phe Glu Lys His His His His His His
625                 630

<210> 60
<211> 611
<212> PRT
<213> Artificial Sequence

<220>
<221> source
<223> /note="Description of Artificial Sequence: Synthetic
      polypeptide"

<400> 60
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Leu His Leu
            500                 505                 510

Glu Gly Glu Val Asn Lys Ile Lys Ser Ala Leu Leu Ser Thr Asn Lys
        515                 520                 525

Ala Val Val Ser Leu Ser Asn Gly Val Ser Val Leu Thr Ser Lys Val
    530                 535                 540

Leu Asp Leu Lys Asn Tyr Ile Asp Lys Gln Leu Leu Pro Ile Val Asn
545                 550                 555                 560

Lys Gly Gly Ser Ala Gly Ser Gly Phe Tyr Asp Pro Leu Val Phe Pro
                565                 570                 575

Ser Asp Glu Phe Asp Ala Ser Ile Ser Gln Val Asn Glu Lys Ile Asn
            580                 585                 590

Gln Ser Leu Ala Phe Ile Arg Lys Ser Asp Glu Leu Leu His Asn Val
        595                 600                 605

Asn Ala Gly
    610

<210> 61
<211> 648
<212> PRT
<213> Artificial Sequence

<220>
<221> source
<223> /note="Description of Artificial Sequence: Synthetic
      polypeptide"

<400> 61
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val

370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Leu His Leu
            500                 505                 510

Glu Gly Glu Val Asn Lys Ile Lys Ser Ala Leu Leu Ser Thr Asn Lys
        515                 520                 525

Ala Val Val Ser Leu Ser Asn Gly Val Ser Val Leu Thr Ser Lys Val
    530                 535                 540

Leu Asp Leu Lys Asn Tyr Ile Asp Lys Gln Leu Leu Pro Ile Val Asn
545                 550                 555                 560

Lys Gly Gly Ser Ala Gly Ser Gly Gly Gly Ser Ala Gly Ser Gly Gly
                565                 570                 575

Gly Ser Ala Gly Ser Gly Phe Tyr Asp Pro Leu Val Phe Pro Ser Asp
            580                 585                 590

Glu Phe Asp Ala Ser Ile Ser Gln Val Asn Glu Lys Ile Asn Gln Ser
        595                 600                 605

Leu Ala Phe Ile Arg Lys Ser Asp Glu Leu Leu His Asn Val Asn Ala
    610                 615                 620

Gly Gly Gly Ser Ala Gly Ser Gly Ser Ala Trp Ser His Pro Gln Phe
625                 630                 635                 640

Glu Lys His His His His His His
                645

<210> 62
<211> 625
<212> PRT
<213> Artificial Sequence

<220>
<221> source
<223> /note="Description of Artificial Sequence: Synthetic
      polypeptide"

<400> 62
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Leu His Leu
            500                 505                 510

Glu Gly Glu Val Asn Lys Ile Lys Ser Ala Leu Leu Ser Thr Asn Lys
        515                 520                 525

Ala Val Val Ser Leu Ser Asn Gly Val Ser Val Leu Thr Ser Lys Val
    530                 535                 540

Leu Asp Leu Lys Asn Tyr Ile Asp Lys Gln Leu Leu Pro Ile Val Asn
545                 550                 555                 560

Lys Gly Gly Ser Ala Gly Ser Gly Gly Gly Ser Ala Gly Ser Gly Gly
                565                 570                 575

Gly Ser Ala Gly Ser Gly Phe Tyr Asp Pro Leu Val Phe Pro Ser Asp
            580                 585                 590

Glu Phe Asp Ala Ser Ile Ser Gln Val Asn Glu Lys Ile Asn Gln Ser
        595                 600                 605

Leu Ala Phe Ile Arg Lys Ser Asp Glu Leu Leu His Asn Val Asn Ala
    610                 615                 620

Gly
625

<210> 63
<211> 648
<212> PRT
<213> Artificial Sequence

<220>
<221> source
<223> /note="Description of Artificial Sequence: Synthetic
polypeptide"

<400> 63
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Gly Gly Ser

500                 505                 510

Ala Gly Ser Gly Gly Gly Ser Ala Gly Ser Gly Leu His Leu Glu Gly
        515                 520                 525

Glu Val Asn Lys Ile Lys Ser Ala Leu Leu Ser Thr Asn Lys Ala Val
    530                 535                 540

Val Ser Leu Ser Asn Gly Val Ser Val Leu Thr Ser Lys Val Leu Asp
545                 550                 555                 560

Leu Lys Asn Tyr Ile Asp Lys Gln Leu Leu Pro Ile Val Asn Lys Gly
                565                 570                 575

Gly Ser Ala Gly Ser Gly Phe Tyr Asp Pro Leu Val Phe Pro Ser Asp
            580                 585                 590

Glu Phe Asp Ala Ser Ile Ser Gln Val Asn Glu Lys Ile Asn Gln Ser
        595                 600                 605

Leu Ala Phe Ile Arg Lys Ser Asp Glu Leu Leu His Asn Val Asn Ala
    610                 615                 620

Gly Gly Gly Ser Ala Gly Ser Gly Ser Ala Trp Ser His Pro Gln Phe
625                 630                 635                 640

Glu Lys His His His His His His
                645

<210> 64
<211> 625
<212> PRT
<213> Artificial Sequence

<220>
<221> source
<223> /note="Description of Artificial Sequence: Synthetic
      polypeptide"

<400> 64
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile

50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Gly Gly Ser
            500                 505                 510

Ala Gly Ser Gly Gly Gly Ser Ala Gly Ser Gly Leu His Leu Glu Gly
        515                 520                 525

Glu Val Asn Lys Ile Lys Ser Ala Leu Leu Ser Thr Asn Lys Ala Val
    530                 535                 540

Val Ser Leu Ser Asn Gly Val Ser Val Leu Thr Ser Lys Val Leu Asp
545                 550                 555                 560

Leu Lys Asn Tyr Ile Asp Lys Gln Leu Leu Pro Ile Val Asn Lys Gly
                565                 570                 575

Gly Ser Ala Gly Ser Gly Phe Tyr Asp Pro Leu Val Phe Pro Ser Asp
            580                 585                 590

Glu Phe Asp Ala Ser Ile Ser Gln Val Asn Glu Lys Ile Asn Gln Ser
        595                 600                 605

Leu Ala Phe Ile Arg Lys Ser Asp Glu Leu Leu His Asn Val Asn Ala
    610                 615                 620

Gly
625

<210> 65
<211> 662
<212> PRT
<213> Artificial Sequence

<220>
<221> source
<223> /note="Description of Artificial Sequence: Synthetic
      polypeptide"

<400> 65
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Gly Gly Ser
            500                 505                 510

Ala Gly Ser Gly Gly Gly Ser Ala Gly Ser Gly Leu His Leu Glu Gly
        515                 520                 525

Glu Val Asn Lys Ile Lys Ser Ala Leu Leu Ser Thr Asn Lys Ala Val
    530                 535                 540

Val Ser Leu Ser Asn Gly Val Ser Val Leu Thr Ser Lys Val Leu Asp
545                 550                 555                 560

Leu Lys Asn Tyr Ile Asp Lys Gln Leu Leu Pro Ile Val Asn Lys Gly
                565                 570                 575

Gly Ser Ala Gly Ser Gly Gly Gly Ser Ala Gly Ser Gly Gly Gly Ser
            580                 585                 590

Ala Gly Ser Gly Phe Tyr Asp Pro Leu Val Phe Pro Ser Asp Glu Phe
        595                 600                 605

Asp Ala Ser Ile Ser Gln Val Asn Glu Lys Ile Asn Gln Ser Leu Ala
    610                 615                 620

Phe Ile Arg Lys Ser Asp Glu Leu Leu His Asn Val Asn Ala Gly Gly

625                 630                 635                 640

Gly Ser Ala Gly Ser Gly Ser Ala Trp Ser His Pro Gln Phe Glu Lys
                645                 650                 655

His His His His His His
            660

<210> 66
<211> 639
<212> PRT
<213> Artificial Sequence

<220>
<221> source
<223> /note="Description of Artificial Sequence: Synthetic
      polypeptide"

<400> 66
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys

                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Gly Gly Ser
            500                 505                 510

Ala Gly Ser Gly Gly Gly Ser Ala Gly Ser Gly Leu His Leu Glu Gly
        515                 520                 525

Glu Val Asn Lys Ile Lys Ser Ala Leu Leu Ser Thr Asn Lys Ala Val
    530                 535                 540

Val Ser Leu Ser Asn Gly Val Ser Val Leu Thr Ser Lys Val Leu Asp
545                 550                 555                 560

Leu Lys Asn Tyr Ile Asp Lys Gln Leu Leu Pro Ile Val Asn Lys Gly
                565                 570                 575

Gly Ser Ala Gly Ser Gly Gly Gly Ser Ala Gly Ser Gly Gly Gly Ser
            580                 585                 590

Ala Gly Ser Gly Phe Tyr Asp Pro Leu Val Phe Pro Ser Asp Glu Phe
        595                 600                 605

Asp Ala Ser Ile Ser Gln Val Asn Glu Lys Ile Asn Gln Ser Leu Ala
    610                 615                 620

Phe Ile Arg Lys Ser Asp Glu Leu Leu His Asn Val Asn Ala Gly
625                 630                 635

<210> 67
<211> 82
<212> PRT
<213> Artificial Sequence

<220>
<221> source

<223> /note="Description of Artificial Sequence: Synthetic
      polypeptide"

<400> 67
Gly Gly Ser Ala Gly Ser Gly Leu His Leu Glu Gly Glu Val Asn Lys
1               5                   10                  15

Ile Lys Ser Ala Leu Leu Ser Thr Asn Lys Ala Val Val Ser Leu Ser
            20                  25                  30

Asn Gly Val Ser Val Leu Thr Ser Lys Val Leu Asp Leu Lys Asn Tyr
        35                  40                  45

Ile Asp Lys Gln Leu Leu Pro Ile Val Asn Lys Gly Gly Ser Ala Gly
    50                  55                  60

Ser Gly Ser Ala Trp Ser His Pro Gln Phe Glu Lys His His His His
65                  70                  75                  80

His His

<210> 68
<211> 59
<212> PRT
<213> Artificial Sequence

<220>
<221> source
<223> /note="Description of Artificial Sequence: Synthetic
      polypeptide"

<400> 68
Gly Gly Ser Ala Gly Ser Gly Leu His Leu Glu Gly Glu Val Asn Lys
1               5                   10                  15

Ile Lys Ser Ala Leu Leu Ser Thr Asn Lys Ala Val Val Ser Leu Ser
            20                  25                  30

Asn Gly Val Ser Val Leu Thr Ser Lys Val Leu Asp Leu Lys Asn Tyr
        35                  40                  45

Ile Asp Lys Gln Leu Leu Pro Ile Val Asn Lys
    50                  55

<210> 69
<211> 96
<212> PRT
<213> Artificial Sequence

<220>
<221> source
<223> /note="Description of Artificial Sequence: Synthetic

polypeptide"

<400> 69
Gly Gly Ser Ala Gly Ser Gly Gly Gly Ser Ala Gly Ser Gly Gly Gly
1               5                   10                  15

Ser Ala Gly Ser Gly Leu His Leu Glu Gly Glu Val Asn Lys Ile Lys
            20                  25                  30

Ser Ala Leu Leu Ser Thr Asn Lys Ala Val Val Ser Leu Ser Asn Gly
        35                  40                  45

Val Ser Val Leu Thr Ser Lys Val Leu Asp Leu Lys Asn Tyr Ile Asp
    50                  55                  60

Lys Gln Leu Leu Pro Ile Val Asn Lys Gly Gly Ser Ala Gly Ser Gly
65                  70                  75                  80

Ser Ala Trp Ser His Pro Gln Phe Glu Lys His His His His His His
                85                  90                  95

<210> 70
<211> 73
<212> PRT
<213> Artificial Sequence

<220>
<221> source
<223> /note="Description of Artificial Sequence: Synthetic
      polypeptide"

<400> 70
Gly Gly Ser Ala Gly Ser Gly Gly Gly Ser Ala Gly Ser Gly Gly Gly
1               5                   10                  15

Ser Ala Gly Ser Gly Leu His Leu Glu Gly Glu Val Asn Lys Ile Lys
            20                  25                  30

Ser Ala Leu Leu Ser Thr Asn Lys Ala Val Val Ser Leu Ser Asn Gly
        35                  40                  45

Val Ser Val Leu Thr Ser Lys Val Leu Asp Leu Lys Asn Tyr Ile Asp
    50                  55                  60

Lys Gln Leu Leu Pro Ile Val Asn Lys
65                  70

<210> 71
<211> 132
<212> PRT
<213> Artificial Sequence

<220>
<221> source
<223> /note="Description of Artificial Sequence: Synthetic
      polypeptide"

<400> 71
Gly Gly Ser Ala Gly Ser Gly Leu His Leu Glu Gly Glu Val Asn Lys
1               5                   10                  15

Ile Lys Ser Ala Leu Leu Ser Thr Asn Lys Ala Val Val Ser Leu Ser
            20                  25                  30

Asn Gly Val Ser Val Leu Thr Ser Lys Val Leu Asp Leu Lys Asn Tyr
        35                  40                  45

Ile Asp Lys Gln Leu Leu Pro Ile Val Asn Lys Gly Gly Ser Ala Gly
    50                  55                  60

Ser Gly Phe Tyr Asp Pro Leu Val Phe Pro Ser Asp Glu Phe Asp Ala
65                  70                  75                  80

Ser Ile Ser Gln Val Asn Glu Lys Ile Asn Gln Ser Leu Ala Phe Ile
                85                  90                  95

Arg Lys Ser Asp Glu Leu Leu His Asn Val Asn Ala Gly Gly Gly Ser
            100                 105                 110

Ala Gly Ser Gly Ser Ala Trp Ser His Pro Gln Phe Glu Lys His His
        115                 120                 125

His His His His
    130


<210> 72
<211> 109
<212> PRT
<213> Artificial Sequence

<220>
<221> source
<223> /note="Description of Artificial Sequence: Synthetic
      polypeptide"

<400> 72
Gly Gly Ser Ala Gly Ser Gly Leu His Leu Glu Gly Glu Val Asn Lys
1               5                   10                  15

Ile Lys Ser Ala Leu Leu Ser Thr Asn Lys Ala Val Val Ser Leu Ser
            20                  25                  30

Asn Gly Val Ser Val Leu Thr Ser Lys Val Leu Asp Leu Lys Asn Tyr
        35                  40                  45

Ile Asp Lys Gln Leu Leu Pro Ile Val Asn Lys Gly Gly Ser Ala Gly
    50                  55                  60

Ser Gly Phe Tyr Asp Pro Leu Val Phe Pro Ser Asp Glu Phe Asp Ala
65                  70                  75                  80

Ser Ile Ser Gln Val Asn Glu Lys Ile Asn Gln Ser Leu Ala Phe Ile
                85                  90                  95

Arg Lys Ser Asp Glu Leu Leu His Asn Val Asn Ala Gly
            100                 105

<210> 73
<211> 146
<212> PRT
<213> Artificial Sequence

<220>
<221> source
<223> /note="Description of Artificial Sequence: Synthetic
      polypeptide"

<400> 73
Gly Gly Ser Ala Gly Ser Gly Leu His Leu Glu Gly Glu Val Asn Lys
1               5                   10                  15

Ile Lys Ser Ala Leu Leu Ser Thr Asn Lys Ala Val Val Ser Leu Ser
            20                  25                  30

Asn Gly Val Ser Val Leu Thr Ser Lys Val Leu Asp Leu Lys Asn Tyr
        35                  40                  45

Ile Asp Lys Gln Leu Leu Pro Ile Val Asn Lys Gly Gly Ser Ala Gly
    50                  55                  60

Ser Gly Gly Gly Ser Ala Gly Ser Gly Gly Gly Ser Ala Gly Ser Gly
65                  70                  75                  80

Phe Tyr Asp Pro Leu Val Phe Pro Ser Asp Glu Phe Asp Ala Ser Ile
                85                  90                  95

Ser Gln Val Asn Glu Lys Ile Asn Gln Ser Leu Ala Phe Ile Arg Lys
            100                 105                 110

Ser Asp Glu Leu Leu His Asn Val Asn Ala Gly Gly Gly Ser Ala Gly
        115                 120                 125

Ser Gly Ser Ala Trp Ser His Pro Gln Phe Glu Lys His His His His
    130                 135                 140

His His
145


<210> 74
<211> 123
<212> PRT
<213> Artificial Sequence

<220>
<221> source
<223> /note="Description of Artificial Sequence: Synthetic
      polypeptide"

<400> 74
Gly Gly Ser Ala Gly Ser Gly Leu His Leu Glu Gly Glu Val Asn Lys
1               5                   10                  15


Ile Lys Ser Ala Leu Leu Ser Thr Asn Lys Ala Val Val Ser Leu Ser
            20                  25                  30


Asn Gly Val Ser Val Leu Thr Ser Lys Val Leu Asp Leu Lys Asn Tyr
        35                  40                  45


Ile Asp Lys Gln Leu Leu Pro Ile Val Asn Lys Gly Gly Ser Ala Gly
    50                  55                  60


Ser Gly Gly Gly Ser Ala Gly Ser Gly Gly Gly Ser Ala Gly Ser Gly
65                  70                  75                  80


Phe Tyr Asp Pro Leu Val Phe Pro Ser Asp Glu Phe Asp Ala Ser Ile
                85                  90                  95


Ser Gln Val Asn Glu Lys Ile Asn Gln Ser Leu Ala Phe Ile Arg Lys
            100                 105                 110


Ser Asp Glu Leu Leu His Asn Val Asn Ala Gly
        115                 120


<210> 75
<211> 146
<212> PRT
<213> Artificial Sequence

<220>
<221> source
<223> /note="Description of Artificial Sequence: Synthetic
      polypeptide"

<400> 75
Gly Gly Ser Ala Gly Ser Gly Gly Gly Ser Ala Gly Ser Gly Gly Gly
1               5                   10                  15

Ser Ala Gly Ser Gly Leu His Leu Glu Gly Glu Val Asn Lys Ile Lys
            20                  25                  30

Ser Ala Leu Leu Ser Thr Asn Lys Ala Val Val Ser Leu Ser Asn Gly
        35                  40                  45

Val Ser Val Leu Thr Ser Lys Val Leu Asp Leu Lys Asn Tyr Ile Asp
    50                  55                  60

Lys Gln Leu Leu Pro Ile Val Asn Lys Gly Gly Ser Ala Gly Ser Gly
65                  70                  75                  80

Phe Tyr Asp Pro Leu Val Phe Pro Ser Asp Glu Phe Asp Ala Ser Ile
                85                  90                  95

Ser Gln Val Asn Glu Lys Ile Asn Gln Ser Leu Ala Phe Ile Arg Lys
            100                 105                 110

Ser Asp Glu Leu Leu His Asn Val Asn Ala Gly Gly Gly Ser Ala Gly
        115                 120                 125

Ser Gly Ser Ala Trp Ser His Pro Gln Phe Glu Lys His His His His
    130                 135                 140

His His
145

<210> 76
<211> 123
<212> PRT
<213> Artificial Sequence

<220>
<221> source
<223> /note="Description of Artificial Sequence: Synthetic
      polypeptide"

<400> 76
Gly Gly Ser Ala Gly Ser Gly Gly Gly Ser Ala Gly Ser Gly Gly Gly
1               5                   10                  15

Ser Ala Gly Ser Gly Leu His Leu Glu Gly Glu Val Asn Lys Ile Lys
            20                  25                  30

Ser Ala Leu Leu Ser Thr Asn Lys Ala Val Val Ser Leu Ser Asn Gly
        35                  40                  45

Val Ser Val Leu Thr Ser Lys Val Leu Asp Leu Lys Asn Tyr Ile Asp
    50                  55                  60

Lys Gln Leu Leu Pro Ile Val Asn Lys Gly Gly Ser Ala Gly Ser Gly
65                  70                  75                  80

Phe Tyr Asp Pro Leu Val Phe Pro Ser Asp Glu Phe Asp Ala Ser Ile
                85                  90                  95

Ser Gln Val Asn Glu Lys Ile Asn Gln Ser Leu Ala Phe Ile Arg Lys
            100                 105                 110

Ser Asp Glu Leu Leu His Asn Val Asn Ala Gly
        115                 120

<210> 77
<211> 160
<212> PRT
<213> Artificial Sequence

<220>
<221> source
<223> /note="Description of Artificial Sequence: Synthetic
      polypeptide"

<400> 77
Gly Gly Ser Ala Gly Ser Gly Gly Gly Ser Ala Gly Ser Gly Gly Gly
1               5                   10                  15

Ser Ala Gly Ser Gly Leu His Leu Glu Gly Glu Val Asn Lys Ile Lys
            20                  25                  30

Ser Ala Leu Leu Ser Thr Asn Lys Ala Val Val Ser Leu Ser Asn Gly
        35                  40                  45

Val Ser Val Leu Thr Ser Lys Val Leu Asp Leu Lys Asn Tyr Ile Asp
    50                  55                  60

Lys Gln Leu Leu Pro Ile Val Asn Lys Gly Gly Ser Ala Gly Ser Gly
65                  70                  75                  80

Gly Gly Ser Ala Gly Ser Gly Gly Gly Ser Ala Gly Ser Gly Phe Tyr
                85                  90                  95

Asp Pro Leu Val Phe Pro Ser Asp Glu Phe Asp Ala Ser Ile Ser Gln
            100                 105                 110

Val Asn Glu Lys Ile Asn Gln Ser Leu Ala Phe Ile Arg Lys Ser Asp
        115                 120                 125

Glu Leu Leu His Asn Val Asn Ala Gly Gly Gly Ser Ala Gly Ser Gly
    130                 135                 140

Ser Ala Trp Ser His Pro Gln Phe Glu Lys His His His His His His
145                 150                 155                 160

<210> 78
<211> 137
<212> PRT
<213> Artificial Sequence

<220>
<221> source
<223> /note="Description of Artificial Sequence: Synthetic
      polypeptide"

<400> 78
Gly Gly Ser Ala Gly Ser Gly Gly Gly Ser Ala Gly Ser Gly Gly Gly
1               5                   10                  15

Ser Ala Gly Ser Gly Leu His Leu Glu Gly Glu Val Asn Lys Ile Lys
            20                  25                  30

Ser Ala Leu Leu Ser Thr Asn Lys Ala Val Val Ser Leu Ser Asn Gly
        35                  40                  45

Val Ser Val Leu Thr Ser Lys Val Leu Asp Leu Lys Asn Tyr Ile Asp
    50                  55                  60

Lys Gln Leu Leu Pro Ile Val Asn Lys Gly Gly Ser Ala Gly Ser Gly
65                  70                  75                  80

Gly Gly Ser Ala Gly Ser Gly Gly Gly Ser Ala Gly Ser Gly Phe Tyr
                85                  90                  95

Asp Pro Leu Val Phe Pro Ser Asp Glu Phe Asp Ala Ser Ile Ser Gln
            100                 105                 110

Val Asn Glu Lys Ile Asn Gln Ser Leu Ala Phe Ile Arg Lys Ser Asp
        115                 120                 125

Glu Leu Leu His Asn Val Asn Ala Gly
    130                 135

<210> 79
<211> 59
<212> PRT
<213> Artificial Sequence

<220>
<221> source
<223> /note="Description of Artificial Sequence: Synthetic
      polypeptide"

<400> 79

Ser Ala Trp Ser His Pro Gln Phe Glu Lys Leu Val Pro Arg Gly Ser
1               5                   10                  15

Phe Tyr Asp Pro Leu Val Phe Pro Ser Asp Glu Phe Asp Ala Ser Ile
            20                  25                  30

Ser Gln Val Asn Glu Lys Ile Asn Gln Ser Leu Ala Phe Ile Arg Lys
        35                  40                  45

Ser Asp Glu Leu Leu His Asn Val Asn Ala Gly
    50                  55

<210> 80
<211> 572
<212> PRT
<213> Artificial Sequence

<220>
<221> source
<223> /note="Description of Artificial Sequence: Synthetic
      polypeptide"

<400> 80
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Leu His Leu Glu Gly Glu Val Asn
        515                 520                 525

Lys Ile Lys Ser Ala Leu Leu Ser Thr Asn Lys Ala Val Val Ser Leu
    530                 535                 540

Ser Asn Gly Val Ser Val Leu Thr Ser Lys Val Leu Asp Leu Lys Asn
545                 550                 555                 560

Tyr Ile Asp Lys Gln Leu Leu Pro Ile Val Asn Lys
                565                 570

<210> 81
<211> 586
<212> PRT
<213> Artificial Sequence

<220>
<221> source
<223> /note="Description of Artificial Sequence: Synthetic
      polypeptide"

<400> 81
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Gly Gly Ser Ala Gly Ser Gly Gly
        515                 520                 525

Gly Ser Ala Gly Ser Gly Leu His Leu Glu Gly Glu Val Asn Lys Ile

530                 535                 540

Lys Ser Ala Leu Leu Ser Thr Asn Lys Ala Val Val Ser Leu Ser Asn
545                 550                 555                 560

Gly Val Ser Val Leu Thr Ser Lys Val Leu Asp Leu Lys Asn Tyr Ile
                565                 570                 575

Asp Lys Gln Leu Leu Pro Ile Val Asn Lys
            580                 585

<210> 82
<211> 561
<212> PRT
<213> Artificial Sequence

<220>
<221> source
<223> /note="Description of Artificial Sequence: Synthetic
      polypeptide"

<400> 82
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu

145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Leu His Leu
            500                 505                 510

Glu Gly Glu Val Asn Lys Ile Lys Ser Ala Leu Leu Ser Thr Asn Lys
        515                 520                 525

Ala Val Val Ser Leu Ser Asn Gly Val Ser Val Leu Thr Ser Lys Val
    530                 535                 540

Leu Asp Leu Lys Asn Tyr Ile Asp Lys Gln Leu Leu Pro Ile Val Asn
545                 550                 555                 560

Lys

<210> 83
<211> 575
<212> PRT
<213> Artificial Sequence

<220>
<221> source
<223> /note="Description of Artificial Sequence: Synthetic
      polypeptide"

<400> 83
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Gly Gly Ser
            500                 505                 510

Ala Gly Ser Gly Gly Gly Ser Ala Gly Ser Gly Leu His Leu Glu Gly
        515                 520                 525

Glu Val Asn Lys Ile Lys Ser Ala Leu Leu Ser Thr Asn Lys Ala Val
    530                 535                 540

Val Ser Leu Ser Asn Gly Val Ser Val Leu Thr Ser Lys Val Leu Asp
545                 550                 555                 560

Leu Lys Asn Tyr Ile Asp Lys Gln Leu Leu Pro Ile Val Asn Lys
                565                 570                 575

<210> 84
<211> 572
<212> PRT
<213> Artificial Sequence

<220>
<221> source
<223> /note="Description of Artificial Sequence: Synthetic
      polypeptide"

<400> 84
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile

420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Leu His Leu Glu Gly Glu Val Asn
        515                 520                 525

Lys Ile Lys Ser Ala Leu Leu Ser Thr Asn Lys Ala Val Val Ser Leu
    530                 535                 540

Ser Asn Gly Val Ser Val Leu Thr Ser Lys Val Leu Asp Leu Lys Asn
545                 550                 555                 560

Tyr Ile Asp Lys Gln Leu Leu Pro Ile Val Asn Lys
                565                 570

<210> 85
<211> 586
<212> PRT
<213> Artificial Sequence

<220>
<221> source
<223> /note="Description of Artificial Sequence: Synthetic
      polypeptide"

<400> 85
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile

50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Gly Gly Ser Ala Gly Ser Gly Gly Gly Ser Ala Gly Ser Gly Gly
        515                 520                 525

Gly Ser Ala Gly Ser Gly Leu His Leu Glu Gly Glu Val Asn Lys Ile
    530                 535                 540

Lys Ser Ala Leu Leu Ser Thr Asn Lys Ala Val Val Ser Leu Ser Asn
545                 550                 555                 560

Gly Val Ser Val Leu Thr Ser Lys Val Leu Asp Leu Lys Asn Tyr Ile
                565                 570                 575

Asp Lys Gln Leu Leu Pro Ile Val Asn Lys
            580                 585

<210> 86
<211> 561
<212> PRT
<213> Artificial Sequence

<220>
<221> source
<223> /note="Description of Artificial Sequence: Synthetic
      polypeptide"

<400> 86
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Leu His Leu
            500                 505                 510

Glu Gly Glu Val Asn Lys Ile Lys Ser Ala Leu Leu Ser Thr Asn Lys
        515                 520                 525

Ala Val Val Ser Leu Ser Asn Gly Val Ser Val Leu Thr Ser Lys Val
    530                 535                 540

Leu Asp Leu Lys Asn Tyr Ile Asp Lys Gln Leu Leu Pro Ile Val Asn
545                 550                 555                 560

Lys

<210> 87
<211> 575
<212> PRT
<213> Artificial Sequence

<220>
<221> source
<223> /note="Description of Artificial Sequence: Synthetic
      polypeptide"

<400> 87
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro

305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Gly Gly Ser Ala Gly Ser Gly Gly Gly Ser
            500                 505                 510

Ala Gly Ser Gly Gly Gly Ser Ala Gly Ser Gly Leu His Leu Glu Gly
        515                 520                 525

Glu Val Asn Lys Ile Lys Ser Ala Leu Leu Ser Thr Asn Lys Ala Val
    530                 535                 540

Val Ser Leu Ser Asn Gly Val Ser Val Leu Thr Ser Lys Val Leu Asp
545                 550                 555                 560

Leu Lys Asn Tyr Ile Asp Lys Gln Leu Leu Pro Ile Val Asn Lys
                565                 570                 575

<210> 88
<211> 6
<212> PRT
<213> Artificial Sequence

<220>
<221> source
<223> /note="Description of Artificial Sequence: Synthetic
      peptide"

<400> 88
Leu Val Pro Arg Gly Ser
1               5

<210> 89
<211> 79
<212> PRT
<213> Artificial Sequence

<220>
<221> source
<223> /note="Description of Artificial Sequence: Synthetic
      polypeptide"

<400> 89
Ser Ala Trp Ser His Pro Gln Phe Glu Lys Gly Gly Gly Ser Gly Gly
1               5                   10                  15

Gly Ser Gly Gly Gly Ser Trp Ser His Pro Gln Phe Glu Lys Leu Val
            20                  25                  30

Pro Arg Gly Ser Phe Tyr Asp Pro Leu Val Phe Pro Ser Asp Glu Phe
        35                  40                  45

Asp Ala Ser Ile Ser Gln Val Asn Glu Lys Ile Asn Gln Ser Leu Ala
    50                  55                  60

Phe Ile Arg Lys Ser Asp Glu Leu Leu His Asn Val Asn Ala Gly
65                  70                  75

<210> 90
<211> 57
<212> PRT
<213> Artificial Sequence

<220>
<221> source
<223> /note="Description of Artificial Sequence: Synthetic
      polypeptide"

<400> 90
His His His His His His Gly Ser Leu Val Pro Arg Gly Ser Phe Tyr
1               5                   10                  15

Asp Pro Leu Val Phe Pro Ser Asp Glu Phe Asp Ala Ser Ile Ser Gln
            20                  25                  30

Val Asn Glu Lys Ile Asn Gln Ser Leu Ala Phe Ile Arg Lys Ser Asp
        35                  40                  45

Glu Leu Leu His Asn Val Asn Ala Gly
    50                  55

<210> 91
<211> 568
<212> PRT
<213> Artificial Sequence

<220>
<221> source
<223> /note="Description of Artificial Sequence: Synthetic
      polypeptide"

<400> 91
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Lys Arg Arg Ser Leu Gly
            100                 105                 110

Phe Leu Leu Gly Val Gly Ser Ala Ile Ala Ser Gly Val Ala Val Cys
        115                 120                 125

Lys Val Leu His Leu Glu Gly Glu Val Asn Lys Ile Lys Ser Ala Leu
    130                 135                 140

Leu Ser Thr Asn Lys Ala Val Val Ser Leu Ser Asn Gly Val Ser Val
145                 150                 155                 160

Leu Thr Phe Lys Val Leu Asp Leu Lys Asn Tyr Ile Asp Lys Gln Leu
                165                 170                 175

Leu Pro Ile Leu Asn Lys Gln Ser Cys Ser Ile Ser Asn Ile Glu Thr
            180                 185                 190

Val Ile Glu Phe Gln Gln Lys Asn Asn Arg Leu Leu Glu Ile Thr Arg
        195                 200                 205

Glu Phe Ser Val Asn Ala Gly Val Thr Thr Pro Val Ser Thr Tyr Met
    210                 215                 220

Leu Thr Asn Ser Glu Leu Leu Ser Leu Ile Asn Asp Met Pro Ile Thr
225                 230                 235                 240

Asn Asp Gln Lys Lys Leu Met Ser Asn Asn Val Gln Ile Val Arg Gln
                245                 250                 255

Gln Ser Tyr Ser Ile Met Cys Ile Ile Lys Glu Glu Val Leu Ala Tyr
            260                 265                 270

Val Val Gln Leu Pro Leu Tyr Gly Val Ile Asp Thr Pro Cys Trp Lys
        275                 280                 285

Leu His Thr Ser Pro Leu Cys Thr Thr Asn Thr Lys Glu Gly Ser Asn
    290                 295                 300

Ile Cys Leu Thr Arg Thr Asp Arg Gly Trp Tyr Cys Asp Asn Ala Gly
305                 310                 315                 320

Ser Val Ser Phe Phe Pro Gln Ala Glu Thr Cys Lys Val Gln Ser Asn
                325                 330                 335

Arg Val Phe Cys Asp Thr Met Asn Ser Leu Thr Leu Pro Ser Glu Val
            340                 345                 350

Asn Leu Cys Asn Val Asp Ile Phe Asn Pro Lys Tyr Asp Cys Lys Ile
        355                 360                 365

Met Thr Ser Lys Thr Asp Val Ser Ser Ser Val Ile Thr Ser Leu Gly
    370                 375                 380

Ala Ile Val Ser Cys Tyr Gly Lys Thr Lys Cys Thr Ala Ser Asn Lys
385                 390                 395                 400

Asn Arg Gly Ile Ile Lys Thr Phe Ser Asn Gly Cys Asp Tyr Val Ser

405                 410                 415

Asn Lys Gly Val Asp Thr Val Ser Val Gly Asn Thr Leu Tyr Tyr Val
            420                 425                 430

Asn Lys Gln Glu Gly Lys Ser Leu Tyr Val Lys Gly Glu Pro Ile Ile
        435                 440                 445

Asn Phe Tyr Asp Pro Leu Val Phe Pro Ser Asp Glu Phe Asp Ala Ser
    450                 455                 460

Ile Ser Gln Val Asn Glu Lys Ile Asn Gln Ser Leu Ala Phe Ile Arg
465                 470                 475                 480

Lys Ser Asp Glu Leu Leu Gly Gly Ser Ala Gly Ser Gly Leu His Leu
                485                 490                 495

Glu Gly Glu Val Asn Lys Ile Lys Ser Ala Leu Leu Ser Thr Asn Lys
            500                 505                 510

Ala Val Val Ser Leu Ser Asn Gly Val Ser Val Leu Thr Ser Lys Val
        515                 520                 525

Leu Asp Leu Lys Asn Tyr Ile Asp Lys Gln Leu Leu Pro Ile Val Asn
    530                 535                 540

Lys Gly Gly Ser Ala Gly Ser Gly Ser Ala Trp Ser His Pro Gln Phe
545                 550                 555                 560

Glu Lys His His His His His His
                565

<210> 92
<211> 545
<212> PRT
<213> Artificial Sequence

<220>
<221> source
<223> /note="Description of Artificial Sequence: Synthetic
      polypeptide"

<400> 92
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu

        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Lys Arg Arg Ser Leu Gly
            100                 105                 110

Phe Leu Leu Gly Val Gly Ser Ala Ile Ala Ser Gly Val Ala Val Cys
        115                 120                 125

Lys Val Leu His Leu Glu Gly Glu Val Asn Lys Ile Lys Ser Ala Leu
    130                 135                 140

Leu Ser Thr Asn Lys Ala Val Val Ser Leu Ser Asn Gly Val Ser Val
145                 150                 155                 160

Leu Thr Phe Lys Val Leu Asp Leu Lys Asn Tyr Ile Asp Lys Gln Leu
                165                 170                 175

Leu Pro Ile Leu Asn Lys Gln Ser Cys Ser Ile Ser Asn Ile Glu Thr
            180                 185                 190

Val Ile Glu Phe Gln Gln Lys Asn Asn Arg Leu Leu Glu Ile Thr Arg
        195                 200                 205

Glu Phe Ser Val Asn Ala Gly Val Thr Thr Pro Val Ser Thr Tyr Met
    210                 215                 220

Leu Thr Asn Ser Glu Leu Leu Ser Leu Ile Asn Asp Met Pro Ile Thr
225                 230                 235                 240

Asn Asp Gln Lys Lys Leu Met Ser Asn Asn Val Gln Ile Val Arg Gln
                245                 250                 255

Gln Ser Tyr Ser Ile Met Cys Ile Ile Lys Glu Glu Val Leu Ala Tyr
            260                 265                 270

Val Val Gln Leu Pro Leu Tyr Gly Val Ile Asp Thr Pro Cys Trp Lys
        275                 280                 285

Leu His Thr Ser Pro Leu Cys Thr Thr Asn Thr Lys Glu Gly Ser Asn
    290                 295                 300

Ile Cys Leu Thr Arg Thr Asp Arg Gly Trp Tyr Cys Asp Asn Ala Gly
305                 310                 315                 320

Ser Val Ser Phe Phe Pro Gln Ala Glu Thr Cys Lys Val Gln Ser Asn
                325                 330                 335

Arg Val Phe Cys Asp Thr Met Asn Ser Leu Thr Leu Pro Ser Glu Val
            340                 345                 350

Asn Leu Cys Asn Val Asp Ile Phe Asn Pro Lys Tyr Asp Cys Lys Ile
        355                 360                 365

Met Thr Ser Lys Thr Asp Val Ser Ser Ser Val Ile Thr Ser Leu Gly
    370                 375                 380

Ala Ile Val Ser Cys Tyr Gly Lys Thr Lys Cys Thr Ala Ser Asn Lys
385                 390                 395                 400

Asn Arg Gly Ile Ile Lys Thr Phe Ser Asn Gly Cys Asp Tyr Val Ser
                405                 410                 415

Asn Lys Gly Val Asp Thr Val Ser Val Gly Asn Thr Leu Tyr Tyr Val
            420                 425                 430

Asn Lys Gln Glu Gly Lys Ser Leu Tyr Val Lys Gly Glu Pro Ile Ile
        435                 440                 445

Asn Phe Tyr Asp Pro Leu Val Phe Pro Ser Asp Glu Phe Asp Ala Ser
    450                 455                 460

Ile Ser Gln Val Asn Glu Lys Ile Asn Gln Ser Leu Ala Phe Ile Arg
465                 470                 475                 480

Lys Ser Asp Glu Leu Leu Gly Gly Ser Ala Gly Ser Gly Leu His Leu
                485                 490                 495

Glu Gly Glu Val Asn Lys Ile Lys Ser Ala Leu Leu Ser Thr Asn Lys
            500                 505                 510

Ala Val Val Ser Leu Ser Asn Gly Val Ser Val Leu Thr Ser Lys Val
        515                 520                 525

Leu Asp Leu Lys Asn Tyr Ile Asp Lys Gln Leu Leu Pro Ile Val Asn
    530                 535                 540

Lys
545

<210> 93
<211> 582
<212> PRT
<213> Artificial Sequence

<220>
<221> source
<223> /note="Description of Artificial Sequence: Synthetic
      polypeptide"

<400> 93
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Lys Arg Arg Ser Leu Gly
            100                 105                 110

Phe Leu Leu Gly Val Gly Ser Ala Ile Ala Ser Gly Val Ala Val Cys
        115                 120                 125

Lys Val Leu His Leu Glu Gly Glu Val Asn Lys Ile Lys Ser Ala Leu
    130                 135                 140

Leu Ser Thr Asn Lys Ala Val Val Ser Leu Ser Asn Gly Val Ser Val
145                 150                 155                 160

Leu Thr Phe Lys Val Leu Asp Leu Lys Asn Tyr Ile Asp Lys Gln Leu
                165                 170                 175

Leu Pro Ile Leu Asn Lys Gln Ser Cys Ser Ile Ser Asn Ile Glu Thr
            180                 185                 190

Val Ile Glu Phe Gln Gln Lys Asn Asn Arg Leu Leu Glu Ile Thr Arg
        195                 200                 205

Glu Phe Ser Val Asn Ala Gly Val Thr Thr Pro Val Ser Thr Tyr Met
    210                 215                 220

Leu Thr Asn Ser Glu Leu Leu Ser Leu Ile Asn Asp Met Pro Ile Thr
225                 230                 235                 240

Asn Asp Gln Lys Lys Leu Met Ser Asn Asn Val Gln Ile Val Arg Gln
                245                 250                 255

Gln Ser Tyr Ser Ile Met Cys Ile Ile Lys Glu Glu Val Leu Ala Tyr
            260                 265                 270

Val Val Gln Leu Pro Leu Tyr Gly Val Ile Asp Thr Pro Cys Trp Lys
        275                 280                 285

Leu His Thr Ser Pro Leu Cys Thr Thr Asn Thr Lys Glu Gly Ser Asn
    290                 295                 300

Ile Cys Leu Thr Arg Thr Asp Arg Gly Trp Tyr Cys Asp Asn Ala Gly
305                 310                 315                 320

Ser Val Ser Phe Phe Pro Gln Ala Glu Thr Cys Lys Val Gln Ser Asn
                325                 330                 335

Arg Val Phe Cys Asp Thr Met Asn Ser Leu Thr Leu Pro Ser Glu Val
            340                 345                 350

Asn Leu Cys Asn Val Asp Ile Phe Asn Pro Lys Tyr Asp Cys Lys Ile
        355                 360                 365

Met Thr Ser Lys Thr Asp Val Ser Ser Ser Val Ile Thr Ser Leu Gly
    370                 375                 380

Ala Ile Val Ser Cys Tyr Gly Lys Thr Lys Cys Thr Ala Ser Asn Lys
385                 390                 395                 400

Asn Arg Gly Ile Ile Lys Thr Phe Ser Asn Gly Cys Asp Tyr Val Ser
                405                 410                 415

Asn Lys Gly Val Asp Thr Val Ser Val Gly Asn Thr Leu Tyr Tyr Val
            420                 425                 430

Asn Lys Gln Glu Gly Lys Ser Leu Tyr Val Lys Gly Glu Pro Ile Ile
        435                 440                 445

Asn Phe Tyr Asp Pro Leu Val Phe Pro Ser Asp Glu Phe Asp Ala Ser
    450                 455                 460

Ile Ser Gln Val Asn Glu Lys Ile Asn Gln Ser Leu Ala Phe Ile Arg
465                 470                 475                 480

Lys Ser Asp Glu Leu Leu Gly Gly Ser Ala Gly Ser Gly Gly Gly Ser
                485                 490                 495

Ala Gly Ser Gly Gly Gly Ser Ala Gly Ser Gly Leu His Leu Glu Gly
            500                 505                 510

Glu Val Asn Lys Ile Lys Ser Ala Leu Leu Ser Thr Asn Lys Ala Val
        515                 520                 525

Val Ser Leu Ser Asn Gly Val Ser Val Leu Thr Ser Lys Val Leu Asp
    530                 535                 540

Leu Lys Asn Tyr Ile Asp Lys Gln Leu Leu Pro Ile Val Asn Lys Gly
545                 550                 555                 560

Gly Ser Ala Gly Ser Gly Ser Ala Trp Ser His Pro Gln Phe Glu Lys
                565                 570                 575

His His His His His His
            580

<210> 94
<211> 559
<212> PRT
<213> Artificial Sequence

<220>
<221> source
<223> /note="Description of Artificial Sequence: Synthetic
      polypeptide"

<400> 94
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Lys Arg Arg Ser Leu Gly
            100                 105                 110

Phe Leu Leu Gly Val Gly Ser Ala Ile Ala Ser Gly Val Ala Val Cys
        115                 120                 125

Lys Val Leu His Leu Glu Gly Glu Val Asn Lys Ile Lys Ser Ala Leu
    130                 135                 140

Leu Ser Thr Asn Lys Ala Val Val Ser Leu Ser Asn Gly Val Ser Val
145                 150                 155                 160

Leu Thr Phe Lys Val Leu Asp Leu Lys Asn Tyr Ile Asp Lys Gln Leu
                165                 170                 175

Leu Pro Ile Leu Asn Lys Gln Ser Cys Ser Ile Ser Asn Ile Glu Thr
            180                 185                 190

Val Ile Glu Phe Gln Gln Lys Asn Asn Arg Leu Leu Glu Ile Thr Arg
        195                 200                 205

Glu Phe Ser Val Asn Ala Gly Val Thr Thr Pro Val Ser Thr Tyr Met
    210                 215                 220

Leu Thr Asn Ser Glu Leu Leu Ser Leu Ile Asn Asp Met Pro Ile Thr
225                 230                 235                 240

Asn Asp Gln Lys Lys Leu Met Ser Asn Asn Val Gln Ile Val Arg Gln
                245                 250                 255

Gln Ser Tyr Ser Ile Met Cys Ile Ile Lys Glu Glu Val Leu Ala Tyr
            260                 265                 270

Val Val Gln Leu Pro Leu Tyr Gly Val Ile Asp Thr Pro Cys Trp Lys
        275                 280                 285

Leu His Thr Ser Pro Leu Cys Thr Thr Asn Thr Lys Glu Gly Ser Asn
    290                 295                 300

Ile Cys Leu Thr Arg Thr Asp Arg Gly Trp Tyr Cys Asp Asn Ala Gly

305                 310                 315                 320

Ser Val Ser Phe Phe Pro Gln Ala Glu Thr Cys Lys Val Gln Ser Asn
                325                 330                 335

Arg Val Phe Cys Asp Thr Met Asn Ser Leu Thr Leu Pro Ser Glu Val
            340                 345                 350

Asn Leu Cys Asn Val Asp Ile Phe Asn Pro Lys Tyr Asp Cys Lys Ile
        355                 360                 365

Met Thr Ser Lys Thr Asp Val Ser Ser Ser Val Ile Thr Ser Leu Gly
    370                 375                 380

Ala Ile Val Ser Cys Tyr Gly Lys Thr Lys Cys Thr Ala Ser Asn Lys
385                 390                 395                 400

Asn Arg Gly Ile Ile Lys Thr Phe Ser Asn Gly Cys Asp Tyr Val Ser
                405                 410                 415

Asn Lys Gly Val Asp Thr Val Ser Val Gly Asn Thr Leu Tyr Tyr Val
            420                 425                 430

Asn Lys Gln Glu Gly Lys Ser Leu Tyr Val Lys Gly Glu Pro Ile Ile
        435                 440                 445

Asn Phe Tyr Asp Pro Leu Val Phe Pro Ser Asp Glu Phe Asp Ala Ser
    450                 455                 460

Ile Ser Gln Val Asn Glu Lys Ile Asn Gln Ser Leu Ala Phe Ile Arg
465                 470                 475                 480

Lys Ser Asp Glu Leu Leu Gly Gly Ser Ala Gly Ser Gly Gly Gly Ser
                485                 490                 495

Ala Gly Ser Gly Gly Gly Ser Ala Gly Ser Gly Leu His Leu Glu Gly
            500                 505                 510

Glu Val Asn Lys Ile Lys Ser Ala Leu Leu Ser Thr Asn Lys Ala Val
        515                 520                 525

Val Ser Leu Ser Asn Gly Val Ser Val Leu Thr Ser Lys Val Leu Asp
    530                 535                 540

Leu Lys Asn Tyr Ile Asp Lys Gln Leu Leu Pro Ile Val Asn Lys
545                 550                 555

<210> 95
<211> 556
<212> PRT
<213> Artificial Sequence

<220>
<221> source
<223> /note="Description of Artificial Sequence: Synthetic
      polypeptide"

<400> 95
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Gly Ser Gly Ser Ala Ile Ala Ser Gly
            100                 105                 110

Val Ala Val Cys Lys Val Leu His Leu Glu Gly Glu Val Asn Lys Ile
        115                 120                 125

Lys Ser Ala Leu Leu Ser Thr Asn Lys Ala Val Val Ser Leu Ser Asn
    130                 135                 140

Gly Val Ser Val Leu Thr Phe Lys Val Leu Asp Leu Lys Asn Tyr Ile
145                 150                 155                 160

Asp Lys Gln Leu Leu Pro Ile Leu Asn Lys Gln Ser Cys Ser Ile Ser
                165                 170                 175

Asn Ile Glu Thr Val Ile Glu Phe Gln Gln Lys Asn Asn Arg Leu Leu
            180                 185                 190

Glu Ile Thr Arg Glu Phe Ser Val Asn Ala Gly Val Thr Thr Pro Val
        195                 200                 205

Ser Thr Tyr Met Leu Thr Asn Ser Glu Leu Leu Ser Leu Ile Asn Asp
    210                 215                 220

Met Pro Ile Thr Asn Asp Gln Lys Lys Leu Met Ser Asn Asn Val Gln
225                 230                 235                 240

Ile Val Arg Gln Gln Ser Tyr Ser Ile Met Cys Ile Ile Lys Glu Glu
                245                 250                 255

Val Leu Ala Tyr Val Val Gln Leu Pro Leu Tyr Gly Val Ile Asp Thr
            260                 265                 270

Pro Cys Trp Lys Leu His Thr Ser Pro Leu Cys Thr Thr Asn Thr Lys
        275                 280                 285

Glu Gly Ser Asn Ile Cys Leu Thr Arg Thr Asp Arg Gly Trp Tyr Cys
    290                 295                 300

Asp Asn Ala Gly Ser Val Ser Phe Phe Pro Gln Ala Glu Thr Cys Lys
305                 310                 315                 320

Val Gln Ser Asn Arg Val Phe Cys Asp Thr Met Asn Ser Leu Thr Leu
                325                 330                 335

Pro Ser Glu Val Asn Leu Cys Asn Val Asp Ile Phe Asn Pro Lys Tyr
            340                 345                 350

Asp Cys Lys Ile Met Thr Ser Lys Thr Asp Val Ser Ser Ser Val Ile
        355                 360                 365

Thr Ser Leu Gly Ala Ile Val Ser Cys Tyr Gly Lys Thr Lys Cys Thr
    370                 375                 380

Ala Ser Asn Lys Asn Arg Gly Ile Ile Lys Thr Phe Ser Asn Gly Cys
385                 390                 395                 400

Asp Tyr Val Ser Asn Lys Gly Val Asp Thr Val Ser Val Gly Asn Thr
                405                 410                 415

Leu Tyr Tyr Val Asn Lys Gln Glu Gly Lys Ser Leu Tyr Val Lys Gly
            420                 425                 430

Glu Pro Ile Ile Asn Phe Tyr Asp Pro Leu Val Phe Pro Ser Asp Glu
        435                 440                 445

Phe Asp Ala Ser Ile Ser Gln Val Asn Glu Lys Ile Asn Gln Ser Leu
    450                 455                 460

Ala Phe Ile Arg Lys Ser Asp Glu Leu Leu Gly Gly Ser Ala Gly Ser
465                 470                 475                 480

Gly Leu His Leu Glu Gly Glu Val Asn Lys Ile Lys Ser Ala Leu Leu
                485                 490                 495

Ser Thr Asn Lys Ala Val Val Ser Leu Ser Asn Gly Val Ser Val Leu
            500                 505                 510

Thr Ser Lys Val Leu Asp Leu Lys Asn Tyr Ile Asp Lys Gln Leu Leu
        515                 520                 525

Pro Ile Val Asn Lys Gly Gly Ser Ala Gly Ser Gly Ser Ala Trp Ser
    530                 535                 540

His Pro Gln Phe Glu Lys His His His His His His
545                 550                 555

<210> 96
<211> 533
<212> PRT
<213> Artificial Sequence

<220>
<221> source
<223> /note="Description of Artificial Sequence: Synthetic
      polypeptide"

<400> 96
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Gly Ser Gly Ser Ala Ile Ala Ser Gly
            100                 105                 110

Val Ala Val Cys Lys Val Leu His Leu Glu Gly Glu Val Asn Lys Ile
        115                 120                 125

Lys Ser Ala Leu Leu Ser Thr Asn Lys Ala Val Val Ser Leu Ser Asn
    130                 135                 140

Gly Val Ser Val Leu Thr Phe Lys Val Leu Asp Leu Lys Asn Tyr Ile
145                 150                 155                 160

Asp Lys Gln Leu Leu Pro Ile Leu Asn Lys Gln Ser Cys Ser Ile Ser
                165                 170                 175

Asn Ile Glu Thr Val Ile Glu Phe Gln Gln Lys Asn Asn Arg Leu Leu
            180                 185                 190

Glu Ile Thr Arg Glu Phe Ser Val Asn Ala Gly Val Thr Thr Pro Val
        195                 200                 205

Ser Thr Tyr Met Leu Thr Asn Ser Glu Leu Leu Ser Leu Ile Asn Asp
    210                 215                 220

Met Pro Ile Thr Asn Asp Gln Lys Lys Leu Met Ser Asn Asn Val Gln
225                 230                 235                 240

Ile Val Arg Gln Gln Ser Tyr Ser Ile Met Cys Ile Ile Lys Glu Glu
                245                 250                 255

Val Leu Ala Tyr Val Val Gln Leu Pro Leu Tyr Gly Val Ile Asp Thr
            260                 265                 270

Pro Cys Trp Lys Leu His Thr Ser Pro Leu Cys Thr Thr Asn Thr Lys
        275                 280                 285

Glu Gly Ser Asn Ile Cys Leu Thr Arg Thr Asp Arg Gly Trp Tyr Cys
    290                 295                 300

Asp Asn Ala Gly Ser Val Ser Phe Phe Pro Gln Ala Glu Thr Cys Lys
305                 310                 315                 320

Val Gln Ser Asn Arg Val Phe Cys Asp Thr Met Asn Ser Leu Thr Leu
                325                 330                 335

Pro Ser Glu Val Asn Leu Cys Asn Val Asp Ile Phe Asn Pro Lys Tyr
            340                 345                 350

Asp Cys Lys Ile Met Thr Ser Lys Thr Asp Val Ser Ser Ser Val Ile
        355                 360                 365

Thr Ser Leu Gly Ala Ile Val Ser Cys Tyr Gly Lys Thr Lys Cys Thr
    370                 375                 380

Ala Ser Asn Lys Asn Arg Gly Ile Ile Lys Thr Phe Ser Asn Gly Cys
385                 390                 395                 400

Asp Tyr Val Ser Asn Lys Gly Val Asp Thr Val Ser Val Gly Asn Thr
                405                 410                 415

Leu Tyr Tyr Val Asn Lys Gln Glu Gly Lys Ser Leu Tyr Val Lys Gly
            420                 425                 430

Glu Pro Ile Ile Asn Phe Tyr Asp Pro Leu Val Phe Pro Ser Asp Glu
        435                 440                 445

Phe Asp Ala Ser Ile Ser Gln Val Asn Glu Lys Ile Asn Gln Ser Leu
    450                 455                 460

Ala Phe Ile Arg Lys Ser Asp Glu Leu Leu Gly Gly Ser Ala Gly Ser
465                 470                 475                 480

Gly Leu His Leu Glu Gly Glu Val Asn Lys Ile Lys Ser Ala Leu Leu
                485                 490                 495

Ser Thr Asn Lys Ala Val Val Ser Leu Ser Asn Gly Val Ser Val Leu
            500                 505                 510

Thr Ser Lys Val Leu Asp Leu Lys Asn Tyr Ile Asp Lys Gln Leu Leu
        515                 520                 525

Pro Ile Val Asn Lys
    530

<210> 97
<211> 570
<212> PRT
<213> Artificial Sequence

<220>
<221> source
<223> /note="Description of Artificial Sequence: Synthetic
      polypeptide"

<400> 97
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Gly Ser Gly Ser Ala Ile Ala Ser Gly
            100                 105                 110

Val Ala Val Cys Lys Val Leu His Leu Glu Gly Glu Val Asn Lys Ile
        115                 120                 125

Lys Ser Ala Leu Leu Ser Thr Asn Lys Ala Val Val Ser Leu Ser Asn
    130                 135                 140

Gly Val Ser Val Leu Thr Phe Lys Val Leu Asp Leu Lys Asn Tyr Ile
145                 150                 155                 160

Asp Lys Gln Leu Leu Pro Ile Leu Asn Lys Gln Ser Cys Ser Ile Ser
                165                 170                 175

Asn Ile Glu Thr Val Ile Glu Phe Gln Gln Lys Asn Asn Arg Leu Leu
            180                 185                 190

Glu Ile Thr Arg Glu Phe Ser Val Asn Ala Gly Val Thr Thr Pro Val
        195                 200                 205

Ser Thr Tyr Met Leu Thr Asn Ser Glu Leu Leu Ser Leu Ile Asn Asp
    210                 215                 220

Met Pro Ile Thr Asn Asp Gln Lys Lys Leu Met Ser Asn Asn Val Gln
225                 230                 235                 240

Ile Val Arg Gln Gln Ser Tyr Ser Ile Met Cys Ile Ile Lys Glu Glu
                245                 250                 255

Val Leu Ala Tyr Val Val Gln Leu Pro Leu Tyr Gly Val Ile Asp Thr
            260                 265                 270

Pro Cys Trp Lys Leu His Thr Ser Pro Leu Cys Thr Thr Asn Thr Lys

275                 280                 285

Glu Gly Ser Asn Ile Cys Leu Thr Arg Thr Asp Arg Gly Trp Tyr Cys
    290                 295                 300

Asp Asn Ala Gly Ser Val Ser Phe Phe Pro Gln Ala Glu Thr Cys Lys
305                 310                 315                 320

Val Gln Ser Asn Arg Val Phe Cys Asp Thr Met Asn Ser Leu Thr Leu
                325                 330                 335

Pro Ser Glu Val Asn Leu Cys Asn Val Asp Ile Phe Asn Pro Lys Tyr
            340                 345                 350

Asp Cys Lys Ile Met Thr Ser Lys Thr Asp Val Ser Ser Ser Val Ile
        355                 360                 365

Thr Ser Leu Gly Ala Ile Val Ser Cys Tyr Gly Lys Thr Lys Cys Thr
    370                 375                 380

Ala Ser Asn Lys Asn Arg Gly Ile Ile Lys Thr Phe Ser Asn Gly Cys
385                 390                 395                 400

Asp Tyr Val Ser Asn Lys Gly Val Asp Thr Val Ser Val Gly Asn Thr
                405                 410                 415

Leu Tyr Tyr Val Asn Lys Gln Glu Gly Lys Ser Leu Tyr Val Lys Gly
            420                 425                 430

Glu Pro Ile Ile Asn Phe Tyr Asp Pro Leu Val Phe Pro Ser Asp Glu
        435                 440                 445

Phe Asp Ala Ser Ile Ser Gln Val Asn Glu Lys Ile Asn Gln Ser Leu
    450                 455                 460

Ala Phe Ile Arg Lys Ser Asp Glu Leu Leu Gly Gly Ser Ala Gly Ser
465                 470                 475                 480

Gly Gly Gly Ser Ala Gly Ser Gly Gly Gly Ser Ala Gly Ser Gly Leu
                485                 490                 495

His Leu Glu Gly Glu Val Asn Lys Ile Lys Ser Ala Leu Leu Ser Thr
            500                 505                 510

Asn Lys Ala Val Val Ser Leu Ser Asn Gly Val Ser Val Leu Thr Ser
        515                 520                 525

Lys Val Leu Asp Leu Lys Asn Tyr Ile Asp Lys Gln Leu Leu Pro Ile
    530                 535                 540

Val Asn Lys Gly Gly Ser Ala Gly Ser Gly Ser Ala Trp Ser His Pro
545                 550                 555                 560

Gln Phe Glu Lys His His His His His His
                565                 570

<210> 98
<211> 547
<212> PRT
<213> Artificial Sequence

<220>
<221> source
<223> /note="Description of Artificial Sequence: Synthetic
      polypeptide"

<400> 98
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Gly Ser Gly Ser Ala Ile Ala Ser Gly
            100                 105                 110

Val Ala Val Cys Lys Val Leu His Leu Glu Gly Glu Val Asn Lys Ile
        115                 120                 125

Lys Ser Ala Leu Leu Ser Thr Asn Lys Ala Val Val Ser Leu Ser Asn
    130                 135                 140

Gly Val Ser Val Leu Thr Phe Lys Val Leu Asp Leu Lys Asn Tyr Ile
145                 150                 155                 160

Asp Lys Gln Leu Leu Pro Ile Leu Asn Lys Gln Ser Cys Ser Ile Ser
                165                 170                 175

Asn Ile Glu Thr Val Ile Glu Phe Gln Gln Lys Asn Asn Arg Leu Leu
            180                 185                 190

Glu Ile Thr Arg Glu Phe Ser Val Asn Ala Gly Val Thr Thr Pro Val
        195                 200                 205

Ser Thr Tyr Met Leu Thr Asn Ser Glu Leu Leu Ser Leu Ile Asn Asp
    210                 215                 220

Met Pro Ile Thr Asn Asp Gln Lys Lys Leu Met Ser Asn Asn Val Gln
225                 230                 235                 240

Ile Val Arg Gln Gln Ser Tyr Ser Ile Met Cys Ile Ile Lys Glu Glu
                245                 250                 255

Val Leu Ala Tyr Val Val Gln Leu Pro Leu Tyr Gly Val Ile Asp Thr
            260                 265                 270

Pro Cys Trp Lys Leu His Thr Ser Pro Leu Cys Thr Thr Asn Thr Lys
        275                 280                 285

Glu Gly Ser Asn Ile Cys Leu Thr Arg Thr Asp Arg Gly Trp Tyr Cys
    290                 295                 300

Asp Asn Ala Gly Ser Val Ser Phe Phe Pro Gln Ala Glu Thr Cys Lys
305                 310                 315                 320

Val Gln Ser Asn Arg Val Phe Cys Asp Thr Met Asn Ser Leu Thr Leu
                325                 330                 335

Pro Ser Glu Val Asn Leu Cys Asn Val Asp Ile Phe Asn Pro Lys Tyr
            340                 345                 350

Asp Cys Lys Ile Met Thr Ser Lys Thr Asp Val Ser Ser Ser Val Ile
        355                 360                 365

Thr Ser Leu Gly Ala Ile Val Ser Cys Tyr Gly Lys Thr Lys Cys Thr
    370                 375                 380

Ala Ser Asn Lys Asn Arg Gly Ile Ile Lys Thr Phe Ser Asn Gly Cys
385                 390                 395                 400

Asp Tyr Val Ser Asn Lys Gly Val Asp Thr Val Ser Val Gly Asn Thr
                405                 410                 415

Leu Tyr Tyr Val Asn Lys Gln Glu Gly Lys Ser Leu Tyr Val Lys Gly
            420                 425                 430

Glu Pro Ile Ile Asn Phe Tyr Asp Pro Leu Val Phe Pro Ser Asp Glu
        435                 440                 445

Phe Asp Ala Ser Ile Ser Gln Val Asn Glu Lys Ile Asn Gln Ser Leu
    450                 455                 460

Ala Phe Ile Arg Lys Ser Asp Glu Leu Leu Gly Gly Ser Ala Gly Ser
465                 470                 475                 480

Gly Gly Gly Ser Ala Gly Ser Gly Gly Gly Ser Ala Gly Ser Gly Leu
                485                 490                 495

His Leu Glu Gly Glu Val Asn Lys Ile Lys Ser Ala Leu Leu Ser Thr
            500                 505                 510

Asn Lys Ala Val Val Ser Leu Ser Asn Gly Val Ser Val Leu Thr Ser
        515                 520                 525

Lys Val Leu Asp Leu Lys Asn Tyr Ile Asp Lys Gln Leu Leu Pro Ile
    530                 535                 540

Val Asn Lys
545

<210> 99
<211> 596
<212> PRT
<213> Artificial Sequence

<220>
<221> source
<223> /note="Description of Artificial Sequence: Synthetic
      polypeptide"

<400> 99
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Lys Arg Arg Ser Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Ser Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Ser Ala Ile Gly Gly Tyr Ile Pro Glu Ala Pro Arg Asp Gly Gln
        515                 520                 525

Ala Tyr Val Arg Lys Asp Gly Glu Trp Val Leu Leu Ser Thr Phe Leu
    530                 535                 540

Gly Gly Leu Val Pro Arg Gly Gly Ser Ala Gly Ser Gly Trp Ser His
545                 550                 555                 560

Pro Gln Phe Glu Lys Gly Gly Gly Ser Gly Gly Gly Ser Gly Gly Gly

                565                 570                 575

Ser Trp Ser His Pro Gln Phe Glu Lys Gly Ser Lys Gly Gly His His
            580                 585                 590

His His His His
        595

<210> 100
<211> 569
<212> PRT
<213> Artificial Sequence

<220>
<221> source
<223> /note="Description of Artificial Sequence: Synthetic
polypeptide"

<400> 100
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Phe Leu Gly
            100                 105                 110

Phe Leu Leu Gly Val Gly Ser Ala Ile Ala Ser Gly Val Ala Val Cys
        115                 120                 125

Lys Val Leu His Leu Glu Gly Glu Val Asn Lys Ile Lys Ser Ala Leu
    130                 135                 140

Leu Ser Thr Asn Lys Ala Val Val Ser Leu Ser Asn Gly Val Ser Val
145                 150                 155                 160

Leu Thr Phe Lys Val Leu Asp Leu Lys Asn Tyr Ile Asp Lys Gln Leu

                165                 170                 175

Leu Pro Ile Leu Asn Lys Gln Ser Cys Ser Ile Ser Asn Ile Glu Thr
            180                 185                 190

Val Ile Glu Phe Gln Gln Lys Asn Asn Arg Leu Leu Glu Ile Thr Arg
        195                 200                 205

Glu Phe Ser Val Asn Ala Gly Val Thr Thr Pro Val Ser Thr Tyr Met
    210                 215                 220

Leu Thr Asn Ser Glu Leu Leu Ser Leu Ile Asn Asp Met Pro Ile Thr
225                 230                 235                 240

Asn Asp Gln Lys Lys Leu Met Ser Asn Asn Val Gln Ile Val Arg Gln
                245                 250                 255

Gln Ser Tyr Ser Ile Met Cys Ile Ile Lys Glu Glu Val Leu Ala Tyr
            260                 265                 270

Val Val Gln Leu Pro Leu Tyr Gly Val Ile Asp Thr Pro Cys Trp Lys
        275                 280                 285

Leu His Thr Ser Pro Leu Cys Thr Thr Asn Thr Lys Glu Gly Ser Asn
    290                 295                 300

Ile Cys Leu Thr Arg Thr Asp Arg Gly Trp Tyr Cys Asp Asn Ala Gly
305                 310                 315                 320

Ser Val Ser Phe Phe Pro Gln Ala Glu Thr Cys Lys Val Gln Ser Asn
                325                 330                 335

Arg Val Phe Cys Asp Thr Met Asn Ser Leu Thr Leu Pro Ser Glu Val
            340                 345                 350

Asn Leu Cys Asn Val Asp Ile Phe Asn Pro Lys Tyr Asp Cys Lys Ile
        355                 360                 365

Met Thr Ser Lys Thr Asp Val Ser Ser Ser Val Ile Thr Ser Leu Gly
    370                 375                 380

Ala Ile Val Ser Cys Tyr Gly Lys Thr Lys Cys Thr Ala Ser Asn Lys
385                 390                 395                 400

Asn Arg Gly Ile Ile Lys Thr Phe Ser Asn Gly Cys Asp Tyr Val Ser
                405                 410                 415

Asn Lys Gly Val Asp Thr Val Ser Val Gly Asn Thr Leu Tyr Tyr Val
            420                 425                 430

Asn Lys Gln Glu Gly Lys Ser Leu Tyr Val Lys Gly Glu Pro Ile Ile
        435                 440                 445

Asn Phe Tyr Asp Pro Leu Val Phe Pro Ser Asp Glu Phe Asp Ala Ser
    450                 455                 460

Ile Ser Gln Val Asn Glu Lys Ile Asn Gln Ser Leu Ala Phe Ile Arg
465                 470                 475                 480

Lys Ser Asp Glu Leu Leu Ser Ala Ile Gly Gly Tyr Ile Pro Glu Ala
                485                 490                 495

Pro Arg Asp Gly Gln Ala Tyr Val Arg Lys Asp Gly Glu Trp Val Leu
            500                 505                 510

Leu Ser Thr Phe Leu Gly Gly Leu Val Pro Arg Gly Gly Ser Ala Gly
        515                 520                 525

Ser Gly Trp Ser His Pro Gln Phe Glu Lys Gly Gly Gly Ser Gly Gly
    530                 535                 540

Gly Ser Gly Gly Gly Ser Trp Ser His Pro Gln Phe Glu Lys Gly Ser
545                 550                 555                 560

Lys Gly Gly His His His His His His
                565

<210> 101
<211> 569
<212> PRT
<213> Artificial Sequence

<220>
<221> source
<223> /note="Description of Artificial Sequence: Synthetic
      polypeptide"

<400> 101
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Lys Arg Arg Phe Leu Gly
            100                 105                 110

Phe Leu Leu Gly Val Gly Ser Ala Ile Ala Ser Gly Val Ala Val Cys
        115                 120                 125

Lys Val Leu His Leu Glu Gly Glu Val Asn Lys Ile Lys Ser Ala Leu
    130                 135                 140

Leu Ser Thr Asn Lys Ala Val Val Ser Leu Ser Asn Gly Val Ser Val
145                 150                 155                 160

Leu Thr Phe Lys Val Leu Asp Leu Lys Asn Tyr Ile Asp Lys Gln Leu
                165                 170                 175

Leu Pro Ile Leu Asn Lys Gln Ser Cys Ser Ile Ser Asn Ile Glu Thr
            180                 185                 190

Val Ile Glu Phe Gln Gln Lys Asn Asn Arg Leu Leu Glu Ile Thr Arg
        195                 200                 205

Glu Phe Ser Val Asn Ala Gly Val Thr Thr Pro Val Ser Thr Tyr Met
    210                 215                 220

Leu Thr Asn Ser Glu Leu Leu Ser Leu Ile Asn Asp Met Pro Ile Thr
225                 230                 235                 240

Asn Asp Gln Lys Lys Leu Met Ser Asn Asn Val Gln Ile Val Arg Gln
                245                 250                 255

Gln Ser Tyr Ser Ile Met Cys Ile Ile Lys Glu Glu Val Leu Ala Tyr
            260                 265                 270

Val Val Gln Leu Pro Leu Tyr Gly Val Ile Asp Thr Pro Cys Trp Lys
        275                 280                 285

Leu His Thr Ser Pro Leu Cys Thr Thr Asn Thr Lys Glu Gly Ser Asn
    290                 295                 300

Ile Cys Leu Thr Arg Thr Asp Arg Gly Trp Tyr Cys Asp Asn Ala Gly
305                 310                 315                 320

Ser Val Ser Phe Phe Pro Gln Ala Glu Thr Cys Lys Val Gln Ser Asn
                325                 330                 335

Arg Val Phe Cys Asp Thr Met Asn Ser Leu Thr Leu Pro Ser Glu Val
            340                 345                 350

Asn Leu Cys Asn Val Asp Ile Phe Asn Pro Lys Tyr Asp Cys Lys Ile
        355                 360                 365

Met Thr Ser Lys Thr Asp Val Ser Ser Ser Val Ile Thr Ser Leu Gly
    370                 375                 380

Ala Ile Val Ser Cys Tyr Gly Lys Thr Lys Cys Thr Ala Ser Asn Lys
385                 390                 395                 400

Asn Arg Gly Ile Ile Lys Thr Phe Ser Asn Gly Cys Asp Tyr Val Ser
                405                 410                 415

Asn Lys Gly Val Asp Thr Val Ser Val Gly Asn Thr Leu Tyr Tyr Val
            420                 425                 430

Asn Lys Gln Glu Gly Lys Ser Leu Tyr Val Lys Gly Glu Pro Ile Ile
        435                 440                 445

Asn Phe Tyr Asp Pro Leu Val Phe Pro Ser Asp Glu Phe Asp Ala Ser
    450                 455                 460

Ile Ser Gln Val Asn Glu Lys Ile Asn Gln Ser Leu Ala Phe Ile Arg
465                 470                 475                 480

Lys Ser Asp Glu Leu Leu Ser Ala Ile Gly Gly Tyr Ile Pro Glu Ala
                485                 490                 495

Pro Arg Asp Gly Gln Ala Tyr Val Arg Lys Asp Gly Glu Trp Val Leu
            500                 505                 510

Leu Ser Thr Phe Leu Gly Gly Leu Val Pro Arg Gly Gly Ser Ala Gly
        515                 520                 525

Ser Gly Trp Ser His Pro Gln Phe Glu Lys Gly Gly Gly Ser Gly Gly
    530                 535                 540

Gly Ser Gly Gly Gly Ser Trp Ser His Pro Gln Phe Glu Lys Gly Ser
545                 550                 555                 560

Lys Gly Gly His His His His His His
                565

<210> 102
<211> 570
<212> PRT
<213> Artificial Sequence

<220>
<221> source
<223> /note="Description of Artificial Sequence: Synthetic
      polypeptide"

<400> 102
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Lys Arg Arg Ser Phe Leu
            100                 105                 110

Gly Phe Leu Leu Gly Val Gly Ser Ala Ile Ala Ser Gly Val Ala Val
        115                 120                 125

Cys Lys Val Leu His Leu Glu Gly Glu Val Asn Lys Ile Lys Ser Ala
    130                 135                 140

Leu Leu Ser Thr Asn Lys Ala Val Val Ser Leu Ser Asn Gly Val Ser
145                 150                 155                 160

Val Leu Thr Phe Lys Val Leu Asp Leu Lys Asn Tyr Ile Asp Lys Gln
                165                 170                 175

Leu Leu Pro Ile Leu Asn Lys Gln Ser Cys Ser Ile Ser Asn Ile Glu
            180                 185                 190

Thr Val Ile Glu Phe Gln Gln Lys Asn Asn Arg Leu Leu Glu Ile Thr
        195                 200                 205

Arg Glu Phe Ser Val Asn Ala Gly Val Thr Thr Pro Val Ser Thr Tyr
    210                 215                 220

Met Leu Thr Asn Ser Glu Leu Leu Ser Leu Ile Asn Asp Met Pro Ile
225                 230                 235                 240

Thr Asn Asp Gln Lys Lys Leu Met Ser Asn Asn Val Gln Ile Val Arg
                245                 250                 255

Gln Gln Ser Tyr Ser Ile Met Cys Ile Ile Lys Glu Glu Val Leu Ala
            260                 265                 270

Tyr Val Val Gln Leu Pro Leu Tyr Gly Val Ile Asp Thr Pro Cys Trp
        275                 280                 285

Lys Leu His Thr Ser Pro Leu Cys Thr Thr Asn Thr Lys Glu Gly Ser
    290                 295                 300

Asn Ile Cys Leu Thr Arg Thr Asp Arg Gly Trp Tyr Cys Asp Asn Ala
305                 310                 315                 320

Gly Ser Val Ser Phe Phe Pro Gln Ala Glu Thr Cys Lys Val Gln Ser
                325                 330                 335

Asn Arg Val Phe Cys Asp Thr Met Asn Ser Leu Thr Leu Pro Ser Glu
            340                 345                 350

Val Asn Leu Cys Asn Val Asp Ile Phe Asn Pro Lys Tyr Asp Cys Lys
        355                 360                 365

Ile Met Thr Ser Lys Thr Asp Val Ser Ser Ser Val Ile Thr Ser Leu
    370                 375                 380

Gly Ala Ile Val Ser Cys Tyr Gly Lys Thr Lys Cys Thr Ala Ser Asn
385                 390                 395                 400

Lys Asn Arg Gly Ile Ile Lys Thr Phe Ser Asn Gly Cys Asp Tyr Val
                405                 410                 415

Ser Asn Lys Gly Val Asp Thr Val Ser Val Gly Asn Thr Leu Tyr Tyr
            420                 425                 430

Val Asn Lys Gln Glu Gly Lys Ser Leu Tyr Val Lys Gly Glu Pro Ile

435                 440                 445

Ile Asn Phe Tyr Asp Pro Leu Val Phe Pro Ser Asp Glu Phe Asp Ala
    450                 455                 460

Ser Ile Ser Gln Val Asn Glu Lys Ile Asn Gln Ser Leu Ala Phe Ile
465                 470                 475                 480

Arg Lys Ser Asp Glu Leu Leu Ser Ala Ile Gly Gly Tyr Ile Pro Glu
                485                 490                 495

Ala Pro Arg Asp Gly Gln Ala Tyr Val Arg Lys Asp Gly Glu Trp Val
            500                 505                 510

Leu Leu Ser Thr Phe Leu Gly Gly Leu Val Pro Arg Gly Gly Ser Ala
        515                 520                 525

Gly Ser Gly Trp Ser His Pro Gln Phe Glu Lys Gly Gly Gly Ser Gly
    530                 535                 540

Gly Gly Ser Gly Gly Gly Ser Trp Ser His Pro Gln Phe Glu Lys Gly
545                 550                 555                 560

Ser Lys Gly Gly His His His His His His
                565                 570

<210> 103
<211> 569
<212> PRT
<213> Artificial Sequence

<220>
<221> source
<223> /note="Description of Artificial Sequence: Synthetic
      polypeptide"

<400> 103
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys

65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Lys Arg Arg Ser Leu Gly
            100                 105                 110

Phe Leu Leu Gly Val Gly Ser Ala Ile Ala Ser Gly Val Ala Val Cys
        115                 120                 125

Lys Val Leu His Leu Glu Gly Glu Val Asn Lys Ile Lys Ser Ala Leu
    130                 135                 140

Leu Ser Thr Asn Lys Ala Val Val Ser Leu Ser Asn Gly Val Ser Val
145                 150                 155                 160

Leu Thr Phe Lys Val Leu Asp Leu Lys Asn Tyr Ile Asp Lys Gln Leu
                165                 170                 175

Leu Pro Ile Leu Asn Lys Gln Ser Cys Ser Ile Ser Asn Ile Glu Thr
            180                 185                 190

Val Ile Glu Phe Gln Gln Lys Asn Asn Arg Leu Leu Glu Ile Thr Arg
        195                 200                 205

Glu Phe Ser Val Asn Ala Gly Val Thr Thr Pro Val Ser Thr Tyr Met
    210                 215                 220

Leu Thr Asn Ser Glu Leu Leu Ser Leu Ile Asn Asp Met Pro Ile Thr
225                 230                 235                 240

Asn Asp Gln Lys Lys Leu Met Ser Asn Asn Val Gln Ile Val Arg Gln
                245                 250                 255

Gln Ser Tyr Ser Ile Met Cys Ile Ile Lys Glu Glu Val Leu Ala Tyr
            260                 265                 270

Val Val Gln Leu Pro Leu Tyr Gly Val Ile Asp Thr Pro Cys Trp Lys
        275                 280                 285

Leu His Thr Ser Pro Leu Cys Thr Thr Asn Thr Lys Glu Gly Ser Asn
    290                 295                 300

Ile Cys Leu Thr Arg Thr Asp Arg Gly Trp Tyr Cys Asp Asn Ala Gly
305                 310                 315                 320

Ser Val Ser Phe Phe Pro Gln Ala Glu Thr Cys Lys Val Gln Ser Asn
                325                 330                 335

Arg Val Phe Cys Asp Thr Met Asn Ser Leu Thr Leu Pro Ser Glu Val
            340                 345                 350

Asn Leu Cys Asn Val Asp Ile Phe Asn Pro Lys Tyr Asp Cys Lys Ile
        355                 360                 365

Met Thr Ser Lys Thr Asp Val Ser Ser Ser Val Ile Thr Ser Leu Gly
    370                 375                 380

Ala Ile Val Ser Cys Tyr Gly Lys Thr Lys Cys Thr Ala Ser Asn Lys
385                 390                 395                 400

Asn Arg Gly Ile Ile Lys Thr Phe Ser Asn Gly Cys Asp Tyr Val Ser
                405                 410                 415

Asn Lys Gly Val Asp Thr Val Ser Val Gly Asn Thr Leu Tyr Tyr Val
            420                 425                 430

Asn Lys Gln Glu Gly Lys Ser Leu Tyr Val Lys Gly Glu Pro Ile Ile
        435                 440                 445

Asn Phe Tyr Asp Pro Leu Val Phe Pro Ser Asp Glu Phe Asp Ala Ser
    450                 455                 460

Ile Ser Gln Val Asn Glu Lys Ile Asn Gln Ser Leu Ala Phe Ile Arg
465                 470                 475                 480

Lys Ser Asp Glu Leu Leu Ser Ala Ile Gly Gly Tyr Ile Pro Glu Ala
                485                 490                 495

Pro Arg Asp Gly Gln Ala Tyr Val Arg Lys Asp Gly Glu Trp Val Leu
            500                 505                 510

Leu Ser Thr Phe Leu Gly Gly Leu Val Pro Arg Gly Gly Ser Ala Gly
        515                 520                 525

Ser Gly Trp Ser His Pro Gln Phe Glu Lys Gly Gly Gly Ser Gly Gly
    530                 535                 540

Gly Ser Gly Gly Gly Ser Trp Ser His Pro Gln Phe Glu Lys Gly Ser
545                 550                 555                 560

Lys Gly Gly His His His His His His
                565

<210> 104
<211> 571
<212> PRT
<213> Artificial Sequence

<220>
<221> source
<223> /note="Description of Artificial Sequence: Synthetic
      polypeptide"

<400> 104
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Lys Arg Arg Ser Gly Phe
            100                 105                 110

Leu Gly Phe Leu Leu Gly Val Gly Ser Ala Ile Ala Ser Gly Val Ala
        115                 120                 125

Val Cys Lys Val Leu His Leu Glu Gly Glu Val Asn Lys Ile Lys Ser
    130                 135                 140

Ala Leu Leu Ser Thr Asn Lys Ala Val Val Ser Leu Ser Asn Gly Val
145                 150                 155                 160

Ser Val Leu Thr Phe Lys Val Leu Asp Leu Lys Asn Tyr Ile Asp Lys
                165                 170                 175

Gln Leu Leu Pro Ile Leu Asn Lys Gln Ser Cys Ser Ile Ser Asn Ile
            180                 185                 190

Glu Thr Val Ile Glu Phe Gln Gln Lys Asn Asn Arg Leu Leu Glu Ile
        195                 200                 205

Thr Arg Glu Phe Ser Val Asn Ala Gly Val Thr Thr Pro Val Ser Thr
    210                 215                 220

Tyr Met Leu Thr Asn Ser Glu Leu Leu Ser Leu Ile Asn Asp Met Pro
225                 230                 235                 240

Ile Thr Asn Asp Gln Lys Lys Leu Met Ser Asn Asn Val Gln Ile Val
                245                 250                 255

Arg Gln Gln Ser Tyr Ser Ile Met Cys Ile Ile Lys Glu Glu Val Leu
            260                 265                 270

Ala Tyr Val Val Gln Leu Pro Leu Tyr Gly Val Ile Asp Thr Pro Cys
        275                 280                 285

Trp Lys Leu His Thr Ser Pro Leu Cys Thr Thr Asn Thr Lys Glu Gly
    290                 295                 300

Ser Asn Ile Cys Leu Thr Arg Thr Asp Arg Gly Trp Tyr Cys Asp Asn
305                 310                 315                 320

Ala Gly Ser Val Ser Phe Phe Pro Gln Ala Glu Thr Cys Lys Val Gln
                325                 330                 335

Ser Asn Arg Val Phe Cys Asp Thr Met Asn Ser Leu Thr Leu Pro Ser
            340                 345                 350

Glu Val Asn Leu Cys Asn Val Asp Ile Phe Asn Pro Lys Tyr Asp Cys
        355                 360                 365

Lys Ile Met Thr Ser Lys Thr Asp Val Ser Ser Ser Val Ile Thr Ser
    370                 375                 380

Leu Gly Ala Ile Val Ser Cys Tyr Gly Lys Thr Lys Cys Thr Ala Ser
385                 390                 395                 400

Asn Lys Asn Arg Gly Ile Ile Lys Thr Phe Ser Asn Gly Cys Asp Tyr
                405                 410                 415

Val Ser Asn Lys Gly Val Asp Thr Val Ser Val Gly Asn Thr Leu Tyr
            420                 425                 430

Tyr Val Asn Lys Gln Glu Gly Lys Ser Leu Tyr Val Lys Gly Glu Pro
        435                 440                 445

Ile Ile Asn Phe Tyr Asp Pro Leu Val Phe Pro Ser Asp Glu Phe Asp
    450                 455                 460

Ala Ser Ile Ser Gln Val Asn Glu Lys Ile Asn Gln Ser Leu Ala Phe
465                 470                 475                 480

Ile Arg Lys Ser Asp Glu Leu Leu Ser Ala Ile Gly Gly Tyr Ile Pro
                485                 490                 495

Glu Ala Pro Arg Asp Gly Gln Ala Tyr Val Arg Lys Asp Gly Glu Trp
            500                 505                 510

Val Leu Leu Ser Thr Phe Leu Gly Gly Leu Val Pro Arg Gly Gly Ser
        515                 520                 525

Ala Gly Ser Gly Trp Ser His Pro Gln Phe Glu Lys Gly Gly Gly Ser
    530                 535                 540

Gly Gly Gly Ser Gly Gly Gly Ser Trp Ser His Pro Gln Phe Glu Lys
545                 550                 555                 560

Gly Ser Lys Gly Gly His His His His His His
                565                 570

<210> 105
<211> 570
<212> PRT
<213> Artificial Sequence

<220>
<221> source
<223> /note="Description of Artificial Sequence: Synthetic
      polypeptide"

<400> 105
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Gly Arg Lys Arg Arg Ser Leu
            100                 105                 110

Gly Phe Leu Leu Gly Val Gly Ser Ala Ile Ala Ser Gly Val Ala Val
        115                 120                 125

Cys Lys Val Leu His Leu Glu Gly Glu Val Asn Lys Ile Lys Ser Ala
    130                 135                 140

Leu Leu Ser Thr Asn Lys Ala Val Val Ser Leu Ser Asn Gly Val Ser
145                 150                 155                 160

Val Leu Thr Phe Lys Val Leu Asp Leu Lys Asn Tyr Ile Asp Lys Gln
                165                 170                 175

Leu Leu Pro Ile Leu Asn Lys Gln Ser Cys Ser Ile Ser Asn Ile Glu
            180                 185                 190

Thr Val Ile Glu Phe Gln Gln Lys Asn Asn Arg Leu Leu Glu Ile Thr
        195                 200                 205

Arg Glu Phe Ser Val Asn Ala Gly Val Thr Thr Pro Val Ser Thr Tyr
    210                 215                 220

Met Leu Thr Asn Ser Glu Leu Leu Ser Leu Ile Asn Asp Met Pro Ile
225                 230                 235                 240

Thr Asn Asp Gln Lys Lys Leu Met Ser Asn Asn Val Gln Ile Val Arg
                245                 250                 255

Gln Gln Ser Tyr Ser Ile Met Cys Ile Ile Lys Glu Glu Val Leu Ala
            260                 265                 270

Tyr Val Val Gln Leu Pro Leu Tyr Gly Val Ile Asp Thr Pro Cys Trp
        275                 280                 285

Lys Leu His Thr Ser Pro Leu Cys Thr Thr Asn Thr Lys Glu Gly Ser
    290                 295                 300

Asn Ile Cys Leu Thr Arg Thr Asp Arg Gly Trp Tyr Cys Asp Asn Ala
305                 310                 315                 320

Gly Ser Val Ser Phe Phe Pro Gln Ala Glu Thr Cys Lys Val Gln Ser
                325                 330                 335

Asn Arg Val Phe Cys Asp Thr Met Asn Ser Leu Thr Leu Pro Ser Glu

340                 345                 350

Val Asn Leu Cys Asn Val Asp Ile Phe Asn Pro Lys Tyr Asp Cys Lys
        355                 360                 365

Ile Met Thr Ser Lys Thr Asp Val Ser Ser Ser Val Ile Thr Ser Leu
    370                 375                 380

Gly Ala Ile Val Ser Cys Tyr Gly Lys Thr Lys Cys Thr Ala Ser Asn
385                 390                 395                 400

Lys Asn Arg Gly Ile Ile Lys Thr Phe Ser Asn Gly Cys Asp Tyr Val
                405                 410                 415

Ser Asn Lys Gly Val Asp Thr Val Ser Val Gly Asn Thr Leu Tyr Tyr
            420                 425                 430

Val Asn Lys Gln Glu Gly Lys Ser Leu Tyr Val Lys Gly Glu Pro Ile
        435                 440                 445

Ile Asn Phe Tyr Asp Pro Leu Val Phe Pro Ser Asp Glu Phe Asp Ala
    450                 455                 460

Ser Ile Ser Gln Val Asn Glu Lys Ile Asn Gln Ser Leu Ala Phe Ile
465                 470                 475                 480

Arg Lys Ser Asp Glu Leu Leu Ser Ala Ile Gly Gly Tyr Ile Pro Glu
                485                 490                 495

Ala Pro Arg Asp Gly Gln Ala Tyr Val Arg Lys Asp Gly Glu Trp Val
            500                 505                 510

Leu Leu Ser Thr Phe Leu Gly Gly Leu Val Pro Arg Gly Gly Ser Ala
        515                 520                 525

Gly Ser Gly Trp Ser His Pro Gln Phe Glu Lys Gly Gly Gly Ser Gly
    530                 535                 540

Gly Gly Ser Gly Gly Gly Ser Trp Ser His Pro Gln Phe Glu Lys Gly
545                 550                 555                 560

Ser Lys Gly Gly His His His His His His
                565                 570

<210> 106
<211> 596
<212> PRT
<213> Artificial Sequence

<220>
<221> source
<223> /note="Description of Artificial Sequence: Synthetic
      polypeptide"

<400> 106
Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Cys Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Phe Lys Val
            180                 185                 190

Leu Asp Leu Lys Asn Tyr Ile Asp Lys Gln Leu Leu Pro Ile Leu Asn
        195                 200                 205

Lys Gln Ser Cys Ser Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Cys Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu Ser Ala Ile Gly Gly Tyr Ile Pro Glu Ala Pro Arg Asp Gly Gln
        515                 520                 525

Ala Tyr Val Arg Lys Asp Gly Glu Trp Val Leu Leu Ser Thr Phe Leu
    530                 535                 540

Gly Gly Leu Val Pro Arg Gly Gly Ser Ala Gly Ser Gly Trp Ser His
545                 550                 555                 560

Pro Gln Phe Glu Lys Gly Gly Gly Ser Gly Gly Gly Ser Gly Gly Gly
                565                 570                 575

Ser Trp Ser His Pro Gln Phe Glu Lys Gly Ser Lys Gly Gly His His
            580                 585                 590

His His His His
        595

<210> 107
<211> 21
<212> PRT
<213> Artificial Sequence

<220>
<221> source
<223> /note="Description of Artificial Sequence: Synthetic
      peptide"

<400> 107
Met Glu Thr Asp Thr Leu Leu Leu Trp Val Leu Leu Leu Trp Val Pro
1               5                   10                  15

Gly Ser Thr Gly Asp
            20

<210> 108
<211> 8
<212> PRT
<213> Artificial Sequence

<220>
<221> source
<223> /note="Description of Artificial Sequence: Synthetic
      peptide"

<400> 108

Trp Ser His Pro Gln Phe Glu Lys
1               5

<210> 109
<211> 4
<212> PRT
<213> Artificial Sequence

<220>
<221> source
<223> /note="Description of Artificial Sequence: Synthetic
      peptide"

<400> 109
Arg Ala Arg Arg
1

<210> 110
<211> 4
<212> PRT
<213> Artificial Sequence

<220>
<221> source
<223> /note="Description of Artificial Sequence: Synthetic
      peptide"

<400> 110
Arg Lys Arg Arg
1

<210> 111
<211> 4
<212> PRT
<213> Artificial Sequence

<220>
<221> source
<223> /note="Description of Artificial Sequence: Synthetic
      peptide"

<400> 111
Gly Pro Gly Ala
1